# EUROPEAN PATENT APPLICATION

(11) **EP 4 368 713 A1**
(43) Date of publication of application: **15.05.2024**
(21) Application number: 22837949.1
(22) Date of filing: 05.07.2022
(51) Int. Cl.: C12N 15/10, C12N 15/113, C12N 9/78, C12N 9/22, C12N 15/63, C12N 9/12, C12N 15/86

(54) **CLEAVAGE-INACTIVE CAS12F1, CLEAVAGE-INACTIVE CAS12F1-BASED FUSION PROTEIN, CRISPR GENE-EDITING SYSTEM COMPRISING SAME, AND PREPARATION METHOD AND USE THEREOF**

(30) Priority: 05.07.2021 KR 20210087956; 06.10.2021 KR 20210132306; 17.12.2021 KR 20210181875
(71) Applicant: GENKORE INC., Yuseong-gu Daejeon 34141 (KR)
(72) Inventor: KIM, Yong-Sam, Daejeon 34118 (KR); KIM, Do Yon, Daejeon 34069 (KR)
(74) Representative: Dehns
(86) International application number: PCT/KR2022/009701
(87) International publication number: WO 2023/282597

(57) **Abstract**

Disclosed in the present specification are: dead Cas12f1 having the nucleic acid cleavage activity removed; and a fusion protein in which a functional domain is fused to the dead Cas12f1. The dead Cas12f1 and the dCas12f1-based fusion protein may: form a CRISPR gene-editing system together with a guide RNA; and exhibit various functions relating to a target gene such as base editing and expression control.

## Description

### Technical Field

The present disclosure relates to an engineered CRISPR/Cas12f1 system. In particular, the present disclosure relates to a cleavage-inactive Cas12f1 (dead Cas12f1) protein and a fusion protein in which an additional domain is fused to the dead Cas12f1 protein. Specifically, the fusion protein includes a CRISPR base editing complex that has a base editing function for a target gene, and a CRISPR expression regulating complex that has an expression regulating function for a target gene.

### Background Art

In addition to applications of the CRISPR/Cas system based on nucleic acid cleavage activity of Cas protein, research is actively conducted to utilize the CRISPR/Cas system in the overall field of gene regulation, such as base editing and gene expression regulation, using its high target-specific binding ability. Here, in order to utilize Cas protein in the technical field such as base editing and gene expression regulation, it is necessary to use a dead form thereof with nucleic acid cleavage activity removed. In addition, it is necessary to add an appropriate functional domain to the dead form of Cas protein, thereby producing a fusion protein capable of achieving a desired effect.

Meanwhile, dead Cas9 protein, which is mainly used for a CRISPR regulation system, is large in size, making it difficult to create a fusion protein for the CRISPR regulation system and package the same in a vector such as AAV to be delivered to cells. To solve this problem, solutions are being sought through efforts such as splitting Cas9 protein so that it can be delivered into cells using several vectors, and developing and applying relatively small Cas proteins.

### Disclosure of Invention

### Technical Problem

The present specification intends to provide a dead Cas12f1 protein.

The present specification intends to provide a dCas12f1-base editing fusion protein in which a dead Cas12f1 protein and a base editing domain are fused together.

The present specification intends to provide a dCas12f1-expression regulating fusion protein in which a dead Cas12f1 protein and a gene expression regulatory domain are fused together.

The present specification intends to provide a guide RNA that can form a CRISPR gene regulation system together with an engineered Cas12f1 protein (including the dead Cas12f1 protein, dCas12f1-base editing fusion protein, and/or dCas12f1-expression regulating fusion protein).

The present specification intends to provide a CRISPR gene regulating complex (system) comprising the engineered Cas12f1 protein and the guide RNA.

The present specification intends to provide a vector capable of expressing respective components of the CRISPR gene regulation system.

The present specification intends to provide an engineered CRISPR/Cas12f1 composition.

The present specification intends to provide a gene regulation method utilizing the CRISPR gene regulation system.

The present specification intends to provide a use of the CRISPR gene regulation system.

### Solution to Problem

Disclosed herein is a wild-type Cas12f1-based dead Cas12f1 protein, represented by the following amino acid sequence:
MAKNTITKTLKLRIVRPYNSAEVEKIVADEKNNREKIALEKNKDKVKE ACSKHLKVAAYCTTQVERNACLFCKARKLDDKFYQKLRGQFPDAVFWQEISE IFRQLQKQAAEIYNQSLIELYYEIFIKGKGX₁ANASX₂VEHYLSDVCYTRAAELF KNAAIASGLRSKIKSNFRLKELKNMKSGLPTTKSDNFPIPLVKQKGGQYTGFEI SNHNSDFIIKIPFGRWQVKKEIDKYRPWEKFDFEQVQKSPKPISLLLSTQRRKR NKGWSKDEGTEAEIKKVMNGDYQTSYIEVKRGSKIGEKSAWMLNLSIDVPKI DKGVDPSIIGGIX₃VGVKSPLVCAINNAFSRYSISDNDLFHFNKKMFARRRILLK KNRHKRAGHGAKNKLKPITILTEKSERRFKKLIERWACEIADFFIKNKVGTVQ MX₄NLESMKRKEDSYFNIRLRGFWPYAEMQNKIEFKLKQYGIEIRKVAPNNTS KTCSKCGHLNNYFNFEYX₅KKNKFPHFKCEKCNFKENAX₆YNAALNISNPKLK STKEEP (SEQ ID NO: 9), wherein X₁ is isoleucine or tryptophan, X₂ is serine or tyrosine, X₃ is aspartic acid, alanine, glutamine, leucine, tryptophan, or valine, X₄ is glutamic acid, alanine, glutamine, leucine, tryptophan, or valine, X₅ is arginine, alanine, glutamine, leucine, tryptophan, or valine, and X₆ is aspartic acid, alanine, glutamine, leucine, tryptophan, or valine, and
at least one of X₃, X₄, X₅, and X₆ is alanine, glutamine, leucine, tryptophan, or valine.

Disclosed herein is a dead Cas12f1 protein, comprising:
a dummy portion comprising 20 to 30 amino acids; and
the wild-type Cas 12f1-based dead Cas12f1 protein,
wherein the dummy portion is represented by an amino acid sequence selected from MGEKSSRRRRNGKSGAWTAAITSCVGGK (SEQ ID NO: 10), MAGGPGAGSAAPVSSTSSLPLAALNMRV (SEQ ID NO: 11), MAGGPGAGSAAPVSSTSSLPLAALNM (SEQ ID NO: 12), and MEKRINKIRKKLSADNATKPVSRSGP (SEQ ID NO: 13), and
the dummy portion and the wild-type Cas 12f1-based dead Cas12f1 protein are sequentially linked to each other in a direction from the N terminus to the C terminus of the dead Cas12f1 protein.

In an embodiment, the dead Cas12f1 protein may be represented by an amino acid sequence selected from SEQ ID NOS: 2 to 8, SEQ ID NOS: 15 to 24, SEQ ID NOS: 26 to 29, SEQ ID NOS: 31 to 34, and SEQ ID NOS: 36 to 39.

Disclosed herein is a dCas 12f1-base editing fusion protein, comprising: the dead Cas12f1 protein; and deaminase, wherein the deaminase has an amino acid sequence selected from SEQ ID NO: 245, SEQ ID NOS: 247 to 249, and SEQ ID NOS: 274 to 283.

In an embodiment, the deaminase may have an amino acid sequence selected from SEQ ID NOS: 274 to 279.

In an embodiment, the deaminase may be fused to the N-terminus and/or C-terminus of the dead Cas12f1 protein.

In an embodiment, the dCas12f1-base editing fusion protein may have an amino acid sequence selected from SEQ ID NOS: 284 to 324 and SEQ ID NO: 418 to 442.

In an embodiment, the dCas12f1-base editing fusion protein may further comprise at least one uracil glycosylase inhibitor (UGI), the deaminase may have an amino acid sequence selected from SEQ ID NOS: 280 to 283, and the at least one UGI may be fused to the dead Cas12f1 protein.

In an embodiment, the deaminase may be fused to the N terminus (C-terminus) of the dead Cas12f1 protein, and the at least one UGI may be fused to the C terminus (N terminus) of the dead Cas 12f1 protein.

In an embodiment, the dCas12f1-base editing fusion protein may be represented by an amino acid sequence selected from SEQ ID NOS: 325 to 328.

In an embodiment, the dead Cas12f1 protein and the deaminase may be linked via a linker, and the linker may be represented by an amino acid sequence selected from SEQ ID NOS: 260 to 273.

In an embodiment, the dCas12f1-base editing fusion protein may further comprise at least one nuclear localization signal (NLS), and the NLS may be located at the N-terminus, the C-terminus, or both termini.

Disclosed herein is a dCas 12f1-expression regulating fusion protein, comprising: the dead Cas 12f1 protein; and at least one expression regulatory domain, each of which is selected from VP64, KRAB, MeCP2, DNMT, HDAC, Tet1, and p300.

In an embodiment, the expression regulatory domains may each independently be represented by an amino acid sequence selected from SEQ ID NOS: 329 to 333.

In an embodiment, the expression regulatory domain may be located at the N-terminus and/or C-terminus of the dead Cas12f1 protein.

In an embodiment, the dCas12f1-expression regulating fusion protein may be represented by an amino acid sequence selected from SEQ ID NOS: 511 to 521.

Provided herein is provided an engineered CRISPR/Cas12f1 composition, comprising: an engineered Cas12f1 protein selected from the dead Cas12f1 protein, the dCas12f1-base editing fusion protein, and the dCas12f1-expression regulating fusion protein, or a nucleic acid encoding the engineered Cas12f1 protein; and at least one guide RNA, or a nucleic acid encoding the guide RNA, wherein each guide RNA comprises a scaffold, a spacer, and a U-rich tail, the scaffold, the spacer, and the U-rich tail are sequentially linked to each other in a 5' to 3' direction, the scaffold is represented by a nucleotide sequence selected from SEQ ID NOS: 197 to 199, the U-rich tail is represented by a nucleotide sequence of (UₐN)_{b}U_{c}, where N is each independently selected from A, U, C, and G, a is an integer of between 1 to 5 inclusive, and b is an integer of 0 or more, and the spacer comprises nucleosides of between 10 and 50 inclusive and has a nucleotide sequence complementary to a predetermined target sequence.

In an embodiment, the engineered CRISPR/Cas12f1 composition may comprise the engineered Cas12f1 protein and the guide RNA in a form of a ribonucleoprotein (RNP).

In an embodiment, the engineered CRISPR/Cas12f1 composition may comprise the nucleic acid encoding the engineered Cas12f1 protein and the nucleic acid encoding the guide RNA in a form of a vector.

In an embodiment, the vector may comprise the nucleic acid encoding the engineered Cas12f1 protein, a nucleic acid encoding a first guide RNA, and a nucleic acid encoding a second guide RNA, and a nucleotide sequence of a spacer in the first guide RNA and a nucleotide sequence of a spacer in the second guide RNA may be different from each other.

Disclosed herein is a method for base editing of a target gene in a cell, comprising: introducing the CRISPR/Cas12f1 composition, which comprises the dCas12f1-base editing protein, into a living cell, wherein the target gene in the cell is double-stranded DNA comprising a target strand and a non-target strand, the target strand has a target sequence, the non-target strand has a protospacer adjacent motif (PAM) and a protospacer, the protospacer is a nucleotide sequence of 10 to 50 nts which is complementary to the target sequence, a spacer in the guide RNA of the engineered CRISPR/Cas12f1 composition is capable of hybridizing with the target sequence in the target strand, and the introduction of the engineered CRISPR/Cas12f1 composition into the cell causes a CRISPR-base editing complex to be formed in the cell, and the CRISPR-base editing complex replaces at least one adenine in the protospacer with guanine.

In an embodiment, the protospacer in the target gene may contain at least one adenine at 2nd, 3rd, 4th, 5th, 6th, 7th, 8th, 9th, 15th, 16th, 17th, 18th, 19th, and 20th positions from the 5' end, and performing the method for base editing may result in replacement of at least one of the adenines at the 2nd, 3rd, 4th, 5th, 6th, 7th, 8th, 9th, 15th, 16th, 17th, 18th, 19th, and 20th positions with guanine.

Provided herein is a method for base editing of a target gene in a cell, comprising: introducing the engineered CRISPR/Cas12f1 composition, which comprises the dCas12f1-base editing protein, into the cell, wherein the target gene in the cell is double-stranded DNA comprising a target strand and a non-target strand, the target strand has a target sequence, the non-target strand has a protospacer adjacent motif (PAM) and a protospacer, the protospacer is a nucleotide sequence of 10 to 50 nts which is complementary to the target sequence, a spacer in the guide RNA of the engineered CRISPR/Cas12f1 composition is capable of hybridizing with the target sequence in the target strand, and the introduction of the engineered CRISPR/Cas12f1 composition into the cell causes a CRISPR-base editing complex to be formed in the cell, and the CRISPR-base editing complex replaces at least one cytosine in the protospacer with thymine.

In an embodiment, the protospacer in the target gene contains at least one cytosine at 2nd, 3rd, 4th, 5th, 6th, 7th, 8th, and 9th positions from the 5' end, and performing the method for base editing results in replacement of at least one of the cytosines at the 2nd, 3rd, 4th, 5th, 6th, 7th, 8th, and 9th positions with thymine.

In an embodiment, the cell may be a eukaryotic cell.

Provided herein is a method for regulating expression of a target gene in a cell, comprising: introducing the engineered CRISPR/Cas12f1 composition, which comprises the dCas12f1-expression regulating fusion protein, into the cell, wherein the introduction of the engineered CRISPR/Cas12f1 composition into the cell causes a CRISPR gene regulating complex to be formed, and the CRISPR gene regulating complex regulates expression of the target gene.

In an embodiment, the cell may be a eukaryotic cell.

### Advantageous Effects of Invention

The CRISPR gene regulating complex and its components disclosed herein can exhibit various gene function regulation effects, such as base editing for a target gene in a cell and expression regulation (promotion or inhibition) for a target gene. In addition, it is possible to load the CRISPR gene regulation system onto a single unit of adeno-associated virus (AAV) vector, from which high efficiency and usability can be expected.

### Brief Description of Drawings

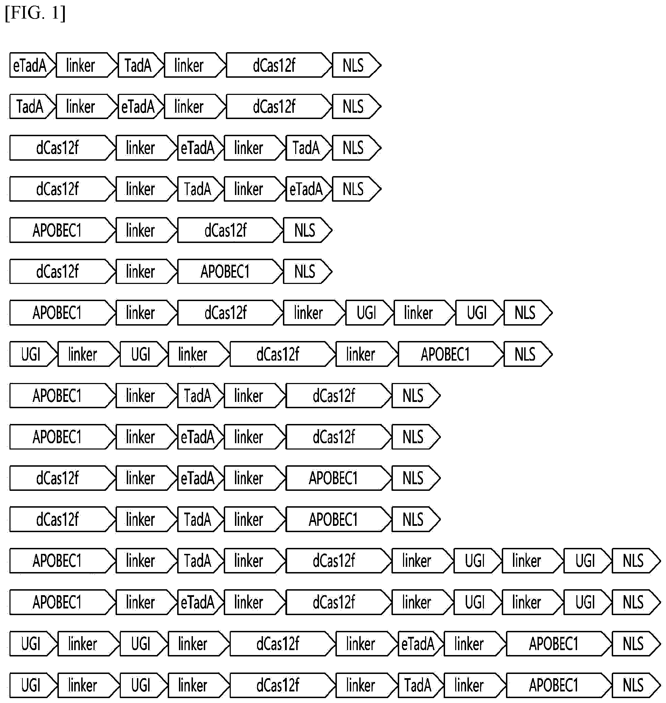

FIG. 1 illustrates a schematic diagram for various examples of hypercompact base editing constructs for base editing according to the present disclosure.

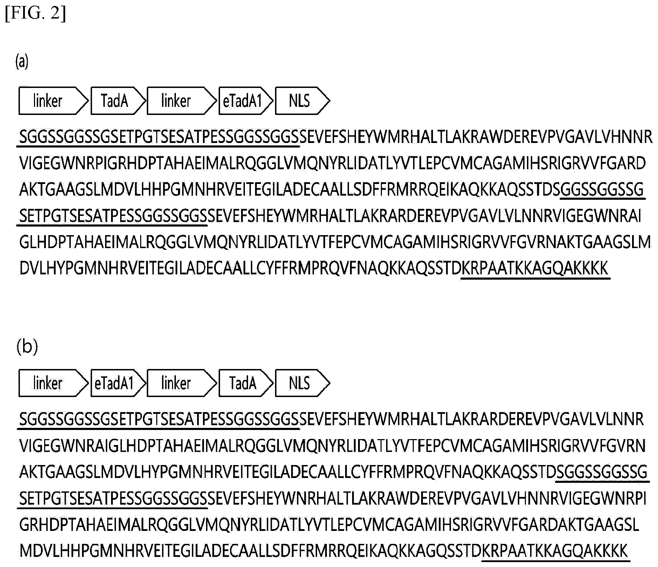

FIG. 2 illustrates heterodimeric structures of adenosine deaminase and amino acid sequences thereof, included in the hypercompact base editing construct for base editing provided by the present disclosure. (a) shows a structure of the adenosine deaminase TadA-eTadA1. The structure has a configuration linked in the order of linker-TadA-linker-eTadA1-NLS, and the amino acid sequences for TadA, eTadA1, linker, and NLS are shown. (b) shows a structure of the adenosine deaminase eTadA1-Tad. The structure has a configuration linked in the order of linker-eTadA1-linker-TadA-NLS, and the amino acid sequences for TadA, eTadA1, linker, and NLS are shown. Each sequence shown in the drawing is an exemplary sequence, and the present disclosure is not limited thereto.

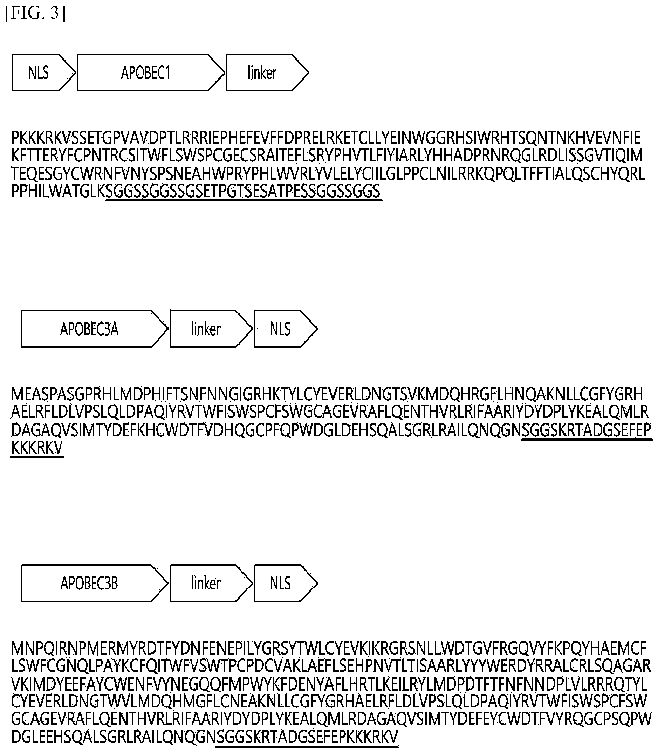

FIG. 3 illustrates cytidine deaminase modules and amino acid sequences thereof, included in the hypercompact base editing constructs for base editing provided by the present disclosure. The structure at the top represents a module structure for the cytidine deaminase APOBEC1. The structure has a configuration linked in the order of NLS-APOBEC1-linker, and the amino acid sequences for APOBEC1, linker, and NLS are shown. The first methionine (M) in APOBEC1 has been omitted. The structure in the middle represents a module structure for the cytidine deaminase APOBEC3A. The structure has a configuration linked in the order APOBEC3A-linker-NLS, and the amino acid sequences for APOBEC3A, linker, and NLS are shown. The structure at the bottom represents a module structure for the cytidine deaminase APOBEC3B. The structure has a configuration linked in the order APOBEC3BLinker-NLS, and the amino acid sequences for APOBEC3B, linker, and NLS are shown. Each sequence shown in the drawing is an exemplary sequence, and the present disclosure is not limited thereto.

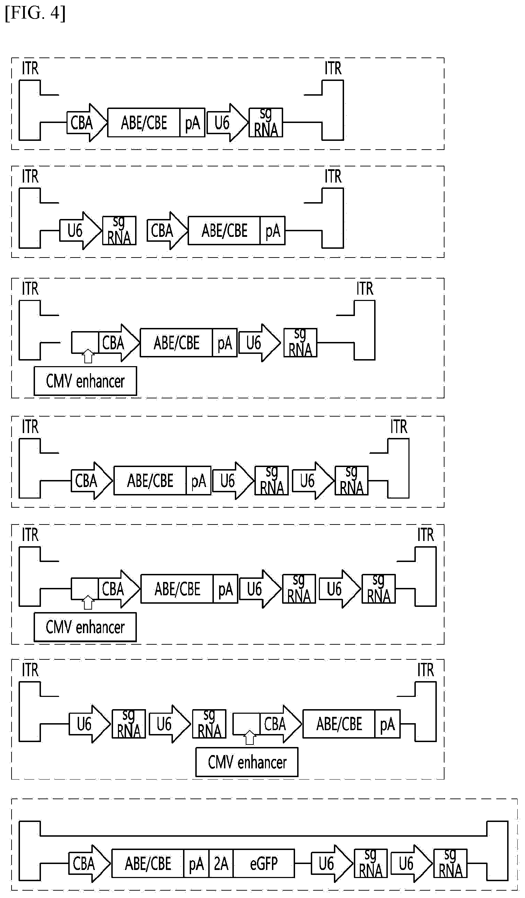

FIG. 4 illustrates structures of exemplary adeno-associated virus (AAV) vectors provided by the present disclosure.

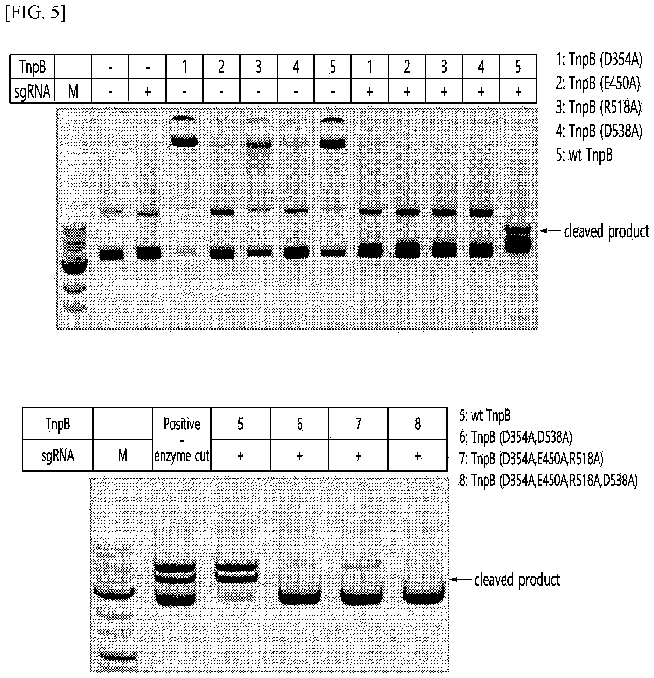

FIG. 5 illustrates results obtained by identifying nucleic acid cleavage activity of the dead-form of Cas12f1 variant 1 (TnpB) provided by the present disclosure. Here, TnpB (D354A) represents dead Cas12f1 of SEQ ID NO: 15, TnpB (D354A) represents dead Cas12f1 of SEQ ID NO: 16, TnpB (D354A) represents dead Cas12f1 of SEQ ID NO: 17, TnpB (D354A) represents dead Cas12f1 of SEQ ID NO: 19, TnpB (D354A) represents dead Cas12f1 of SEQ ID NO: 22, TnpB (D354A) represents dead Cas12f1 of SEQ ID NO: 23, and TnpB (D354A) represents dead Cas12f1 of SEQ ID NO: 24. wt TnpB is a control and represents the Cas12f1 variant protein of SEQ ID NO: 14.

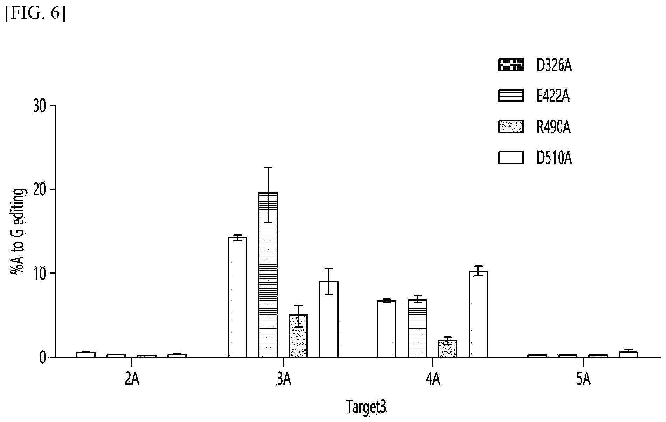

FIG. 6 illustrates base editing efficiency of CRISPR base editing complexes depending on the type of dead Cas12f1 included in dCas12f1-base editing protein. Here, the target protospacer sequence was Target-3, and the guide RNA used was v4.1. Here, D326A, E422A, R490A, and D510Arepresent the dCas12f1-base editing fusion proteins of SEQ ID NO: 287, SEQ ID NO: 292, SEQ ID NO: 293, and SEQ ID NO: 294, respectively.

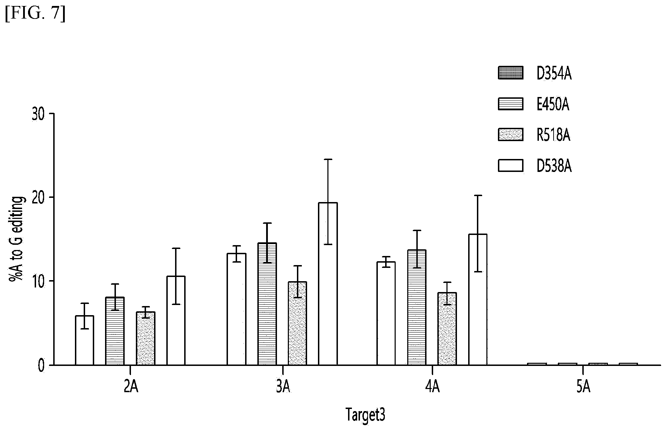

FIG. 7 illustrates base editing efficiency of CRISPR base editing complexes depending on the type of dead Cas12f1 included in dCas12f1-base editing protein. Here, the target protospacer sequence was Target-3, and the guide RNA used was v4.1. Here, D354A, E450A, R518A, and D538A represent the dCas12f1-base editing fusion proteins of SEQ ID NO: 291, SEQ ID NO: 295, SEQ ID NO: 296, and SEQ ID NO: 297, respectively.

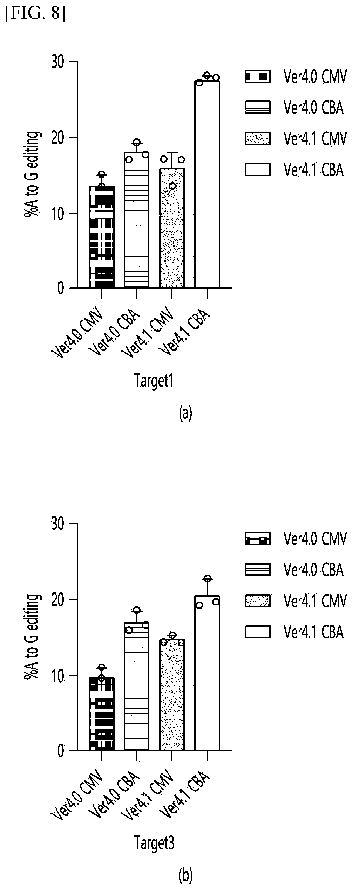

FIG. 8 illustrates experimental results showing base editing efficiency depending on the type of promoter included in expression vectors, according to Experimental Example 3.4. (a) and (b) show results of base editing rate depending on the type of promoter for Target-1 and Target-3, respectively. Here, configurations of the CRISPR base editing complexes for respective labels are as shown in Table 11.

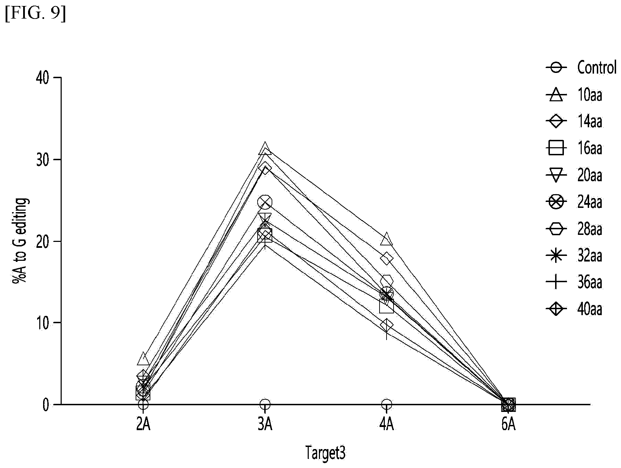

FIG. 9 illustrates experimental results showing base editing efficiency depending on the length of linker, according to Experimental Example 3.5. Here, configurations of the CRISPR base editing complexes for respective labels are as shown in Table 12.

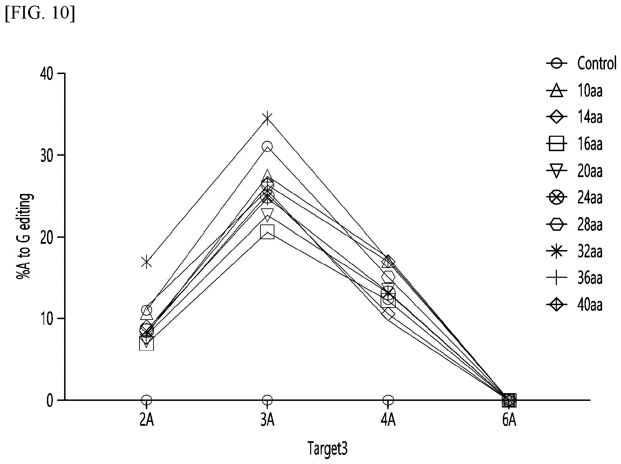

FIG. 10 illustrates experimental results showing base editing efficiency depending on the length of linker, according to Experimental Example 3.5. Here, configurations of the CRISPR base editing complexes for respective labels are as shown in Table 13.

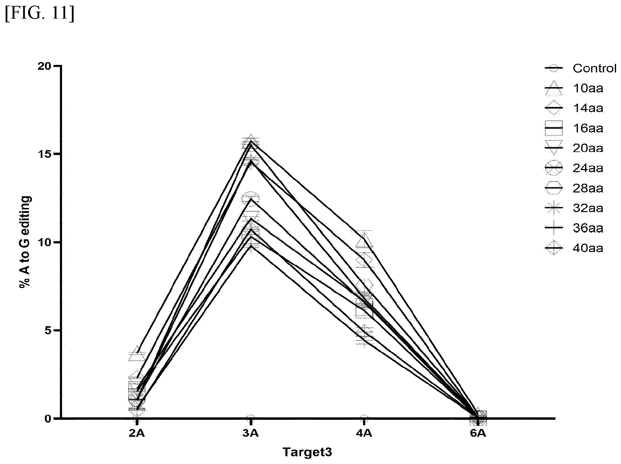

FIG. 11 illustrates experimental results showing base editing efficiency depending on the length of linker, according to Experimental Example 3.5. Here, configurations of the CRISPR base editing complexes for respective labels are as shown in Table 14.

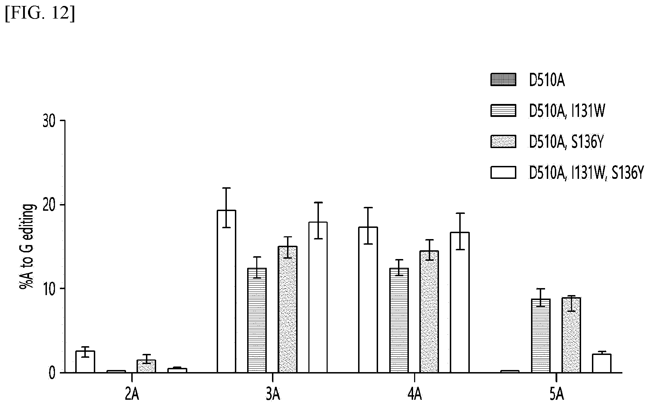

FIG. 12 illustrates results obtained by identifying base editing efficiency of CRISPR base editing systems that comprise various dead Cas12f1 variant-based dCas12f1-base editing proteins, according to Experimental Example 3.6. Here, configurations of the CRISPR base editing complexes for respective labels are as shown in Table 15.

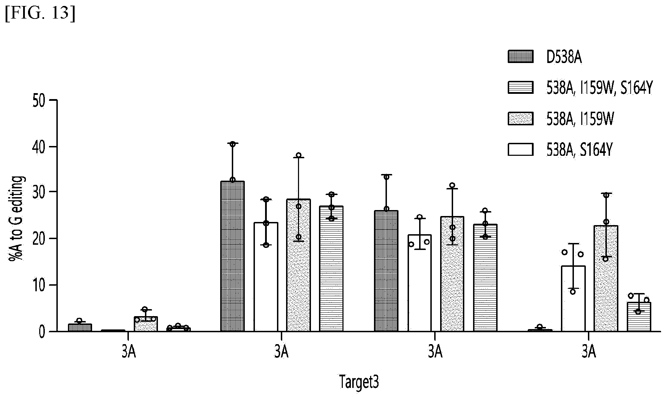

FIG. 13 illustrates results obtained by identifying base editing efficiency of CRISPR base editing systems that comprise various dead Cas12f1 variant-based dCas12f1-base editing proteins, according to Experimental Example 3.6. Here, configurations of the CRISPR base editing complexes for respective labels are as shown in Table 15.

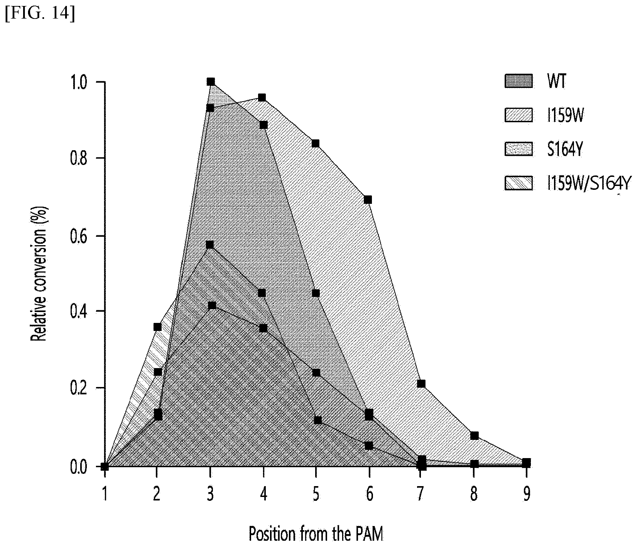

FIG. 14 illustrates results obtained by identifying base editing efficiency of CRISPR base editing systems that comprise various dead Cas12f1 variant-based dCas12f1-base editing proteins, according to Experimental Example 3.6. Here, I159W corresponds to "D538A, I159W" in Table 15, S164Y corresponds to "D538A, S164Y" in Table 15, and I159W/S164Y corresponds to "D538A, I159W, S164Y" in Table 15. WT is a control and represents a CRISPR/Cas12f1 system comprising Cas12f1 variant 1 of SEQ ID NO: 14.

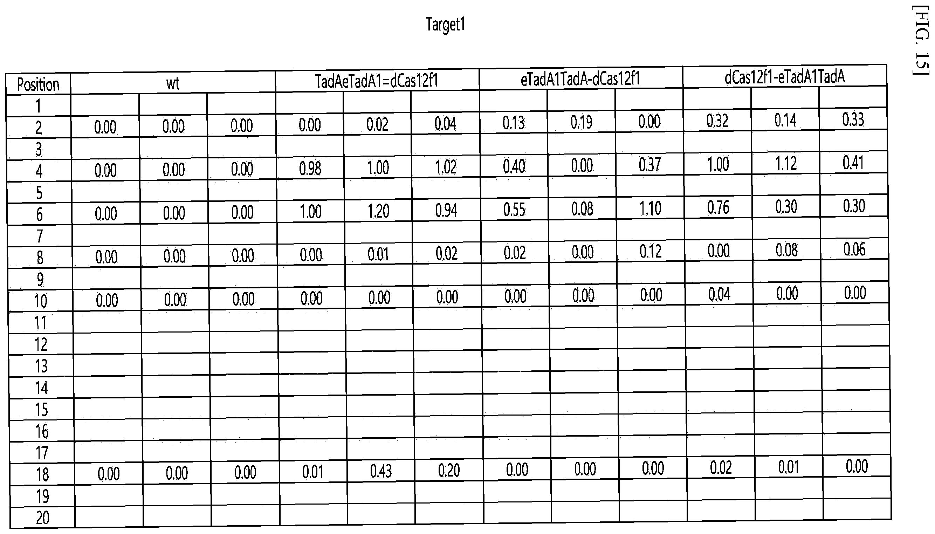

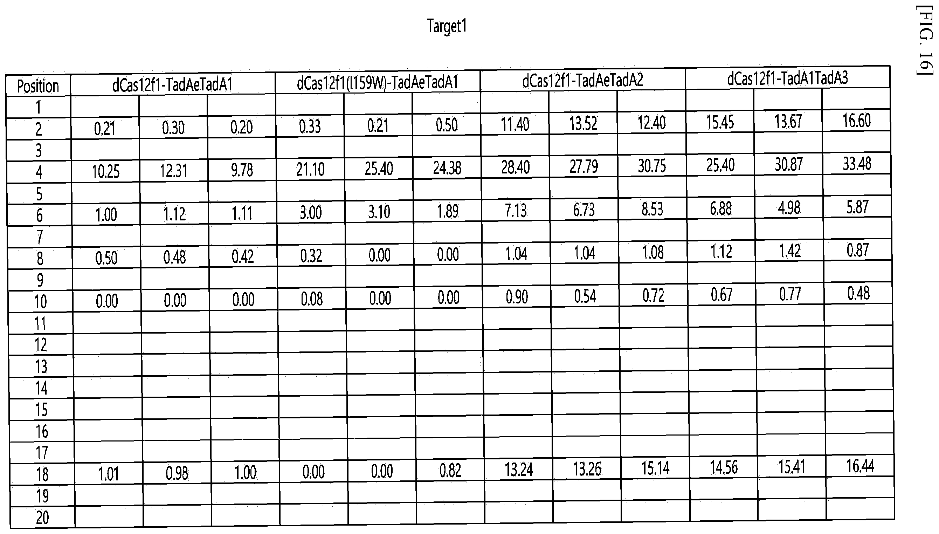

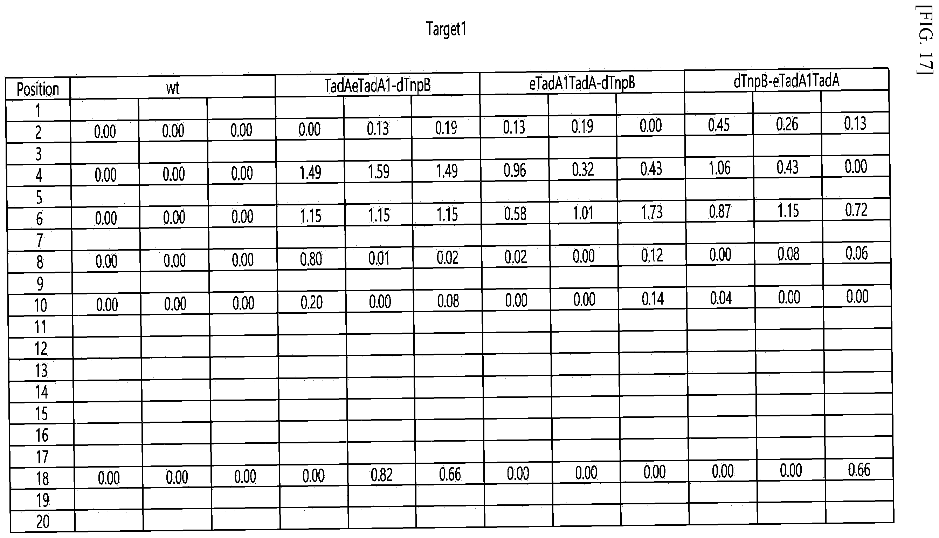

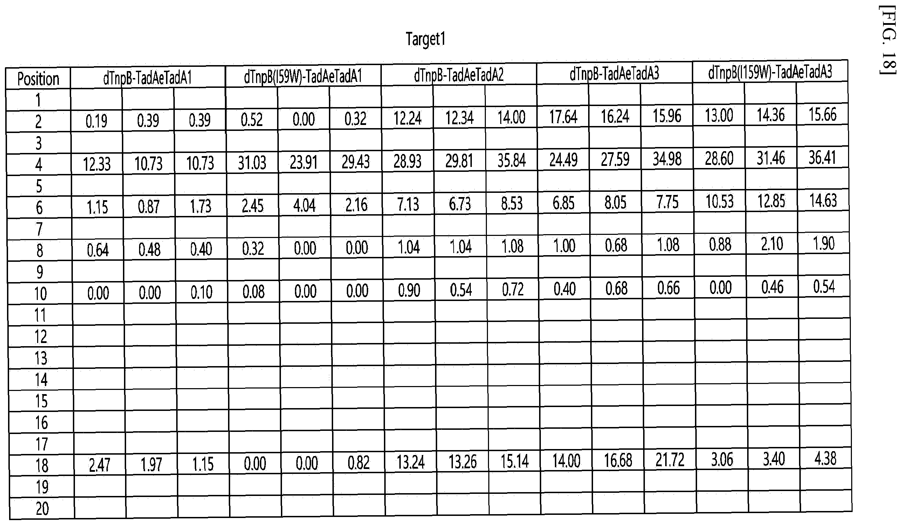

FIGS. 15, 16, 17, and 18 illustrate results obtained by identifying base editing efficiency of CRISPR base editing systems that comprise various dead Cas12f1 variant-based dCas12f1-base editing proteins, according to Experimental Example 3.7. Here, configurations of the CRISPR base editing complexes for respective labels are as shown in Tables 16 and 17.

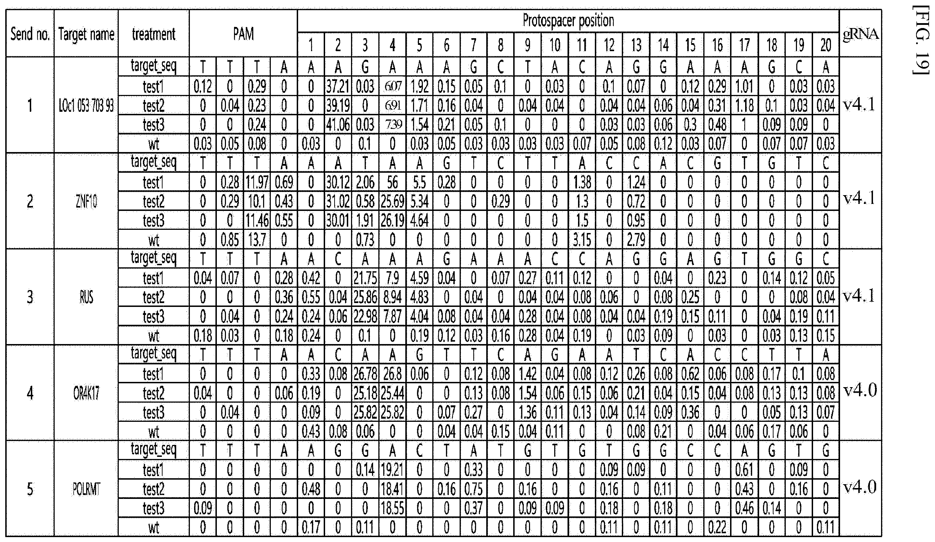

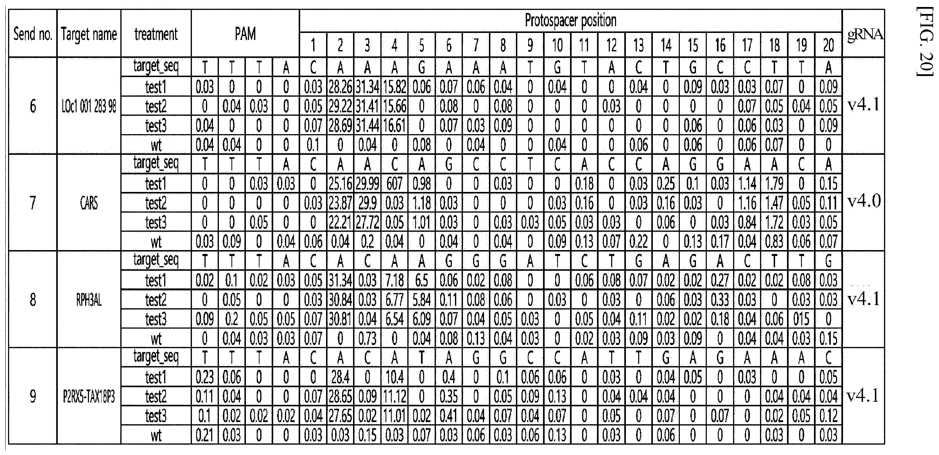

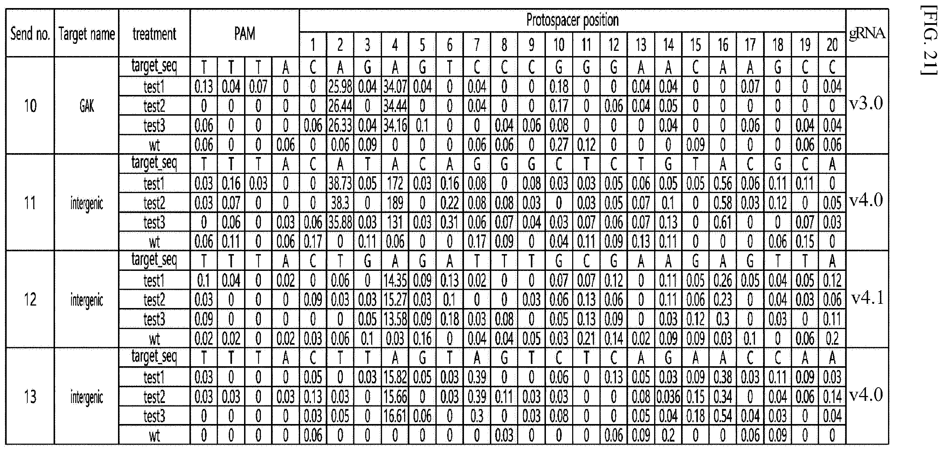

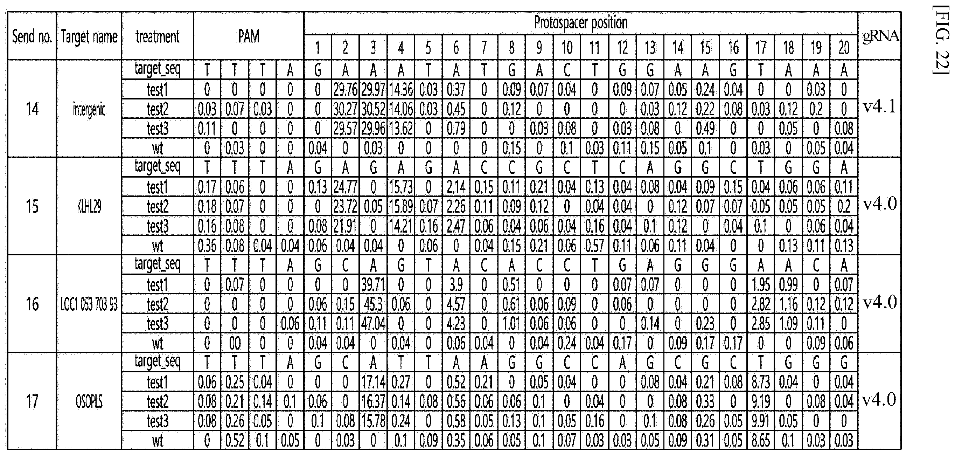

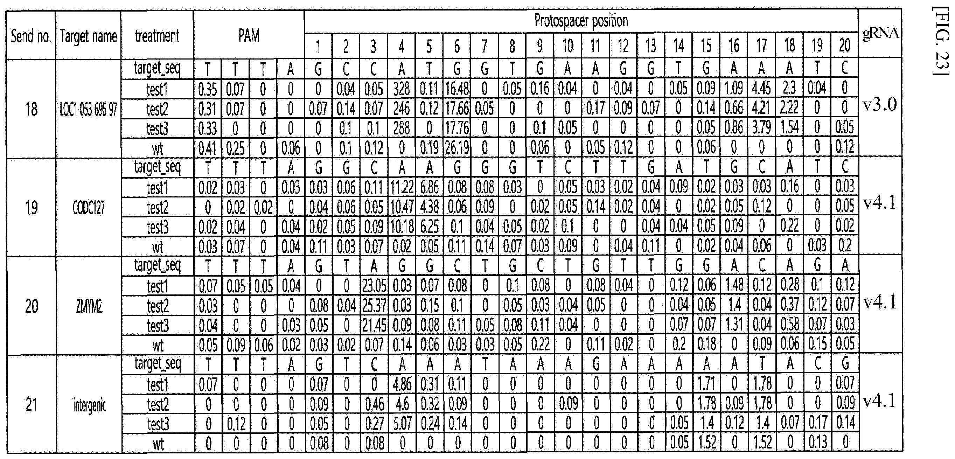

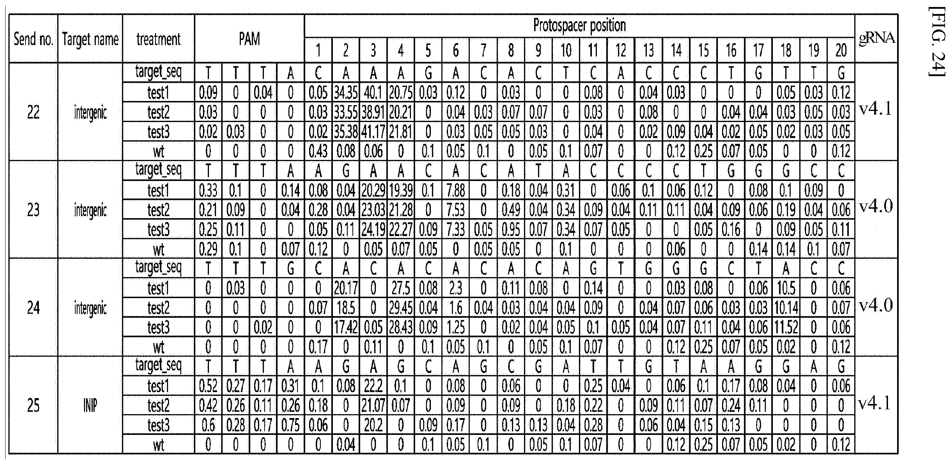

FIGS. 19, 20, 21, 22, 23, and 24 illustrate results obtained by identifying base editing efficiency of CRISPR base editing systems, which target various genes and each of which comprises a dead Cas12f1 variant-based dCas12f1-base editing protein, according to Experimental Example 3.8. Here, the dCas12f1-base editing fusion protein used in the CRISPR base editing systems is SEQ ID NO: 299, and each guide RNA for each target is as shown in the gRNA column of each drawing.

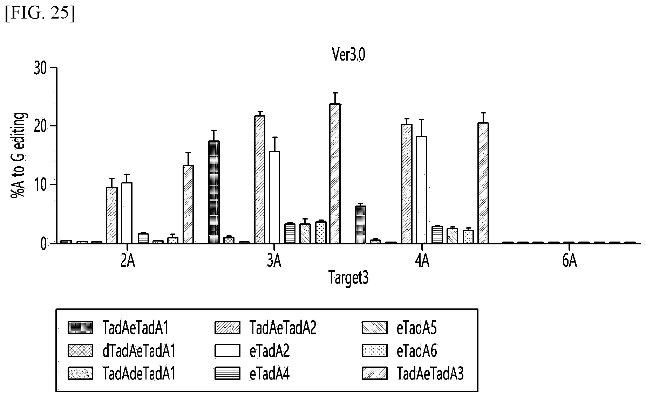

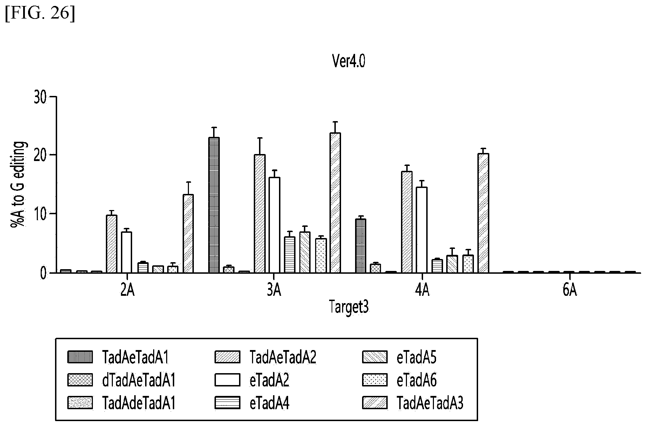

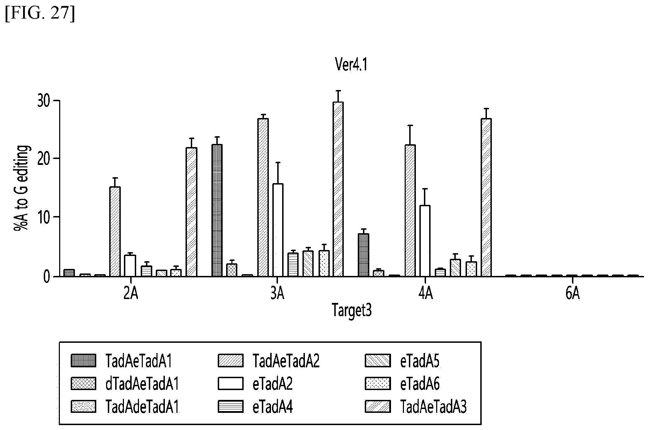

FIGS. 25, 26, and 27 illustrate results obtained by identifying base editing efficiency of CRISPR base editing systems that comprise various base editing domains, according to Experimental Example 3.9. Here, respective labels are as shown in Table 18, and FIGS. 25, 26, and 27 illustrate results obtained by using the guide RNAs v3.0, v4.0, and v4.1, respectively.

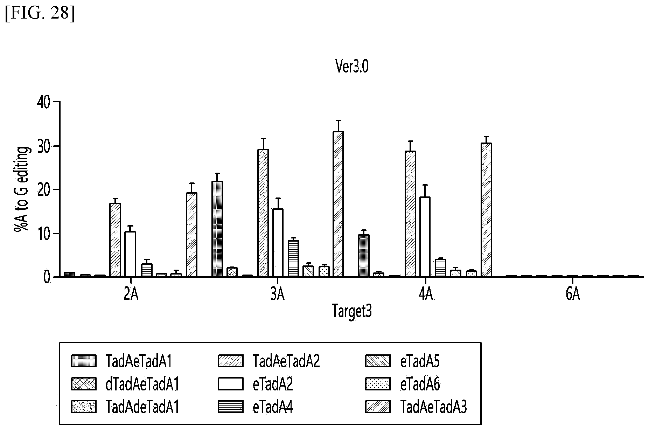

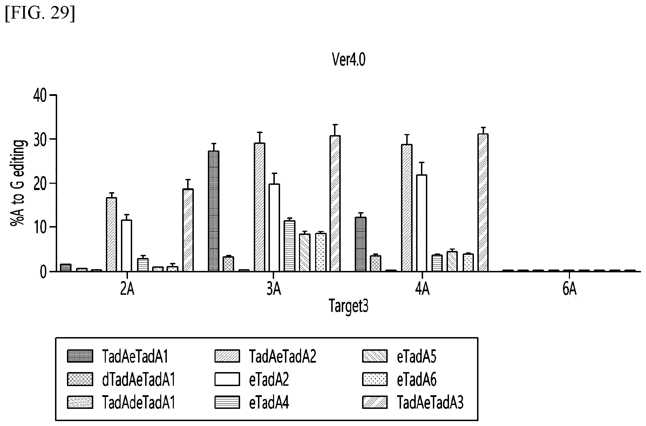

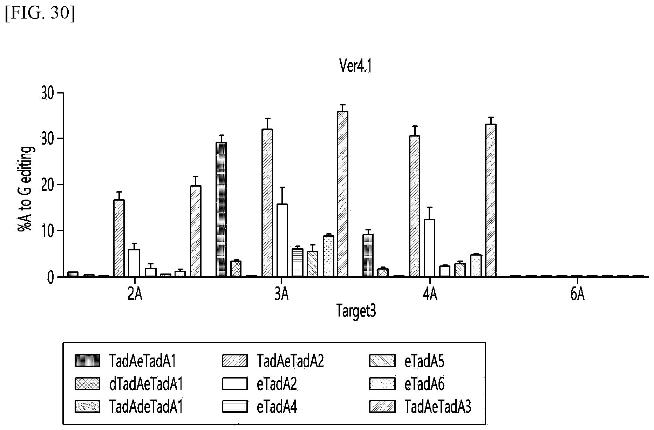

FIGS. 28, 29, and 30 illustrate results obtained by identifying base editing efficiency of CRISPR base editing systems that comprise various base editing domains, according to Experimental Example 3.9. Here, respective labels are as shown in Table 19, and FIGS. 28, 29, and 30 illustrate results obtained by using the guide RNAs v3.0, v4.0, and v4.1, respectively.

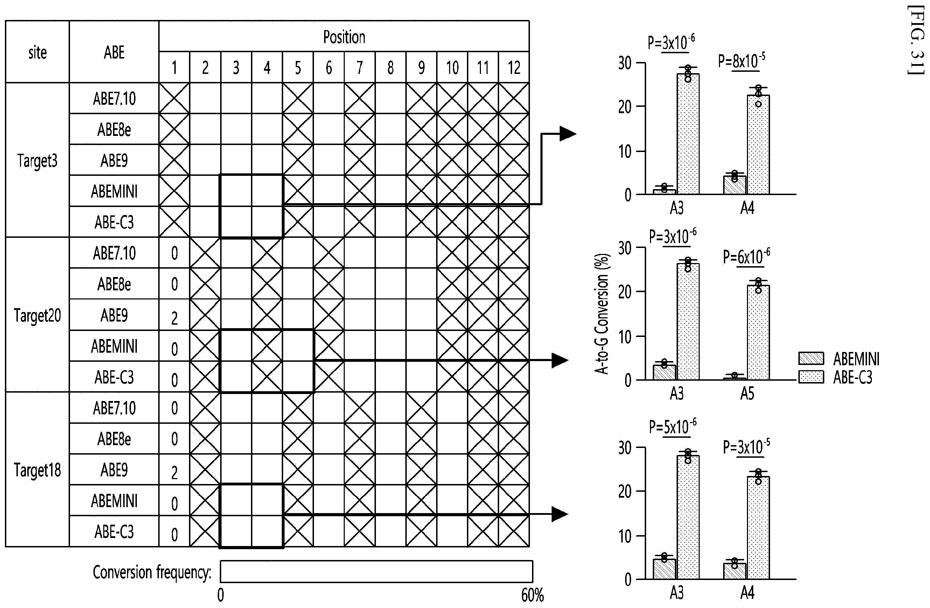

FIG. 31 illustrates results obtained by comparing base editing efficiency of various dead Cas12f1 variant-based dCas12f1-base editing proteins with that of existing Cas9-based base editing systems, according to Experimental Example 3.11. Here, configurations of the CRISPR base editing complexes for respective labels are as shown in Table 22.

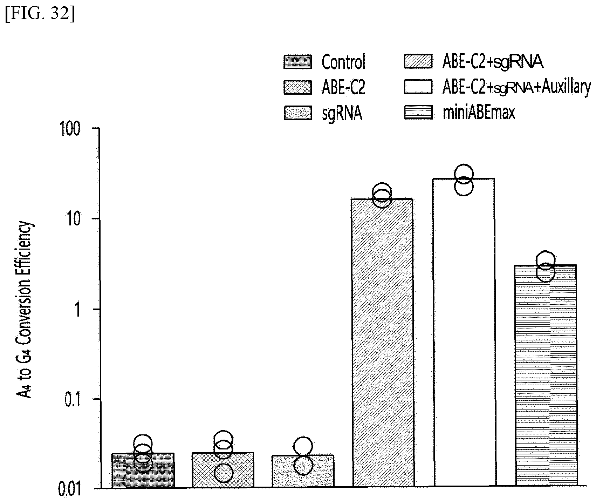

FIG. 32 illustrates results obtained by comparing base editing efficiency of the existing miniABEmax with base editing efficiency of the CRISPR base editing system of the present disclosure, according to Experimental Example 3.12. Here, configurations of the CRISPR base editing complexes for respective labels are as shown in Experimental Example 3.12.

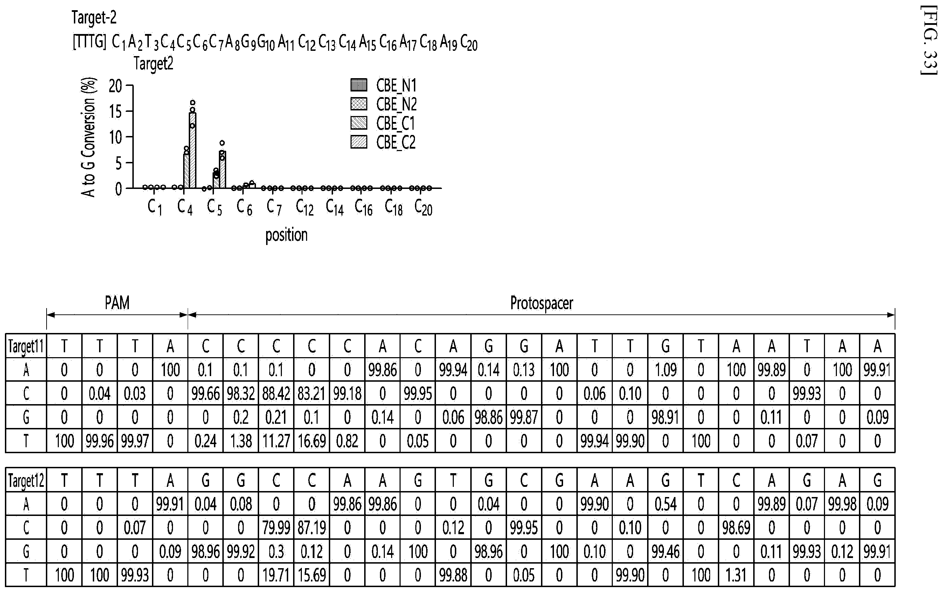

FIG. 33 illustrates results obtained by identifying whether CRISPR base editing complexes, which comprise cytidine deaminase as a base editing domain, exhibit cytosine base editing activity, according to Experimental Example 3.13. Here, configurations of the CRISPR base editing complexes for respective labels are as shown in Table 23.

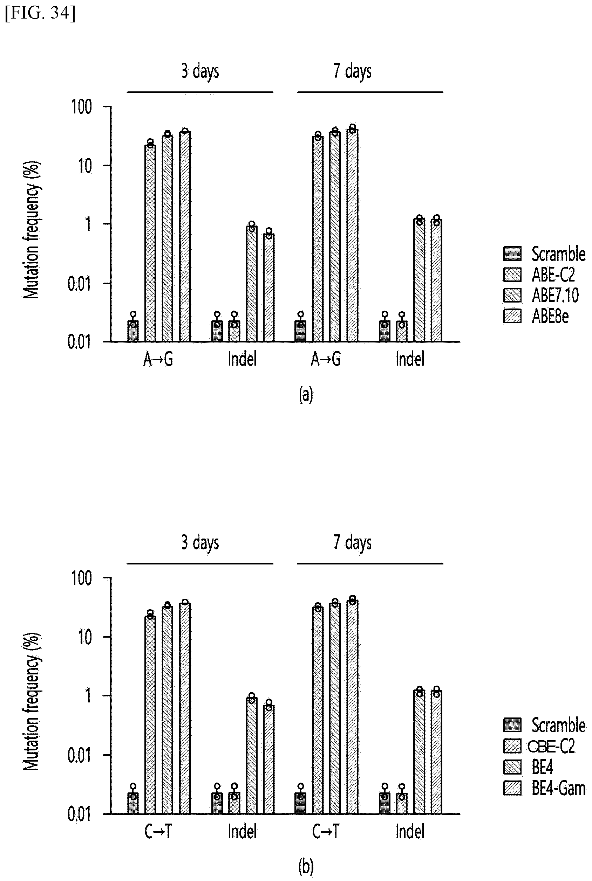

FIG. 34 illustrates results obtained by identifying whether the CRISPR base editing complex of the present disclosure generates unwanted indels, according to Experimental Example 3.14. Here, compositions of the CRISPR base editing complexes for respective labels are as shown in Table 24.

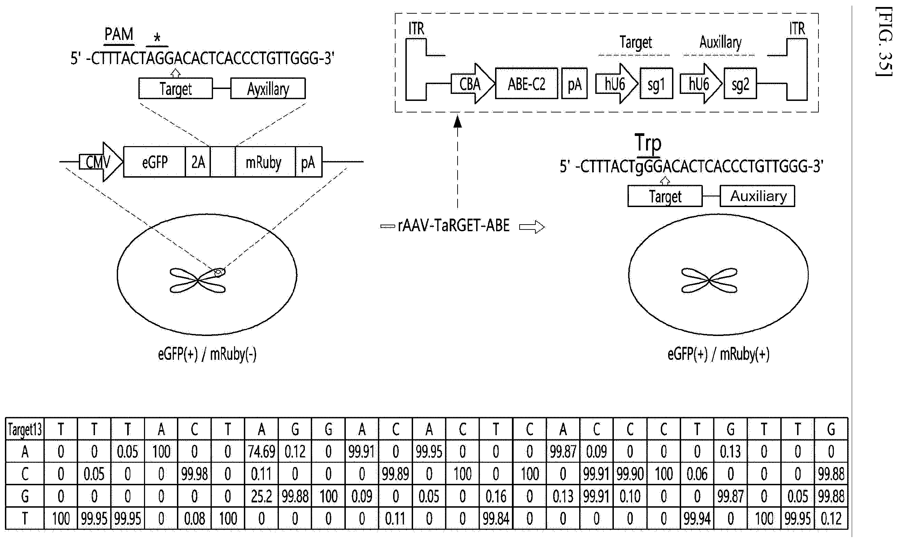

FIG. 35 illustrates results obtained by identifying intracellular base editing rates of an AAV vector loaded with respective components of a CRISPR base editing system, according to Experimental Example 3.15. The schematic diagram on the left schematically shows a transformed cell line. The schematic diagram on the right schematically shows an AAV vector loaded with a CRISPR base editing system comprising the dCas12f1-base editing fusion protein of SEQ ID NO: 287 and a cell that has been transfected with the vector and has undergone base editing. The table at the bottom shows adenine base editing rates at the target protospacer site of the target gene in the cell, achieved by the AAV vector.

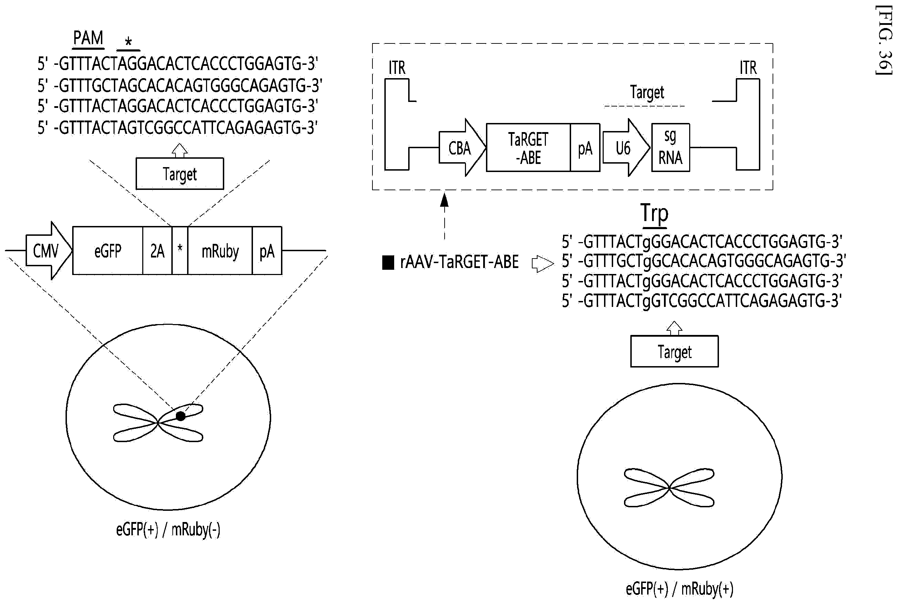

FIG. 36 illustrates results obtained by identifying intracellular base editing rates of an AAV vector loaded with respective components of a CRISPR base editing system, according to Experimental Example 3.15. The schematic diagram on the left schematically shows a transformed cell line. The schematic diagram on the right schematically shows an AAV vector loaded with a CRISPR base editing system comprising the dCas12f1-base editing fusion protein of SEQ ID NO: 291 and a cell that has been transfected with the vector and has undergone base editing. The table at the bottom shows adenine base editing rates at the target protospacer site of the target gene in the cell, achieved by the AAV vector.

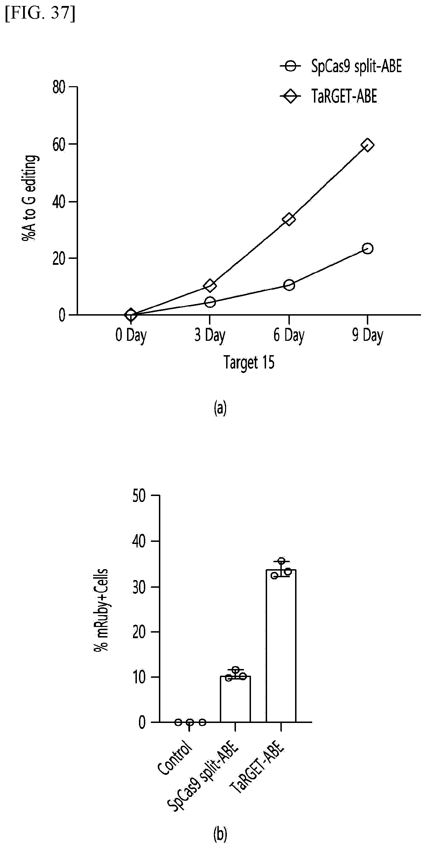

FIG. 37 illustrates results obtained by comparing a base editing effect of an AAV vector loaded with the CRISPR base editing system of the present disclosure with that of SpCas9-ABE split AAV, according to Experimental Example 3.15. The graph at the top shows a base editing rate over time. The graph at the bottom shows a level of expression of mRuby gene caused by base editing.

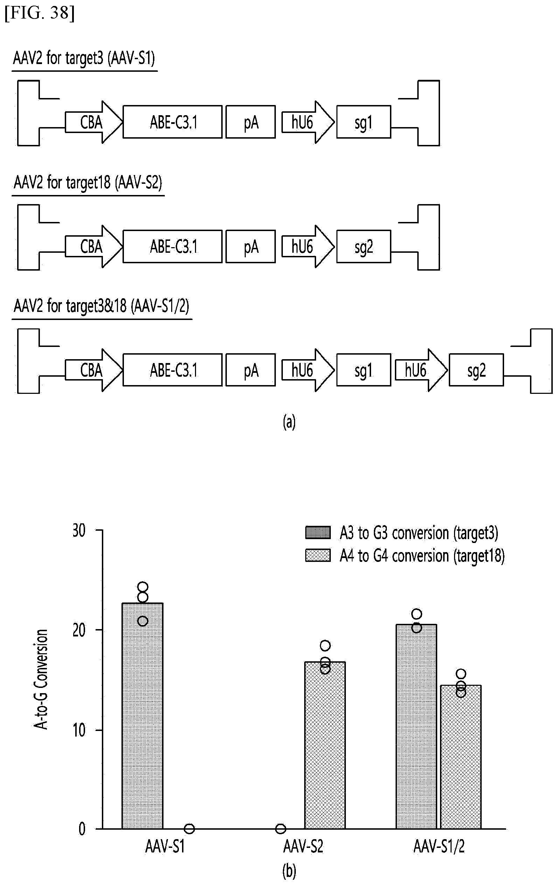

FIG. 38 illustrates results obtained by identifying multiple gene editing caused by delivery of an AAV vector loaded with the CRISPR base editing system of the present disclosure. (a) shows a schematic diagram of the AAV vectors AAV-S1 and AAV-S2, each of which comprises one type of gRNA and the AAV vector AAV-S1/2 that comprises two different types of gRNA. (b) shows results of base editing rates (%) at the target protospacer site in the cell, achieved by the AAV vectors.

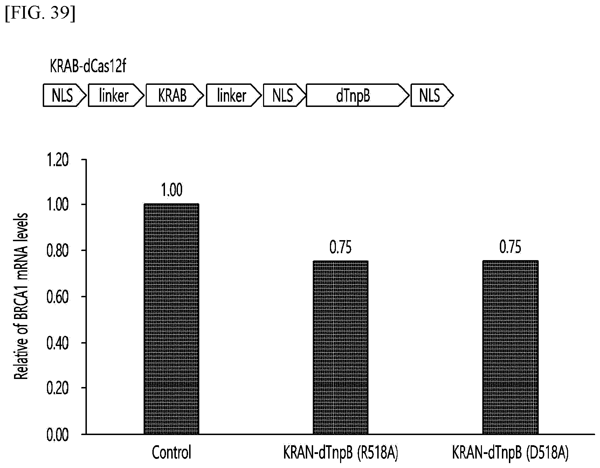

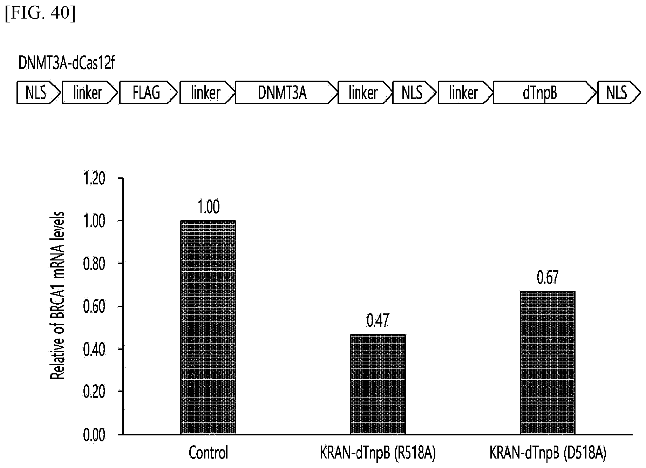

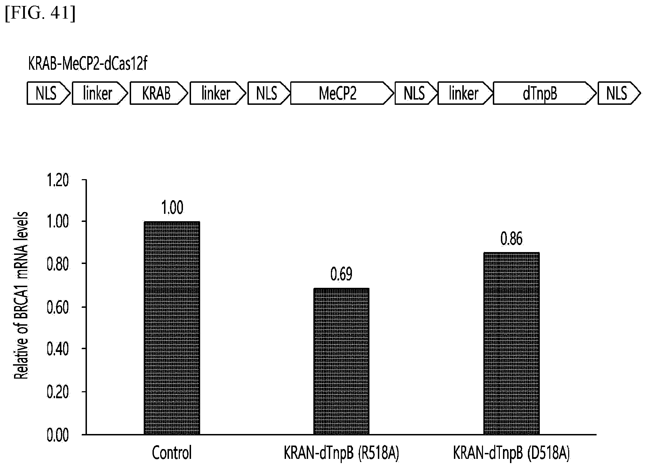

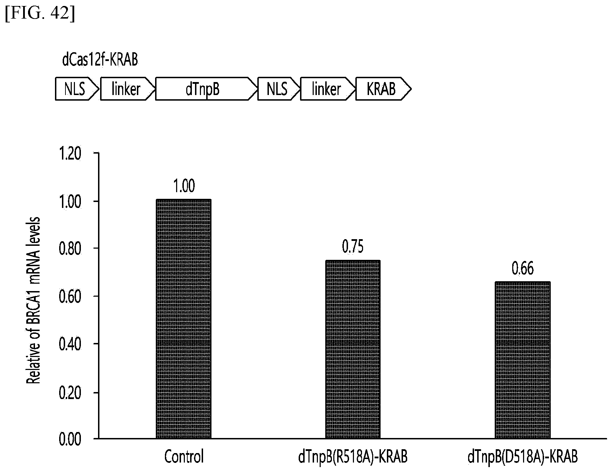

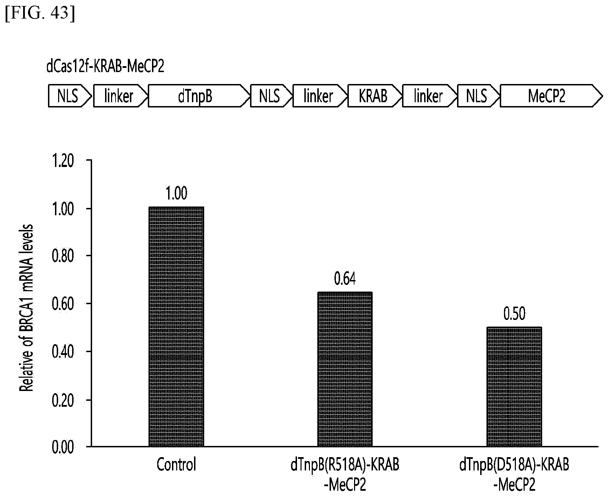

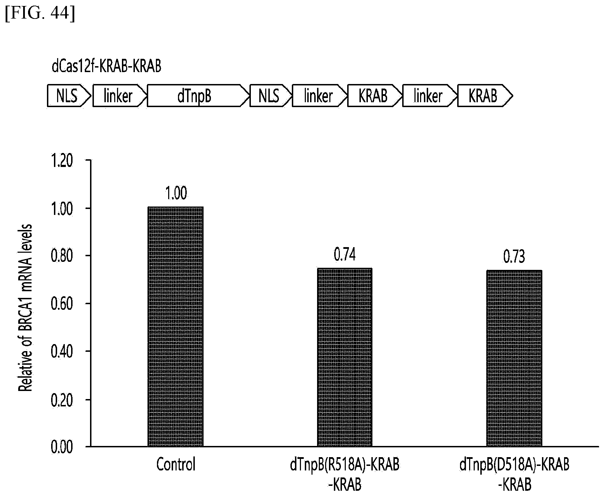

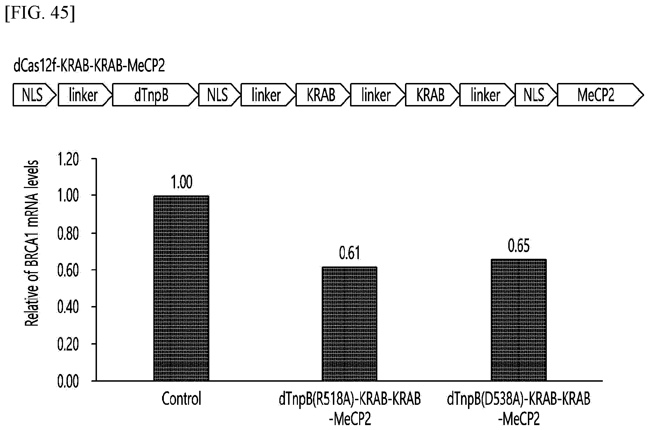

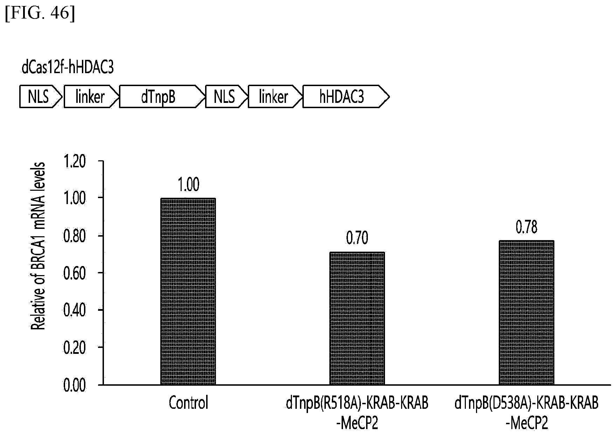

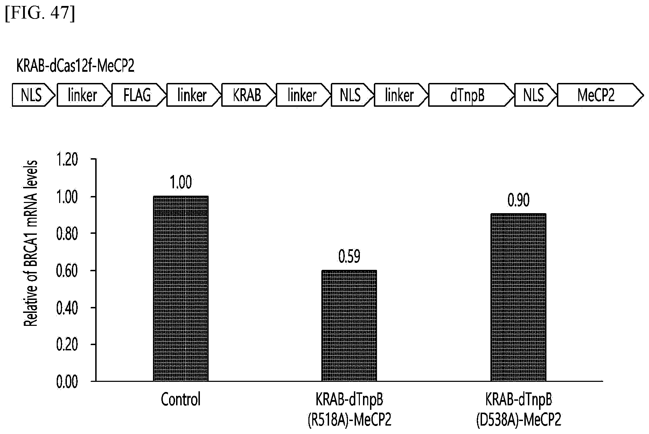

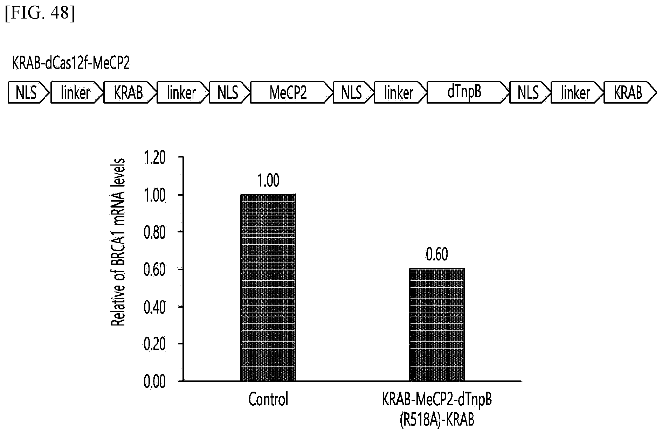

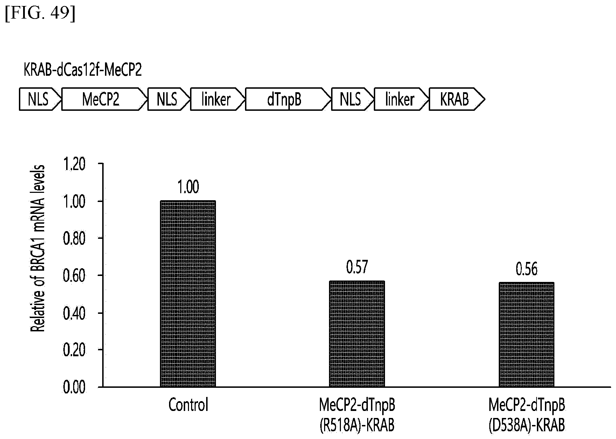

FIGS. 39, 40, 41, 42, 43, 44, 45, 46, 47, 48, and 49 illustrate results obtained by identifying an effect of inhibiting expression of BRCA1 gene, achieved by CRISPR interference systems having an expression inhibition function, among the CRISPR expression regulatory systems of the present disclosure, according to Experimental Example 4.1. Here, each dCas12f1-expression regulating fusion protein included in the CRISPR interference systems is schematically shown at the top of each drawing. Here, KRAB, MeCP2, hHDAC3, and DNMT3A are as described herein, and TnpB is the same as the dead Cas12f1 protein of SEQ ID NO: 17. Based on the experimental results, difference in relative expression level of mRNA of BRCA1 gene is shown in the graph at the bottom.

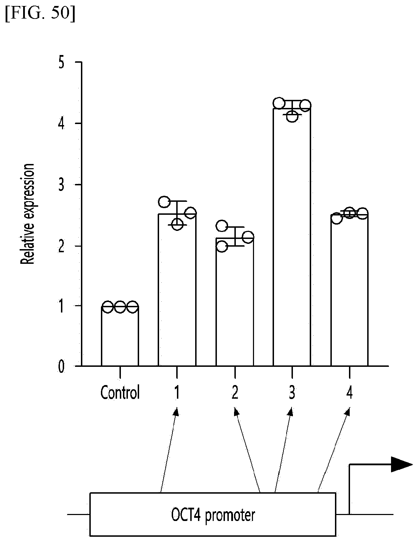

FIG. 50 illustrates results obtained by identifying an effect of promoting expression of OCT4 gene, achieved by CRISPR activation systems having an expression promotion function, among the CRISPR expression regulatory systems of the present disclosure, according to Experimental Example 4.2. Here, 1, 2, 3, and 4 correspond to targeting activation-1, activation-2, activation-3, and activation-4 in Table 28, respectively.

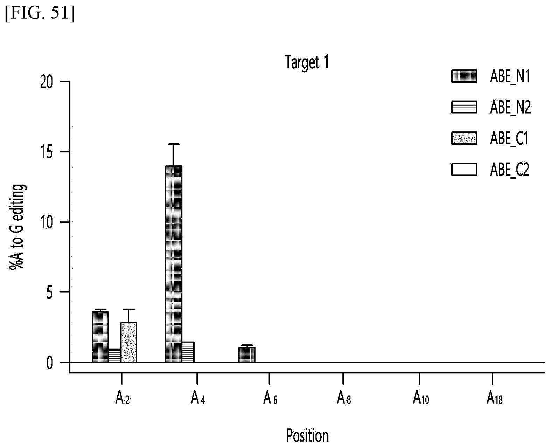

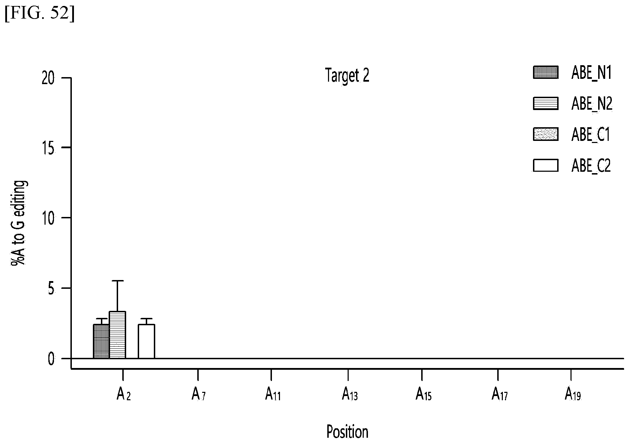

FIGS. 51, 52, 53, and 54 illustrate results obtained by identifying base editing efficiency of CRISPR base editing systems that comprise dCas12f1-base editing proteins of various structures of the present disclosure, according to Experimental Example 3.2. Here, the target protospacer sequences are as shown in Tables 1 and 2. The dCas12f1-base editing fusion proteins are as shown in Table 9. Specifically, ABE_N1, ABE_N2, ABE_C1, and ABE_C2 represent dCas12f1-ABE-N1, dCas12f1-ABE-N2, dCas12f1-ABE-C1, and dCas12f1-ABE-C2, respectively.

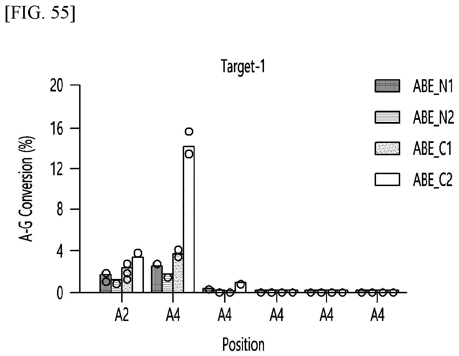

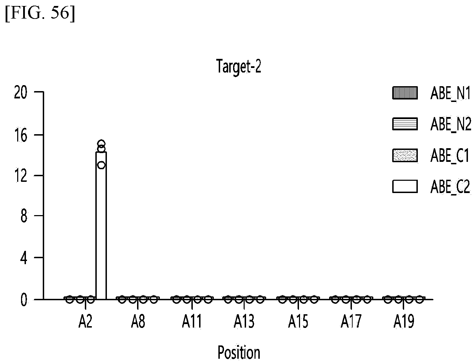

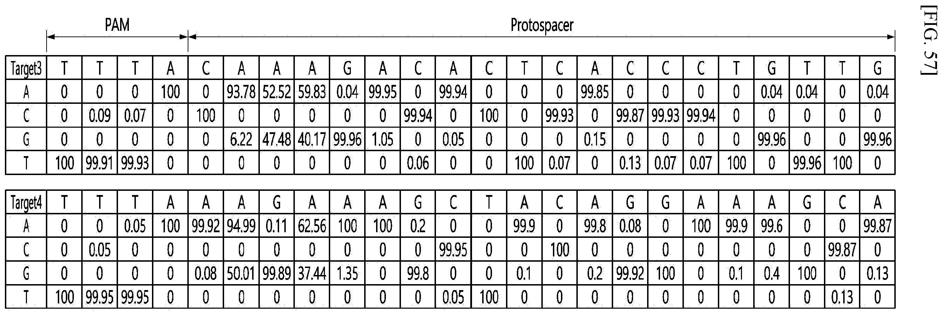

FIGS. 55, 56, and 57 illustrate results obtained by identifying base editing efficiency of CRISPR base editing systems that comprise dCas12f1-base editing proteins of various structures of the present disclosure, according to Experimental Example 3.2. Here, the target protospacer sequences are as shown in Tables 1 and 2. The dCas12f1-base editing fusion proteins are as shown in Table 9. Specifically, ABE_N1, ABE_N2, ABE_C1, and ABE_C2 represent dTnpB-ABE-N1, dTnpB-ABE-N2, dTnpB-ABE-C1, and dTnpB-ABE-C2, respectively.

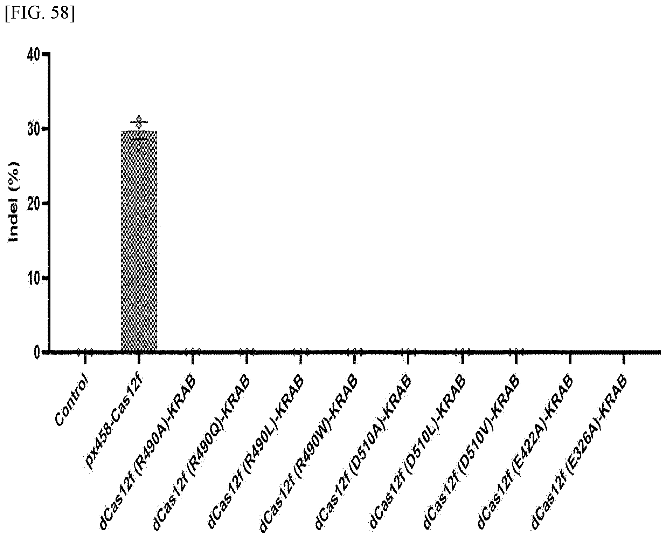

FIG. 58 illustrates a graph obtained by performing experiments on whether dead Cas12f1 proteins, which were prepared by substitution using various amino acids within mutation positions, have their nucleic acid cleavage activity removed.

Here, each label indicates mutation position and mutated target based on the Cas12f1 amino acid sequence of SEQ ID NO: 1. For example, dCas12f(R490A)-KRAB means a variant obtained by replacing arginine at position 490 of the Cas12f1 amino acid sequence of SEQ ID NO: 1 with alanine. The other labels were created using the same rule.

### Best Mode for Carrying out Invention

Hereinafter, with reference to the attached drawings, the present disclosure will be described in more detail through specific embodiments and examples. It should be noted that the attached drawings include some, but not all, embodiments of the present disclosure. The disclosure described herein may be embodied in various ways and is not limited to the specific embodiments described herein. These embodiments should be regarded as being provided to satisfy statutory requirements applicable to the present specification. Those skilled in the art to which the present disclosure belongs will be able to contemplate many modifications and other embodiments for the disclosure described herein. Accordingly, it should be understood that the disclosure described herein is not limited to the specific embodiments described herein, and modifications therefor and other embodiments are also included within the scope of the claims.

### Definition of terms

### About

As used herein, the term "about" refers to an amount, level, value, number, frequency, percent, dimension, size, amount, weight or length that varies by approximately 30%, 25%, 20%, 15%, 10%, 9%, 8%, 7%, 6%, 5%, 4%, 3%, 2% or 1% with respect to a reference amount, level, value, number, frequency, percent, dimension, size, amount, weight or length.

### Notation of amino acid sequence

Unless otherwise stated, in a case of describing an amino acid sequence herein, the amino acid sequence is described from the N-terminus to the C-terminus using single-letter notation or triple-letter notation for amino acid. For example, in a case of being described as MAKN, it means a peptide in which methionine, alanine, lysine, and asparagine are sequentially linked to each other from the N-terminus to the C-terminus. As another example, in a case of being described as Thr-Leu-Lys, it means a peptide in which threonine, leucine, and lysine are sequentially linked to each other from the N-terminus to the C-terminus. For amino acids that cannot be described using the single-letter notation, they are described using other letters and are additionally provided with supplementary explanation.

The notation for each amino acid is as follows: alanine (Ala, A); arginine (Arg, R); asparagine (Asn, N); aspartic acid (Asp, D); cysteine (Cys, C); glutamic acid (Glu, E); glutamine (Gln, Q); glycine (Gly, G); histidine (His, H); isoleucine (Ile, I); leucine (Leu, L); lysine (Lys, K); methionine (Met, M); phenylalanine (Phe, F); proline (Pro, P); serine (Ser, S); threonine (Thr, T); tryptophan (Trp, W); tyrosine (Tyr, Y); and valine (Val, V).
A, T, C, G, and U

The symbols A, T, C, G, and U as used herein are interpreted as having the same meanings as commonly understood by those skilled in the art. The symbol may be properly interpreted as a base, a nucleoside, or a nucleotide in DNA or RNA depending on the context and description. For example, in a case where the symbols mean bases, they may be interpreted as adenine (A), thymine (T), cytosine (C), guanine (G), or uracil (U), respectively; in a case where the symbols mean nucleosides, they may be interpreted as adenosine (A), thymidine (T), cytidine (C), guanosine (G), or uridine (U), respectively; and in a case where the symbols mean nucleotides in a sequence, they may be interpreted to mean nucleotides including the respective nucleosides.

### Operably linked

As used herein, the term "operably linked" means that, in gene expression techniques, a particular component is linked to another component so that the particular component can function as intended. For example, in a case where a promoter sequence is operably linked to a coding sequence, it means that the promoter is linked thereto so as to affect transcription and/or expression of the coding sequence in a cell. In addition, the term includes all meanings recognized by those skilled in the art and may be appropriately interpreted depending on the context.

### Target gene or target nucleic acid

As used herein, "target gene" or "target nucleic acid" basically means a gene or nucleic acid in a cell which becomes a target for regulation of gene expression. The target gene or target nucleic acid may be used interchangeably and may refer to the same target. Unless otherwise stated, the target gene or target nucleic acid may refer to both a gene or nucleic acid inherent in a target cell or an externally-derived gene or nucleic acid, and is not particularly limited as long as it can be a target for regulation of gene expression. The target gene or target nucleic acid may be single-stranded DNA, double-stranded DNA, and/or RNA. In addition, the term includes all meanings recognized by those skilled in the art and may be appropriately interpreted depending on the context.

### Target sequence

As used herein, "target sequence" refers to a particular sequence recognized by a CRISPR activation complex or CRISPR interference complex to regulate expression of a target gene or target nucleic acid. The target sequence may be appropriately selected depending on the purpose.

As an example, the "target sequence" means a sequence contained in a target gene or target nucleotide sequence, which has complementarity with a sequence of spacer (guide domain) contained in the guide RNA provided herein or binds complementarily to the spacer. The target sequence is located within a target strand recognized by the guide RNA. Here, a PAM sequence is a sequence recognized by Cas protein and is located within a non-target strand that is a strand complementary to the target strand. The non-target strand comprises a protospacer sequence, in which the protospacer sequence is located at the 3' end of the PAM sequence. The protospacer sequence is a sequence that forms a complementary bond with the target sequence.

As another example, the target sequence may refer only to a specific strand that binds complementarily to a guide RNA of a CRISPR/Cas system, or may refer to an entire target double strand including the specific strand portion, and interpretation thereof is made appropriately depending on the context.

In addition, the term includes all meanings recognized by those skilled in the art and may be appropriately interpreted depending on the context.

### Vector

As used herein, unless otherwise specified, the "vector" refers collectively to any material capable of transporting a genetic material into a cell. For example, a vector may be a DNA molecule including a genetic material of interest, for example, a nucleic acid encoding a fusion protein comprising a dead Cas12f1 protein, and/or a nucleic acid encoding a guide RNA; however, the vector is not limited thereto. The term includes all meanings recognized by those skilled in the art and may be appropriately interpreted depending on the context.

### Naturally occurring

As used herein, the term "naturally occurring" refers to an object that is found in nature and is not modified. The term is used to distinguish it from an "engineered object" obtained by artificial modification. The "naturally occurring" gene, nucleic acid, DNA, RNA, and the like are used as concepts that encompass all genes, nucleic acids, DNA, and RNA in wild-type and mature form (active form). The term includes all meanings recognized by those skilled in the art and should be appropriately interpreted depending on the context.

### Engineered

As used herein, the term "engineered" is used to distinguish it from a material, a molecule, or the like whose configuration already exists in nature, and this means that the material, the molecule, or the like has undergone artificial modification. For example, the "engineered Cas12f1 protein" collectively means one obtained by applying artificial modification to the configuration of a naturally occurring Cas 12f1 protein. In addition, the term includes all meanings recognized by those skilled in the art and may be appropriately interpreted depending on the context.

### Nuclear localization sequence or signal (NLS)

In a case where a substance outside the cell's nucleus is transported into the nucleus by nuclear transport, the term "NLS" as used herein refers to a peptide of a certain length or a sequence thereof, wherein the peptide is attached to a protein to be transported and acts as a type of "tag." Specifically, the NLS may be, but is not limited to, an NLS sequence derived from: the NLS of an SV40 virus large T-antigen having the amino acid sequence PKKKRKV (SEQ ID NO: 200); the NLS from a nucleoplasmin (for example, the nucleoplasmin bipartite NLS having the sequence KRPAATKKAGQAKKKK (SEQ ID NO: 201)); the c-myc NLS having the amino acid sequence PAAKRVKLD (SEQ ID NO: 202) or RQRRNELKRSP (SEQ ID NO: 203); the hRNPA1 M9 NLS having the sequence NQSSNFGPMKGGNFGGRSSGPYGGGGQYFAKPRNQGGY (SEQ ID NO: 204); the sequence RMRIZFKNKGKDTAELRRRRVEVSVELRKAKKDEQILKRRNV (SEQ ID NO: 205) of an IBB domain from importin alpha; the sequences VSRKRPRP (SEQ ID NO: 206) and PPKKARED (SEQ ID NO: 207) of myoma T protein; the sequence PQPKKKPL (SEQ ID NO: 208) of human p53; the sequence SALIKKKKKMAP (SEQ ID NO: 209) of mouse c-abl IV; the sequences DRLRR (SEQ ID NO: 210) and PKQKKRK (SEQ ID NO: 211) of influenza virus NS1; the sequence RKLKKKIKKL (SEQ ID NO: 212) of hepatitis virus delta antigen; the sequence REKKKFLKRR (SEQ ID NO: 213) of mouse Mx1 protein; the sequence KRKGDEVDGVDEVAKKKSKK (SEQ ID NO: 214) of human poly (ADP-ribose) polymerase; or the sequence RKCLQAGMNLEARKTKK (SEQ ID NO: 215) of a steroid hormone receptor (human) glucocorticoid. As used herein, the term "NLS" includes all meanings recognized by those skilled in the art and may be appropriately interpreted depending on the context.

### Nuclear export sequence or signal (NES)

In a case where a substance inside the cell's nucleus is transported outside the nucleus by nuclear transport, the term "NES" as used herein refers to a peptide of a certain length or a sequence thereof wherein the peptide is attached to a protein to be transported and acts as a type of "tag." As used herein, the term "NES" includes all meanings recognized by those skilled in the art, and may be appropriately interpreted depending on the context.

### Tag

As used herein, the term "tag" refers collectively to a functional domain added to facilitate tracking and/or separation and purification of a peptide or protein. Specifically, the tag includes, but is not limited to, tag proteins such as a histidine (His) tag, a V5 tag, a FLAG tag, an influenza hemagglutinin (HA) tag, an Myc tag, a VSV-G tag, and a thioredoxin (Trx) tag; autofluorescent proteins such as a green fluorescent protein (GFP), a yellow fluorescent protein (YFP), a cyan fluorescent protein (CFP), a blue fluorescent protein (BFP), HcRED, and DsRed; and reporter proteins such as a glutathione-S-transferase (GST), a horseradish peroxidase (HRP), a chloramphenicol acetyltransferase (CAT) beta-galactosidase, a beta-glucuronidase, and a luciferase. As used herein, the term "tag" includes all meanings recognized by those skilled in the art and may be appropriately interpreted depending on the context.

Unless defined otherwise, all technical and scientific terms used herein have the same meanings as commonly understood by those skilled in the art to which the present disclosure belongs. Although methods and materials similar or equivalent to those described herein may be used in practice or experimentation of the present disclosure, suitable methods and materials are described below. All publications, patents, and other references mentioned herein are incorporated by reference in their entirety. In addition, the materials, methods, and examples are illustrative only and not intended to limit the present disclosure.

### Adenosine deaminase

As used herein, the term "adenosine deaminase" refers to a protein involved in a deamination reaction, or a domain that performs such function, the deamination reaction being such that adenine (A) in RNA/DNA and DNA duplexes is targeted and adenine or an adenine moiety of an adenine-containing molecule (for example, adenosine, DNA, RNA) is hydrolyzed to hypoxanthine or a hypoxanthine moiety of a hypoxanthine-containing molecule (for example, inosine (I)). The adenosine deaminase is rarely found in higher animals, and is known to be present in small quantities in cow muscle, milk, and murine blood, and to be present in large quantities in crayfish intestine and insects.

### Cytidine deaminase

As used herein, the term "cytidine deaminase" refers to an enzyme protein that targets and deaminates cytosine (C), resulting in its conversion to uracil (U). In a case where cytosine is converted into uracil due to removal of amine group, uracil is converted to thymine (T) through a series of intracellular repair mechanisms. In such a manner, base editing of cytosine (C) base to thymine (T) base can be ultimately induced. Cytidine deaminase generally functions on RNA. However, some cytidine deaminases are known to be able to function on single strand DNA (ssDNA) (Harris et al., 2002), examples of which include, but are not limited to, human activation-induced cytidine deaminase (AID), human APOBEC3G, murine APOBEC1, APOBEC3A, APOBEC3B, CDA, AID, and lamprey PmCDA1.

### Linker

As used herein, the term "linker" refers to a linking moiety that links two molecules or two components. The linker may be a nucleic acid, an amino acid, or other compound, and its type may be determined depending on the two molecules to be linked. Unless otherwise stated, in a case where components or molecules of the same type are linked by a linker, the linker can also be understood as a molecule of the same type as the targets to be linked. For example, in a case where targets to be linked by a linker are proteins, peptides, or amino acids, the linker may also be a protein, peptide, or amino acid. As another example, in a case where targets to be linked by a linker are nucleic acids, the linker may also be a nucleic acid. Specifically, in a case where all targets to be linked are DNA, the linker may also be DNA, and in a case where all targets to be linked are RNA, the linker may also be RNA.

In a case where it is described herein that different components are linked, fused, and/or bound to each other, such a case should be understood to encompass both cases where the two components are directly linked to each other and the two components are linked via a linker of the same type.

In a case where the linker is RNA or DNA, it may be, but is not limited to, a nucleic acid having a nucleotide sequence of 5'-GAAA-3'. In a case where the linker is an amino acid, peptide, or protein, it may be selected from, but is not limited to, (GGGGS)n, (G)n, (EAAAK)n, (GGS)n, (XP)n, SGGSSGGSSG (SEQ ID NO: 260), SGGSSGGSSGSETP (SEQ ID NO: 261), SGGSSGGSSGSETPGT (SEQ ID NO: 262), SGGSSGGSSGSETPGTSESA (SEQ ID NO: 263), SGGSSGGSSGSETPGTSESATPES (SEQ ID NO: 264), SGGSSGGSSGSETPGTSESATPESSGGS (SEQ ID NO: 265), SGGSSGGSSGSETPGTSESATPESSGGSSGGS (SEQ ID NO: 266), SGGSSGGSSGSETPGTSESATPESSGGSSGGSSGSE (SEQ ID NO: 267), SGGSSGGSSGSETPGTSESATPESSGGSSGGSSGSETPGT (SEQ ID NO: 268), GGGGS (SEQ ID NO: 269), EAAAK (SEQ ID NO: 270), SGSETPGTSESATPES (SEQ ID NO: 271), SGGS (SEQ ID NO: 272), and SGGSKRTADGSEFE (SEQ ID NO: 273). Here, n is an integer of 1 or more, and X is one selected from standard amino acids.

The term "linker" as used herein includes all other meanings recognized by those skilled in the art, and may be appropriately interpreted depending on the context.

The present disclosure will be described below.

### CRISPR/Cas12f1 system

A CRISPR/Cas12f system belongs to a V-F subtype among type V CRISPR/Cas systems, which is further divided into V-F1 to V-F3 variants. The CRISPR/Cas 12f system includes CRISPR/Cas 14 systems, which comprise Cas 14a, Cas 14b, and Cas 14c variants, among the effector proteins named Cas14 in a previous study (Harrington et al., Programmed DNA destruction by miniature CRISPR-Cas14 enzymes, Science 362, 839-842 (2018)). Among them, the CRISPR/Cas14a system comprising a Cas14a effector protein is classified as a CRISPR/Cas12f1 system (Makarova et al., Nature Reviews, Microbiology volume 18, 67 (2020)). Recent previous studies (Takeda et al., Structure of the miniature type V-F CRISPR-Cas effector enzyme, Molecular Cell 81, 1-13 (2021), Xiao et al., Structural basis for the dimerization-dependent CRISPR-Cas12f nuclease, bioRxiv (2020)) and the like have revealed a structure of the CRISPR/Cas12f1 complex.

As revealed in previous studies (Harrington et al., Science 362, 839-842 (2018), Tautvydas Karvelis et al., Nucleic Acids Research 48, 5016-5023 (2020)), the CRISPR/Cas12f1 system shows no cleavage activity or shows cleavage activity with extremely low efficiency on double-stranded DNA in a cell, which limits its active application to gene editing. However, to overcome such limitation, the present inventors have recently developed an engineered Cas12f1 guide RNA to increase intracellular gene editing activity of the CRISPR/Cas12f1 system.

### Limitations of prior art

In order to effectively utilize Cas nuclease or various fusion proteins based on Cas nuclease, it is essential to use techniques of encoding the Cas nuclease and the guide RNA, loading them onto a vector, and delivering the vector to cells. However, for the most previously studied Cas nucleases, it is difficult to load them onto a vector due to their size. Adeno-associated virus (AAV), which is the most widely used and important vector, has a limited length (~4.7kb) of nucleotide sequence that can be loaded thereonto. However, nucleic acids encoding the most Cas nucleases have lengths that exceed loading capacity of AAV

Meanwhile, studies are actively conducted to develop fusion proteins with functions such as base editing and regulation of gene expression by fusion of a functional domain to Cas nuclease, in which a high target sequence recognition ability of the Cas nuclease is used. However, since such studies also involve fusing an additional domain to Cas nuclease having a large size, it becomes more and more difficult to develop such fusion proteins and vectors for delivery thereof.

### Dead Cas12f1 protein and dCas12f1-based fusion protein

### Overview of dead Cas12f1 protein and dCas12f1-based fusion protein

The present inventors sought to develop a Cas12f1-based fusion protein having a very small size so that 1) it is easy to develop a new fusion protein formed by fusion with an additional domain, and 2) the completed fusion protein is easily loaded onto AAV

The present inventors have first invented dead Cas12f1 proteins obtained from a wild-type Cas12f1 protein and Cas12f1 variants by losing nucleic acid cleavage activity thereof so that they can be applied to various fusion proteins.

In addition, the present inventors have invented dCas12f1-base editing fusion proteins, each of which has a base editing function for a target gene in a cell, the fusion proteins being obtained by fusing a base editing domain to the thus developed dead Cas12f1 proteins. Specifically, the dCas12f1-base editing fusion proteins, depending on their specific configurations, have 1) a function of editing adenine (A) at a specific position in the target gene to guanine (G), and/or 2) a function of editing cytosine (C) at a specific position in the target gene to thymine (T).

In addition, the present inventors have invented dCas 12f1-expression regulating fusion proteins, each of which has a function of regulating expression of a target gene in a cell, the fusion proteins being obtained by fusing a gene expression regulatory domain to the thus developed dead Cas12f1 protein. Specifically, the dCas12f1-expression regulating fusion proteins, depending on their specific configurations, have 1) a function of promoting transcription of the target gene, and/or 2) a function of inhibiting transcription of the target gene.

Hereinafter, for convenience of description, such various dead Cas12f1 proteins, dCas12f1-base editing fusion proteins, and dCas12f1-expression regulating fusion proteins may be collectively referred to as engineered Cas12f1 proteins.

Through previous research, the present inventors had invented engineered guide RNAs that dramatically increase target sequence recognition and nucleic acid cleavage activity of Cas12f1 protein and enable double-stranded nucleic acid cleavage. By combining these guide RNAs with the dead Cas12f1 and the dCas12f1-based fusion proteins and identifying, through experiments, whether such engineered CRISPR/Cas12f1 systems perform their intended function, the present inventors have completed the present disclosure.

### Dead Cas12f1 protein

The dead Cas12f1 protein disclosed herein may be a modified form of a Cas12f1 protein which has lost nucleic acid cleavage activity. Here, the Cas12f1 protein may be a wild-type Cas12f1 protein or a Cas12f1 variant protein. The present inventors have invented dead Cas12f1 proteins having lost nucleic acid cleavage activity by modification of amino acids at positions that are known to be involved in nucleic acid cleavage activity of wild-type Cas 12f1 and are selected through research. Meanwhile, the present inventors have revealed that among variants obtained by adding amino acid(s) to the N-terminus and/or C-terminus of the amino acid sequence of the wild-type Cas12f1 protein, there are Cas12f1 variants that have the same or improved function as the Cas12f1 protein, and have invented dead Cas12f1 proteins based on the Cas12f1 variants by applying a technique of modifying the wild-type Cas12f1 protein into a dead form thereof.

In an embodiment, the wild-type Cas12f1-based dead Cas12f1 protein may have a sequence selected from SEQ ID NOS: 2 to 8. In an embodiment, the Cas12f1 variant-based dead Cas12f1 protein may have a sequence selected from SEQ ID NOS: 15 to 24, SEQ ID NO: 26 to 29, SEQ ID NO: 31 to 34, and SEQ ID NO: 36 to 39.

### dCas12f1-base editing fusion protein

Disclosed herein is a dCas12f1-base editing fusion protein in which a base editing domain is fused to the dead Cas12f1 protein. The base editing domain is a domain that has a function of editing a specific base in a gene to another base, and is capable of editing a base at a specific site to a desired base in a case of being combined with a high target sequence recognition ability of the dead Cas 12f1 protein.

In an embodiment, the base editing domain may be adenosine deaminase and/or cytidine deaminase, and the dCas12f1-base editing fusion protein formed by fusion with the domain functions as an adenine and/or cytosine base editor.

### dCas12f1-expression regulating fusion protein

Disclosed herein is a dCas12f1-expression regulating fusion protein in which a base editing domain is fused to the dead Cas12f1 protein. The expression regulatory domain is a domain that has a function of regulating expression of a target gene, and is capable of promoting or inhibiting expression of a specific gene in a case of being combined with a high target sequence recognition ability of the dead Cas12f1 protein.

In an embodiment, the expression regulatory domain may be VP64, and the dCas12f1-expression regulating fusion protein formed by fusion with VP64 functions to promote expression of a specific gene. In another embodiment, the expression regulatory domain may be selected from KRAB, MeCP2, DNMT3A, and hHDAC3, and the dCas12f1-expression regulating fusion protein formed by fusion with the domain functions to inhibit expression of a specific gene.

### Characteristics of dead Cas12f1 protein - making it easy to develop fusion protein

The dead Cas12f1 protein disclosed herein is very small in size, works in conjunction with the guide RNA disclosed herein, and has very high target-specific binding activity, which makes it very easy to develop a functional protein by fusion of an additional functional domain to the dead Cas12f1 protein.

### Characteristics of dead Cas12f1 protein and dCas12f1-based fusion protein - easily loaded onto vector

Dead Cas12f1 proteins and dCas12f1-based fusion proteins disclosed herein are so small that they can be loaded onto AAV, and guide RNAs targeting two or more target sequences can also be loaded thereonto in some cases. Therefore, such proteins make it easy to produce a vector as compared with other Cas systems that cannot be loaded onto a single unit of AAV due to their very large size, which allows all components such as protein and guide RNA to be expressed from a single unit of AAV. From this point of view, very high efficacy can be expected in a case of actually developing a genetic therapeutic agent.

### Cas12f1 protein

### Cas12f1 protein - overview

The dead Cas12f1 proteins or dCas12f1-based fusion proteins disclosed herein are those which are 1) based on a wild-type Cas12f1 protein or Cas12f1 variant proteins and are 2) modified therefrom not to show nucleic acid cleavage activity (dead Cas12f1), and 3) those in which an additional functional domain is fused to the dead Cas12f1 in a case where the protein is a dCas12f1-based fusion protein.

The wild-type Cas12f1 protein includes a CRISPR/Cas14a system comprising the Cas14a effector protein as described above (Makarova et al., Nature Reviews, Microbiology volume 18, 67 (2020)).

The present inventors have developed Cas12f1 variant proteins having the same function as the wild-type Cas12f1 protein, and the Cas12f1 variant proteins correspond to functional analogs of the wild-type Cas12f1 protein.

Hereinafter, the term "Cas12f1 protein" as used herein, unless otherwise stated, refers collectively to the wild-type Cas12f1 protein and a Cas12f1 variant protein (functional analogue of the wild-type Cas12f1) having the same function as the wild-type Cas12f1 protein, and should be interpreted appropriately depending on the context.

### Wild-type Cas12f1 protein

The Cas12f1 protein may be a wild-type Cas12f1 protein. Here, the Cas12f1 protein is capable of cleaving a double strand or single strand of a target nucleic acid or target gene.

As an embodiment, the Cas12f1 protein may be derived from the Cas14 family (Harrington et al., Science 362, 839-842 (2018); US 2020/0172886 A1).

As another embodiment, the Cas12f1 protein may be Cas14a1 protein derived from an uncultured archaeon (Harrington et al., Science 362, 839-842 (2018); US 2020/0172886 A1). For example, the Cas14a1 protein may have the amino acid sequence of SEQ ID NO: 1.

### Cas12f1 variant protein 1 - general

The Cas12f1 protein may be a Cas12f1 variant protein. The Cas12f1 variant may be a variant of a wild-type Cas12f1 protein in which at least one amino acid in the amino acid sequence of the wild-type Cas12f1 protein is modified. Here, the modification may be deletion and/or substitution. Alternatively, the Cas12f1 variant may be a variant of a wild-type Cas12f1 protein in which at least one amino acid sequence is added to both ends of the amino acid sequence of the wild-type Cas12f1 protein and/or within the amino acid sequence thereof. Here, the modification may be insertion. Here, the Cas12f1 variant is referred to as "Cas12f1 mutant" or "Cas14a1 mutant. "

In an embodiment, the Cas12f1 mutant may be obtained by deleting at least one amino acid in the amino acid sequence of the wild-type Cas 12f1 protein. For example, the Cas12f1 mutant may be obtained by deleting at least one amino acid in a RuvC domain included in the wild-type Cas12f1 protein. Alternatively, the Cas12f1 mutant may be obtained by deleting at least one amino acid in a domain that recognizes a PAM included in the wild-type Cas12f1 protein. Alternatively, the Cas12f1 mutant may be obtained by deleting at least one amino acid in the amino acid sequence of SEQ ID NO: 1.

In another embodiment, the Cas12f1 mutant may be obtained by replacing at least one amino acid in the amino acid sequence of the wild-type Cas12f1 protein with other amino acid(s). Here, the replacement may be such that one amino acid is replaced with one other amino acid. Alternatively, the replacement may be such that one amino acid is replaced with a plurality of other amino acids. Alternatively, the replacement may be such that a plurality of amino acids are replaced with one other amino acid. Alternatively, the replacement may be such that a plurality of amino acids are replaced with a plurality of other amino acids, in which the number of amino acids to be replaced and the number of replacing amino acids may be the same or different from each other. For example, the Cas12f1 mutant may be obtained by replacing at least one amino acid in a RuvC domain included in the wild-type Cas 12f1 protein with other amino acid(s). Alternatively, the Cas12f1 mutant may be obtained by replacing at least one amino acid in a domain, which recognizes a PAM included in the wild-type Cas12f1 protein, with other amino acid(s). Alternatively, the Cas12f1 mutant may be obtained by replacing at least one amino acid in the amino acid sequence of SEQ ID NO: 1 with other amino acid(s). For example, the Cas12f1 mutant may be represented by an amino acid sequence selected from.

The Cas 12f1 mutant may be a variant having the same function as the wild-type Cas 12f1 protein or a variant of which some or all functions are modified as compared with the wild-type Cas12f1 protein. For example, the Cas12f1 mutant may be a variant in which modification is made to cleave only one strand in a double-strand of a target nucleic acid. Alternatively, the Cas12f1 mutant may be a variant in which modification is made to recognize a PAM sequence other than 5'-TTTA-3' or 5'-TTTG-3'.

### Cas12f1 variant protein 2 - Cas12f1 variant protein comprising dummy sequence

The present inventors made research and have found that among the mutants in which an amino acid(s) is added to the N-terminus and/or C-terminus of the amino acid sequence of the wild-type Cas12f1 protein, there is a Cas12f1 variant having the same or improved function as the Cas12f1 protein. Furthermore, the present inventors invented a Cas12f1 variant having the same function as the wild-type Cas12f1 protein by adding an amino acid sequence of a certain length to the N-terminus of the wild-type Cas12f1 protein. The Cas12f1 variant is disclosed in detail in Korean Patent Application No. 10-2021-0181875. Unless otherwise stated, reference may be made herein to the contents of the above application regarding the Cas12f1 variant or functional analogs thereof.

In an embodiment, the Cas12f1 variant may be a variant of a wild-type Cas12f1 protein in which 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids are added to the N-terminus and/or C-terminus of the wild-type Cas12f1 protein. In an embodiment, the Cas12f1 variant may be a variant of a wild-type Cas12f1 protein in which the number of amino acids within the above-mentioned two numerical ranges is added to the N-terminus and/or C-terminus of the wild-type Cas12f1 protein. For example, the Cas12f1 variant may be a variant of a wild-type Cas12f1 protein in which 26 to 28 amino acids are added to the N-terminus of the wild-type Cas12f1 protein.

The amino acid sequence of a certain length may be referred to as a dummy sequence, and the dummy sequence is not particularly limited as long as 1) it does not prevent the Cas12f1 variant protein from interacting with a guide RNA to form a complex, 2) it does not affect an ability of the Cas 12f1 variant protein to exhibit nucleic acid cleavage activity, and 3) it does not affect an ability of the Cas12f1 variant protein to recognize a PAM sequence in a target gene.

In an embodiment, the dummy sequence may comprise 1 to 40 amino acids. For example, the dummy sequence may be an amino acid sequence selected from SEQ ID NOS: 10 to 13.

In an embodiment, the Cas12f1 variant may be a protein represented by an amino acid sequence selected from SEQ ID NO: 14, SEQ ID NO: 25, SEQ ID NO: 30, and SEQ ID NO: 35. Among these, the protein represented by SEQ ID NO: 14 is also known as transposon-associated transposase B (TnpB) derived from Candidatus Woesearchaeota archaeon, and the terms such as Cas12f1 protein, Cas12f1 variant, Cas12f1 functional analog, TnpB, and TnpB functional analog may be used interchangeably in the present specification.

In the present specification, the Cas12f1 variant protein of SEQ ID NO: 14 may be referred to as Cas12f1 variant 1, the Cas12f1 variant protein of SEQ ID NO: 25 may be referred to as Cas12f1 variant 2, the Cas12f1 variant protein of SEQ ID NO: 30 may be referred to as Cas12f1 variant 3, and the Cas12f1 variant protein of SEQ ID NO: 35 may be referred to as Cas12f1 variant 4. The above-mentioned names are only used for convenience, and the Cas12f1 protein variants are not limited by such terms.

### PAM sequence of Cas12f1 protein

The Cas12f1 protein is capable of recognizing a protospacer adjacent motif (PAM) sequence present in a target nucleic acid or target gene. Here, the PAM sequence is a unique sequence determined by the Cas14a1 protein.

The PAM sequence for the Cas12f1 protein may be a T-rich sequence. The PAM sequence for the Cas12f1 protein may be 5'-TTTN-3'. Here, N may be A, T, C, or G. For example, the PAM sequence may be 5'-TTTA-3', 5'-TTTT-3', 5'-TTTC-3', or 5'-TTTG-3'.

### Dead Cas12f1 protein

### Overview of dead Cas12f1 protein

Disclosed herein is a cleavage-inactive Cas12f1 (dead Cas12f1) protein, which is a Cas12f1 protein with nucleic acid cleavage activity removed. The dead Cas12f1 protein refers to a functional variant of the wild-type Cas12f1 protein and/or Cas12f1 protein variant, with its nucleic acid cleavage activity removed.

The present inventors made research and have found that specific amino acids in a wild-type Cas12f1 protein are significantly involved in nucleic acid cleavage activity, thereby having invented dead Cas12f1 proteins with nucleic acid cleavage activity removed through modification at the corresponding sites.

Furthermore, the present inventors have revealed that the Cas12f1 variant protein-based dead Cas12f1 protein can be prepared by applying the modification performed to make the wild-type Cas12f1 protein into a dead form thereof. Accordingly, the present inventors have invented dead forms of the Cas12f1 variant protein.

As compared with fusion proteins comprising the wild-type Cas 12f1-based dead Cas12f1 protein, base editing/expression regulating fusion proteins comprising the Cas12f1 variant-based dead Cas12f1 protein show at least equivalent functions or show better functions depending on target sequences.

### Mutation position for preparation of dead Cas12f1

As compared with the wild-type Cas12f1 protein, the dead Cas12f1 protein disclosed herein is characterized by loss of nucleic acid cleavage activity which is caused by replacement of the amino acid involved in the nucleic acid cleavage activity with another amino acid. The target mutation position is not particularly limited as long as it is an amino acid position involved in nucleic acid cleavage activity, and the mutation may occur at one or more positions.

In an embodiment, the mutation position may be aspartic acid at position 326, glutamic acid at position 422, arginine at position 490, and/or aspartic acid at position 510, based on SEQ ID NO: 1.

### Mutated target for preparation of dead Cas12f1

It can be expected for the Cas12f1 protein to lose its nucleic acid cleavage activity in a case where mutation is appropriately performed at a target mutation position of the Cas12f1 protein. Here, to achieve such loss of activity, the original amino acid at the target mutation position needs to be replaced with another appropriate amino acid in consideration of the type and structure of the original amino acid.

In an embodiment, the dead Cas12f1 protein may be such that the amino acid(s) at one or more target mutation positions involved in the nucleic acid cleavage activity is replaced with other standard amino acid(s). Specifically, such replacement may be made with alanine, glutamine, leucine, tryptophan, and/or valine.

### Dead form of Cas12f1 variant protein

The mutation position and mutated target for preparation of the dead Cas12f1 may also be applied to a Cas12f1 variant protein, thereby preparing a dead form of the Cas12f1 variant protein. Specifically, in a case where the Cas12f1 variant protein is a variant comprising a dummy sequence, the Cas12f1 variant protein may be subjected to replacement of the amino acid at the mutation position, which corresponds to the amino acid sequence of the wild-type Cas12f1 protein, with a mutated target amino acid, thereby preparing a Cas12f1 variant protein-based dead Cas12f1 protein.

In an embodiment, the dead Cas12f1 protein may be such that one or more respective mutation positions in the wild-type Cas12f1 represented by SEQ ID NO: 14 have undergone replacement with one or more mutated targets, with the mutation position and the mutated target being selected from the following:
Mutation position: aspartic acid at position 354, glutamic acid at position 450, arginine at position 518, and/or aspartic acid at position 538; and
mutated target: alanine, glutamine, leucine, tryptophan, and/or valine.

More specific examples thereof are described in the subsection "dead Cas12f1 protein" of the section "Possible Embodiments of Invention."

### Examples of amino acid sequence of dead Cas12f1 protein

In an embodiment, the dead Cas12f1 protein may have the amino acid sequence selected from SEQ ID NOS: 2 to 5, SEQ ID NOS: 15 to 24, SEQ ID NOS: 26 to 29, SEQ ID NOS: 31 to 34, and SEQ ID NOS: 36 to 39.

### dCas12f1-base editing fusion protein

### Overview of dCas12f1-base editing fusion protein

Disclosed herein is a dCas12f1-base editing fusion protein in which a dead Cas12f1 protein and a base editing domain are fused together. The dCas12f1-base editing fusion protein is a protein in which the dead Cas12f1 protein, at least one base editing domain, and other additional component(s) are fused together; and upon delivery into a cell, it may function together with a guide RNA to edit a base(s) of a predetermined target sequence in a target gene in the cell or a nucleotide sequence adjacent to the target sequence. The dCas12f1-base editing fusion protein may essentially comprise the dead Cas12f1 protein and at least one base editing domain, and may comprise an additional component(s).

As a result, the dCas12f1-base editing fusion protein may serve as a CRISPR base editing system that functions to edit a base(s) at a site adjacent to a target sequence in a target gene.

Hereinafter, a configuration of the dCas12f1-base editing fusion protein will be described in detail.

### Component 1 of dCas12f1-base editing fusion protein - dead Cas12f1 protein

The dCas 12f1-base editing fusion protein comprises a dead Cas 12f1 protein that has lost nucleic acid cleavage activity. As described above, the dead Cas12f1 protein recognizes a predetermined target sequence in a target gene and functions to bind to the corresponding site, so that it serves to allow the base editing domain, which is another component of the dCas12f1-base editing fusion protein, to edit a base(s) at the target sequence or at a site adjacent to the target sequence. In other words, the dead Cas12f1 protein is a moiety designed to position-specifically deliver the base editing domain to a target sequence site having a base(s) to be edited. The dead Cas12f1 protein is characterized by loss of nucleic acid cleavage activity, and thus does not cause unwanted nucleic acid cleavage and indel generation. A specific configuration of the dead Cas12f1 protein is as described in the section "Dead Cas12f1 protein."

### Component 2 of dCas12f1-base editing fusion protein - base editing domain

The dCas12f1-base editing fusion protein comprises a base editing domain. The base editing domain is not particularly limited as long as it is a domain that has a function of editing a specific base in a target gene. More specifically, adenosine deaminase, which is capable of editing adenine at a specific position in a target gene to guanine, and cytidine deaminase, which is capable of editing cytosine to thymine, are known as the base editing domain. The present inventors combined such deaminase with the dead Cas12f1, and thus have invented an adenine base-editor (ABE) and a cytosine base-editor (CBE).

### Base editing domain 1 - adenosine deaminase

The base editing domain may be the entire adenosine deaminase or a partial domain thereof. In a case where the dCas12f1-base editing fusion protein comprises adenosine deaminase, the dCas12f1-base editing fusion protein functions as an adenine base-editor (hereinafter referred to as ABE). The dCas12f1-base editing fusion protein comprising the entire adenosine deaminase or a partial domain thereof functions to replace at least one adenine, which is contained in a target sequence or a sequence adjacent to the target sequence, with guanine. The adenosine deaminase is not particularly limited as long as it allows the dCas12f1-base editing fusion protein to perform such a function. For example, the adenosine deaminase may be Escherichia coli (E. coli)-derived tRNA adenosine deaminase (TadA) and/or a variant of TadA. In addition, the dCas12f1-base editing fusion protein may comprise at least one unit of TadA and/or the variant of TadA.

### Base editing domain 2 - cytidine deaminase

The base editing domain may be the entire cytidine deaminase or a partial domain thereof. In a case where the dCas12f1-base editing fusion protein comprises cytidine deaminase, the dCas12f1-base editing fusion protein functions as a cytosine base-editor (hereinafter referred to as CBE). The dCas 12f1-base editing fusion protein comprising the entire cytidine deaminase or a partial domain thereof functions to replace at least one cytosine, which is contained in a target sequence or a sequence adjacent to the target sequence, with thymine. The cytidine deaminase is not particularly limited as long as it allows the dCas12f1-base editing fusion protein to perform such a function. For example, it may be human activation-induced cytidine deaminase (AID), human APOBEC3G, murine APOBEC1, APOBEC3A, APOBEC3B, CDA, AID, and/or lamprey PmCDA1.

### Other component 1 - other domain(s) involved in base editing

The dCas12f1-base editing fusion protein may comprise an additional domain involved in base editing, in addition to the base editing domain. The additional domain may serve to assist a base editing function of the deaminase or increase efficiency thereof. The dCas12f1-base editing fusion protein may comprise one, two, or three or more of the additional domains.

In an embodiment, the additional domain may be a uracil glycosylase inhibitor. In another embodiment, the additional domain may be a gam protein.

### Other component 2 - NLS, linker, and the like

The dCas 12f1-base editing fusion protein disclosed herein may further comprise at least one additional domain. The additional domain may be located at the N-terminus and/or C-terminus of the dCas12f1-base editing fusion protein. The additional domain may be located between the dead Cas12f1 protein and the base editing domain which are included in the dCas12f1-base editing fusion protein.

The dCas12f1-base editing fusion protein may further comprise one or more of NLS (nuclear localization sequence) and/or NES (nuclear export sequence). The NLS may be as described in the subsection "Nuclear localization sequence or signal (NLS)" of the section "Definition of terms."

The dCas12f1-base editing fusion protein may comprise at least one linker. The linker links respective components in the fusion protein, and is not particularly limited as long as it does not affect functions of the respective components. The respective components of the dCas12f1-base editing fusion protein may be directly linked to each other, or may be linked via a linker. The linker may be as described in the subsection "Linker" of the section "Definition of terms."

### Structure of dCas12f1-base editing fusion protein

The dCas12f1-base editing fusion protein disclosed herein is a fusion protein with a structure in which the dead Cas12f1 protein and the base editing domain are sequentially linked to each other. Here, the dCas12f1-base editing fusion protein is characterized in that it can be designed by variously combining the base editing domain and the additional component(s), in terms of type, number, combination, and fusion position. Such various modularization methods for the dCas12f1-base editing fusion protein enable development of more effective dCas12f1-base editing fusion proteins by utilizing the advantage that the Cas12f1 protein has a small size. Depending on configuration of each module, the dCas12f1-base editing fusion protein may have different base editing efficiency; and an optimal CRISPR base editing system may be designed through various modularization methods depending on each target gene.

In an embodiment, the dCas12f1-base editing fusion protein may comprise at least one base editing domain. In an embodiment, the dCas12f1-base editing fusion protein may comprise at least one other domain involved in base editing. In an embodiment, the dCas12f1-base editing fusion protein may comprise at least one other component.

In an embodiment, the dead Cas12f1 protein and the base editing domain may be directly linked to each other, or may be linked via a linker. In an embodiment, based on the structure in which the dead Cas12f1 protein and the base editing domain are linked to each other, the dCas12f1-base editing fusion protein may comprise various additional components.

In an embodiment, the dCas12f1-base editing fusion protein may be represented by a structure selected from the following:

NH₂-[dCas12f1]-[Linker]-[BE]-COOH;

and

NH₂-[BE]-[Linker]-[dCas12f1]-COOH.

In an embodiment, the dCas12f1-base editing fusion protein may be represented by the structure shown in FIG. 1.

### Exemplary sequences for dCas12f1-base editing fusion protein

In an embodiment, the dCas12f1-base editing fusion protein may be represented by an amino acid sequence selected from SEQ ID NOS: 284 to 328, and SEQ ID NOS: 418 to 442.

### dCas12f1-expression regulating fusion protein

### Overview of dCas12f1-expression regulating fusion protein

Disclosed herein is a dCas12f1-expression regulating fusion protein in which the dead Cas12f1 protein and a gene expression regulatory domain are fused together. The dCas12f1-expression regulating fusion protein is a protein in which the dead Cas12f1 protein, at least one gene expression regulatory domain, and other component(s) are fused together, and upon delivery into a cell, it may function together with a guide RNA to regulate expression of a predetermined target gene in the cell. The dCas12f1-expression regulating fusion protein may essentially comprise the dead Cas12f1 protein and at least one gene regulatory domain, and may comprise an additional component(s). As a result, the dCas12f1-expression regulating fusion protein may serve as a CRISPR activation system that promotes expression of a gene and/or a CRISPR interference system that inhibits expression of a gene.

### Component 1 of dCas12f1-expression regulating fusion protein - dead Cas12f1 protein

The dCas12f1-expression regulating fusion protein includes a dead Cas12f1 protein that has lost nucleic acid cleavage activity. As described above, the dead Cas12f1 protein recognizes a predetermined target sequence in a target gene and functions to bind to the corresponding site, so that it serves to allow the gene expression regulatory domain, which is another component of the dCas12f1-expression regulating fusion protein, to regulate expression of the target gene. In other words, the dead Cas12f1 protein is a moiety designed to position-specifically deliver the expression regulating domain to a target gene site to be regulated. The dead Cas12f1 protein is characterized by loss of nucleic acid cleavage activity, and thus does not cause unwanted nucleic acid cleavage and indel generation. A specific configuration of the dead Cas12f1 protein is as described in the section "Dead Cas12f1 protein."

### Component 2 of dCas12f1-expression regulating fusion protein - gene expression regulatory domain

The dCas 12f1-expression regulating fusion protein comprises a gene expression regulatory domain. The gene expression regulatory domain is not particularly limited as long as it is capable of regulating expression of a target gene. More specifically, the gene expression regulatory domain may be a transcriptional activator protein that serves to activate or promote transcription of a specific gene, and/or a transcriptional inhibitor protein that serves to inhibit or suppress transcription of a specific gene. The present inventors fused such a gene expression regulatory domain to the dead Cas12f1, and thus have invented a dCas12f1-expression regulating fusion protein that can form a CRISPR activation system and a CRISPR interference system.

### Gene expression regulatory domain 1 - transcriptional promoter factor

The gene expression regulatory domain may be a transcriptional activator protein. The transcriptional activator protein may be a protein that serves to activate or promote transcription of a target gene. The transcriptional activator protein may be a DNA-binding protein capable of binding to an enhancer or promoter-proximal element of a target gene. The transcriptional activator protein may bind to a regulatory DNA site located near a promoter and make protein-protein interactions with general transcription machinery (RNA polymerase and general transcription factors), thereby facilitating binding of the transcription machinery to the promoter so that transcription of a gene is promoted. Alternatively, the transcriptional activator protein may trigger RNA polymerase to be released from a promoter and proceed with synthesis along DNA so that transcription of a gene is promoted.

In an embodiment, the transcriptional activator protein may be VP64, Sun Tag, VPR (VP64, p65, Rta), or TV (TAL, VP64).

### Gene expression regulatory domain 2 - transcriptional inhibitor factor

The expression regulatory domain may be a transcriptional inhibitor protein. The transcriptional inhibitor protein may be a protein that serves to inhibit or suppress transcription of a target gene. The transcriptional inhibitor protein may be a DNA-binding protein or peptide that binds to an operator or silencer of a target gene to inhibit or suppress expression of the target gene. Here, the transcriptional inhibitor protein may block RNA polymerase from attaching to a promoter, thereby inhibiting or suppressing transcription of a gene. Alternatively, the transcriptional inhibitor protein may be a protein or peptide that induces structural change in chromatin to inhibit or suppress transcription of a gene. Here, the structural change in chromatin may be caused by methylation, demethylation, acetylation, deacetylation, or the like.

In an embodiment, the transcriptional inhibitor protein may be KRAB, DNMT, MeCP2, HDAC, LSD, SRDX SALL1, and/or SDS3. Here, DNMT may be DNMT1, TRDMT1, or DNMT3. Here, HDAC may be HDAC1, HDAC2, HDAC3, HDAC4, HDAC5, HDAC6, HDAC7, HDAC8, HDAC9, HDAC10, or HDAC11.

### Other component - NLS, linker, and the like

The dCas 12f1-expression regulating fusion protein disclosed herein may further comprise at least one additional domain. The additional domain may be located at the N-terminus and/or C-terminus of the dCas12f1-expression regulating fusion protein. Alternatively, the additional domain may be located between the dead Cas12f1 protein and the gene expression regulatory domain which are included in the dCas12f1-expression regulating fusion protein.

The Cas12f1 fusion protein provided herein is characterized by comprising a linker that links the modified Cas12f1 protein and the expression regulatory domain. Here, the linker is characterized by an amino acid sequence that does not affect functions and structures of the modified Cas 12f1 protein and the expression regulatory domain. Specifically, the linker may be as described in the subsection "Linker" of the section "Definition of terms."

The dCas12f1-expression regulating fusion protein may further comprise one or more of NLS (nuclear localization sequence) and/or NES (nuclear export sequence). Specifically, the NLS may be as described in the subsection "Nuclear localization sequence or signal (NLS)" of the section "Definition of terms."

The dCas12f1-expression regulating fusion protein may comprise at least one tag. Specifically, the tag may be as described in the subsection "Tag" of the section "Definition of terms."

### Structure of dCas12f1-expression regulating fusion protein

The dCas 12f1-expression regulating fusion protein disclosed herein is a fusion protein with a structure in which the dead Cas12f1 protein and the gene expression regulatory domain are sequentially linked to each other. Here, the dCas12f1-expression regulating fusion protein is characterized in that it can be designed by variously combining the gene expression regulatory domain and the additional component(s), in terms of type, number, combination, and fusion position. Such various modularization methods for the dCas 12f1-expression regulating fusion protein enable development of more effective dCas12f1-expression regulating fusion proteins by utilizing the advantage that the Cas12f1 protein has a small size. Depending on configuration of each module, the dCas12f1-expression regulating fusion protein may have different expression regulation efficiency; and an optimal CRISPR expression regulating system may be designed through various modularization methods depending on each target gene.

In an embodiment, the dCas12f1-expression regulating fusion protein may comprise at least one gene expression regulatory domain. In an embodiment, the dCas12f1-expression regulating fusion protein may comprise at least one other domain involved in expression regulation. In an embodiment, the dCas12f1-expression regulating fusion protein may comprise at least one other component.

In an embodiment, the dead Cas12f1 protein and the expression regulatory domain may be directly linked to each other, or may be linked via a linker and/or NLS. In an embodiment, based on the structure in which the dead Cas12f1 protein and the expression regulatory domain are linked to each other, the dCas12f1-expression regulating fusion protein may comprise various additional components.

Specific exemplary configurations are described in the subsection "dCas12f1-expression regulatory domain fusion protein" of the section "Possible Embodiments of Invention."

### Guide RNA

### Overview of guide RNA

Disclosed herein is a guide RNA that is capable of forming a complex with the dead Cas12f1 and the dCas12f1-based fusion protein (hereinafter collectively referred to as engineered Cas12f1 protein) and allows the engineered Cas12f1 protein to function in a target-specific manner. The guide RNA is capable of forming a CRISPR/Cas12f1 system with the wild-type Cas12f1 to exhibit double-stranded nucleic acid cleavage activity, and was invented by the present inventors by performing various modifications based on a wild-type guide RNA. The guide RNA is such that a scaffold, a spacer, and a U-rich tail are sequentially linked to each other in a 5' to 3' direction.

The scaffold is a moiety that is capable of interacting with the engineered Cas12f1 protein to form a complex and corresponds to tracrRNA and crRNA direct repeat portions of a wild-type guide RNA; and the scaffold is a portion of a wild-type guide RNA that is modified in various ways. The spacer is a moiety that is capable of binding complementarily to a target sequence targeted by the engineered Cas12f1 protein, and a sequence thereof is determined depending on the target sequence. The U-rich tail is a moiety that is located at the 3' end of the spacer and is rich in uridine (U). The U-rich tail acts in synergy with the scaffold to increase efficiency with which the engineered Cas12f1 functions in a target-specific manner.

### Scaffold

The scaffold is a moiety that is capable of interacting with the engineered Cas12f1 protein to form a complex, and is a component specific to the (engineered) Cas12f1 disclosed herein. The scaffold is made by applying various manipulations to a tracrRNA portion and a crRNA direct repeat portion of a wild-type Cas12f1 guide RNA, and comprises an engineered tracrRNA portion and an engineered crRNA direct repeat portion which correspond to a tracrRNA portion of a wild-type guide RNA and a crRNA direct repeat portion of a wild-type guide RNA. For efficient expression and use, the scaffold generally takes a form in which the engineered tracrRNA portion and the engineered crRNA direct repeat portion are linked via a linker; however, the linker is not essential. In other words, the scaffold may be composed of one molecule of nucleic acid, or may be composed of two molecules of nucleic acid in which a part of each nucleic acid molecule forms complementary bonds to each other.

In an embodiment, the scaffold may have a structure of 5'-[engineered tracrRNA]-linker-[engineered crRNA direct repeat]-3'. Various embodiments for each component of the scaffold are described in the section "Possible Embodiments of Invention."

### Spacer

The spacer is a moiety designed to specifically bind to a target sequence in a target gene and function in a target-specific manner after the engineered Cas12f1 protein and the guide RNA form a complex. The spacer is an RNA sequence that recognizes, binds to, or targets a target gene. More specifically, the spacer is an RNA sequence that binds complementarily to a target sequence, an RNA sequence that is capable of forming a complementary bond with a target sequence, or an RNA sequence that has complementarity to a target sequence. The spacer is an RNA sequence that is identical to, similar to, or corresponds to a protospacer sequence. Here, the protospacer sequence has a close relationship with a target sequence, and description therefor is as described in the subsection "Target sequence, target strand, and non-target strand" in the section "Definition of terms." The spacer is a sequence that changes depending on a target sequence. The spacer varies depending on a target sequence. In addition, the spacer is an RNA sequence and comprises uridine (U) capable of forming a complementary bond to adenosine (A) present in a target sequence of a target gene. Alternatively, for thymidine (T) present in a protospacer sequence of a target gene, the spacer comprises uridine (U) instead of thymidine (T). In addition, the spacer is also referred to as a guide domain. Hereinafter, the spacer and the guide domain may be used interchangeably.

### U-rich tail

The U-rich tail is a moiety that is located at the 3' end of the spacer and is rich in uridine (U). Specifically, the U-rich tail acts in synergy with the scaffold to increase efficiency with which the engineered Cas12f1 functions in a target-specific manner. For example, the U-rich tail may be UUUUAUUUUUU (SEQ ID NO: 231). Various embodiments of the U-rich tail are described in the section "Possible Embodiments of Invention."

### Engineered Cas12f1 protein - guide RNA complex

### Components of engineered Cas12f1 protein-guide RNA complex

Disclosed herein is an engineered Cas12f1 protein-guide RNA complex. The engineered Cas12f1 protein-guide RNA complex comprises 1) an engineered Cas12f1 protein selected from a dead Cas12f1 protein, a dCas 12f1-base editing fusion protein, and a dCas12f1-expression regulating fusion protein, and 2) a guide RNA. Here, each of the dead Cas12f1 protein, the dCas12f1-base editing fusion protein, and the dCas12f1-expression regulating fusion protein is as described above.

The engineered Cas12f1 protein-guide RNA complex may be referred to as a CRISPR gene regulating complex (system). For example, in a case where the engineered Cas12f1 protein is a dCas12f1-base editing fusion protein, the engineered Cas12f1 protein-guide RNA complex may be referred to as a CRISPR base editing complex (system). As another example, in a case where the engineered Cas12f1 protein is a dCas12f1-expression regulating fusion protein, the engineered Cas12f1 protein-guide RNA complex may be referred to as a CRISPR expression regulating complex (system).

### Engineered Cas12f1 protein

The engineered Cas 12f1 protein-guide RNA complex comprises an engineered Cas12f1 protein. The engineered Cas12f1 protein collectively refers to the above-described dead Cas12f1 protein, dCas12f1-base editing fusion protein, and dCas12f1-expression regulating fusion protein, and may be appropriately selected and optimized depending on an intended use of the CRISPR gene regulating complex. Description for each of the dead Cas 12f1 protein, the dCas12f1-base editing fusion protein, and the dCas12f1-expression regulating fusion protein is as described in the corresponding section.

### Guide RNA

The engineered Cas12f1 protein-guide RNA complex comprises a guide RNA. The guide RNA is capable of performing its function in a CRISPR/Cas 12f1 system, as described in the corresponding section. A scaffold in the guide RNA may interact with the engineered Cas12f1 protein to form a complex.

### Engineered Cas12f1 protein-guide RNA complex 1 - CRISPR base editing complex

In an embodiment, in a case where the engineered Cas12f1 is a dCas12f1-base editing fusion protein, the CRISPR gene regulating complex has a function of editing at least one base in a target gene, which may be referred to as a CRISPR base editing complex (system).

Specifically, in a case where the dCas 12f1-base editing fusion protein comprises adenosine deaminase as a base editing domain, the CRISPR base editing complex may be referred to as a CRISPR adenine base editor (ABE) complex (system), which is capable of binding to a target sequence in a target gene and editing an adenine base, which is present in or adjacent to a protospacer sequence, with a guanine base.

Specifically, in a case where the dCas 12f1-base editing fusion protein comprises cytidine deaminase as a base editing domain, the CRISPR base editing complex may be referred to as a CRISPR cytosine base editor (CBE) complex (system), which is capable of binding to a target sequence in a target gene and editing a cytosine base, which is present in or adjacent to a protospacer sequence, with a thymine base.

### Engineered Cas12f1 protein-guide RNA complex 2 - CRISPR activation/interference complex

In an embodiment, in a case where the engineered Cas12f1 is a dCas12f1-expression regulating fusion protein, the CRISPR gene regulating complex has a function of regulating (promoting or inhibiting) expression of a target gene, which may be referred to as a CRISPR expression regulating complex (system).

Specifically, in a case where the dCas12f1-expression regulating fusion protein comprises a transcriptional activator protein as an expression regulatory domain, the CRISPR expression regulating complex may be referred to as a CRISPR activation complex (system), which is capable of binding to a regulatory DNA site located near an enhancer or promoter and triggering RNA polymerase to be released from a promoter and proceed with synthesis along DNA so that transcription of a gene is promoted.

On the other hand, in a case where the dCas12f1-expression regulating fusion protein comprises a transcriptional inhibitor protein as an expression regulatory domain, the CRISPR expression regulating complex may be referred to as a CRISPR interference complex (system), which is capable of binding to an operator or silencer of a target gene to block RNA polymerase from attaching to a promoter, thereby inhibiting or suppressing transcription of the gene.

### Vector encoding engineered Cas12f1 protein and guide RNA

### Vector encoding engineered Cas12f1 protein and guide RNA - overview

Provided herein is a vector for expressing respective components of the CRISPR gene regulation system. Specifically, the vector comprises a nucleic acid encoding an engineered Cas12f1 protein and a nucleic acid encoding a guide RNA, and is configured to be capable of expressing the engineered Cas12f1 protein and the guide RNA. A sequence of the vector comprises at least one promoter sequence. The promoter is operably linked to a nucleotide sequence encoding the engineered Cas12f1 protein and/or a nucleotide sequence encoding the guide RNA so that transcription of the nucleotide sequence(s) can be promoted in a cell. Here, the engineered Cas12f1 protein is selected from dead Cas12f1 protein, dCas12f1-base editing fusion protein, and dCas12f1-expression regulating fusion protein, and the respective proteins are as described in the sections "Dead Cas12f1 protein," "dCas12f1-base editing fusion protein," and "dCas12f1-expression regulating fusion protein." In addition, the guide RNA is as described in the section "Guide RNA."

### Component of vector - engineered Cas12f1 protein-expressing sequence

The vector comprises a nucleic acid encoding the engineered Cas12f1 protein. Here, the engineered Cas12f1 protein is selected from dead Cas12f1 protein, dCas12f1-base editing fusion protein, and dCas12f1-expression regulating fusion protein, and the respective proteins are as described in the sections "Dead Cas12f1 protein," "dCas12f1-base editing fusion protein," and "dCas12f1-expression regulating fusion protein." The nucleic acid encoding the engineered Cas12f1 protein is a sequence capable of expressing the engineered Cas12f1 protein, and may be appropriately codon-optimized depending on an environment where it is expressed.

### Component of vector - guide RNA-expressing sequence

The vector comprises a nucleic acid encoding the guide RNA. Here, the guide RNA is as described in the section "Guide RNA."

### Component of vector - promoter sequence

A sequence of the vector comprises a promoter sequence operably linked to a sequence encoding each component. In order to cause an expression target of the vector to be expressed in a cell, a promoter sequence needs to be operably linked to a sequence encoding each component so that an RNA transcription factor can be activated in the cell. The promoter sequence may be designed differently depending on its corresponding RNA transcription factor or an environment where the expression target is expressed, and is not limited as long as the promoter sequence is capable of appropriately expressing each component of the CRISPR gene regulation system in the cell. The promoter sequence may be a promoter that promotes transcription of an RNA polymerase (for example, RNA Pol I, Pol II, or Pol III). For example, the promoter may be, but is not limited to, any one of an SV40 early promoter, a mouse mammary tumor virus long terminal repeat (LTR) promoter, an adenovirus major late promoter (Ad MLP), a herpes simplex virus (HSV) promoter, a cytomegalovirus (CMV) promoter such as a CMV immediate early promoter region (CMVIE), a rous sarcoma virus (RSV) promoter, a human U6 small nuclear promoter (U6) (Miyagishi et al., Nature Biotechnology 20, 497-500 (2002)), an enhanced U6 promoter (for example, Xia et al., Nucleic Acids Res. 2003 Sep. 1; 31(17)), a human H1 promoter (H1), and a 7SK promoter.

In an embodiment, a sequence of the vector may comprise a sequence encoding the engineered Cas12f1 protein, and a promoter sequence. Here, the promoter sequence may be operably linked to the sequence encoding the engineered Cas12f1 protein. In an embodiment, a sequence of the vector may comprise a sequence encoding the guide RNA and a promoter sequence. Here, the promoter sequence may be operably linked to the sequence encoding the guide RNA. In an embodiment, a sequence of the vector may comprise a sequence encoding the engineered Cas12f1 protein, a sequence encoding the guide RNA, and a promoter sequence. Here, the promoter sequence is operably linked to the sequence encoding the engineered Cas12f1 protein and the sequence encoding the guide RNA, wherein a transcription factor activated by the promoter sequence causes expression of the engineered Cas12f1 protein and the guide RNA.

### Component of vector - possible to comprise two or more promoter sequences

In an embodiment, a sequence of the vector may comprise a first promoter sequence, a first sequence encoding the engineered Cas12f1 protein, a second promoter sequence, and a second sequence encoding the guide RNA. Here, the first promoter sequence is operably linked to the first sequence and the second promoter sequence is operably linked to the second sequence, wherein transcription of the first sequence is induced by the first promoter sequence and transcription of the second sequence is induced by the second promoter sequence. Here, the first promoter and the second promoter may be the same type of promoter. Here, the first promoter and the second promoter may be different types of promoters.

In an embodiment, a sequence of the vector may comprise a first promoter sequence, a first sequence encoding the engineered Cas 12f1 protein, a second promoter sequence, a second sequence encoding a first guide RNA, a third promoter sequence, and a third sequence encoding a second guide RNA. Here, the first promoter sequence is operably linked to the first sequence, the second promoter sequence is operably linked to the second sequence, and the third promoter sequence is operably linked to the third sequence, wherein transcription of the first sequence is induced by the first promoter sequence, transcription of the second sequence is induced by the second promoter sequence, and transcription of the third sequence is induced by the third promoter sequence. Here, the second promoter and the third promoter may be the same type of promoters. Specifically, the second promoter sequence and the third promoter sequence may be U6 promoter sequences, but are not limited thereto. Here, the second promoter and the third promoter may be different types of promoters. Specifically, the second promoter may be a U6 promoter sequence, and the third promoter may be an H1 promoter sequence; however, these promoters are not limited thereto.

### Component of vector - terminating signal

The vector may comprise a termination signal operably linked to the promoter sequence. In a case where a sequence of the vector comprises the promoter sequence, transcription of a sequence operably linked to the promoter is induced by an RNA transcription factor; and a sequence, which induces transcription termination of the RNA transcription factor, is referred to as a termination signal. The termination signal may vary depending on the type of promoter sequence. For example, in a case where the promoter is a U6 or H1 promoter, the promoter recognizes a thymidine contiguous sequence (for example, TTTTTT (T6)) as a termination signal.

In an embodiment, in a case where a sequence of the vector comprises a U6 promoter sequence, a thymidine contiguous sequence operably linked to the U6 promoter sequence may serve as a termination signal. In an embodiment, the thymidine contiguous sequence may be a sequence in which five or more thymidines are continuously linked. In an embodiment, in a case where a sequence of the vector comprises an H1 promoter sequence, a thymidine contiguous sequence operably linked to the H1 promoter sequence may serve as a termination signal. In an embodiment, the thymidine contiguous sequence may be a sequence in which five or more thymidines are continuously linked.

### Component of vector - other components

A sequence of the vector may comprise a necessary component depending on the purpose in addition to the above components.

In an embodiment, a sequence of the vector may comprise a sequence of a regulatory/control component, and/or a sequence of an additional component. In an embodiment, the additional component may be added for the purpose of distinguishing transfected cells from non-transfected cells. Here, sequences of the regulatory/control component and the additional component may comprise, but are not limited to, promoter, enhancer, intron, polyadenylation signal, Kozak consensus sequence, internal ribosome entry site (IRES), splice acceptor, 2A sequence, and/or replication origin. Here, the origin of replication may be, but is not limited to, an f1 origin of replication, a SV40 origin of replication, a pMB 1 origin of replication, an adeno origin of replication, an AAV origin of replication, and/or a BBV origin of replication.

### Additional component

The vector may be configured to express additional components such as NLS and tag protein, in addition to the expression targets described above. In an embodiment, the additional component may be expressed independently of the engineered Cas12f1 protein and/or the guide RNA. In another embodiment, the additional component may be expressed in conjunction with the engineered Cas12f1 protein and/or the guide RNA. Here, the additional component may be a component that is generally expressed in a case where a CRISPR gene regulation system is to be expressed, and reference may be made to known techniques for details thereof. For example, the additional component may be one of the tags described in the subsection "Tag" of the section "Definition of terms", but is not limited thereto. For example, the additional component may be a herbicide resistance gene such as glyphosate, glufosinate ammonium, or phosphinothricin; or an antibiotic resistance gene such as ampicillin, kanamycin, G418, bleomycin, hygromycin, or chloramphenicol, but is not limited thereto.

### Type of vector - viral vector

### The vector may be a viral vector.

In an embodiment, the viral vector may be at least one selected from the group consisting of retrovirus, lentivirus, adenovirus, adeno-associated virus, vaccinia virus, poxvirus, and herpes simplex virus. In an embodiment, the viral vector may be adeno-associated virus.

### Type of vector - non-viral vector

The vector may be a non-viral vector.

In an embodiment, the non-viral vector may be at least one selected from the group consisting of plasmid, phage, naked DNA, DNA complex, and mRNA. In an embodiment, the plasmid may be selected from the group consisting of pcDNA series, pS456, pG1806, pACYC177, ColE1, pKT230, pME290, pBR322, pUC8/9, pUC6, pBD9, pHC79, pIJ61, pLAFR1, pHV14, pGEX series, pET series, and pUC19. In an embodiment, the phage may be selected from the group consisting of λgt4λB, λ-Charon, λΔz1, and M13. In an embodiment, the vector may be a PCR amplicon.

### Form of vector - circular or linear vector

The vector may have a circular or linear form. In a case where the vector is a linear vector, RNA transcription is terminated at the 3' end even if a sequence of the linear vector does not separately comprise a termination signal. In contrast, in a case where the vector is a circular vector, RNA transcription is not terminated unless a sequence of the circular vector separately comprises a termination signal. Therefore, in a case where the vector is used in a form of a circular vector, a termination signal corresponding to a transcription factor related to each promoter sequence has to be included in order for the vector to express an intended target.

### Engineered CRISPR/Cas12f1 composition

Disclosed herein is an engineered CRISPR/Cas12f1 composition comprising respective components of the CRISPR gene regulating complex, or a nucleic acid encoding the same. Specifically, the engineered CRISPR/Cas12f1 composition comprises an engineered Cas12f1 protein or a nucleic acid encoding the Cas12f1 protein, and a guide RNA or a nucleic acid encoding the guide RNA. Here, the engineered Cas12f1 protein is selected from dead Cas12f1 protein, dCas12f1-base editing fusion protein, and dCas12f1-expression regulating fusion protein, as described in each of the sections above. The guide RNA is as described in the section "Guide RNA." The engineered CRISPR/Cas12f1 composition may further comprise an appropriate additional component in addition to the above components.

### Gene regulation method using engineered CRISPR/Cas12f1 composition - common

### Gene regulation method using CRISPR complex - overview

Disclosed herein is a method for regulating a target gene in a cell using the engineered CRISPR/Cas12f1 composition. Here, the "gene regulation" collectively refers to artificial genetic manipulation and function regulation, such as cleavage of gene, promotion of mutation generation, base editing, promotion of gene expression, and inhibition of gene expression, and is not limited to specific methods. Therefore, it should be understood that the term "gene regulation method" comprehensively refers to a base editing method for a target gene, a method of regulating expression of a target gene, and other methods of regulating a gene.

Disclosed specifically herein is 1) a method of base editing for a target gene using an engineered CRISPR/Cas12f1 composition comprising the dCas12f1-base editing fusion protein, and 2) a method of regulating expression of a target gene using an engineered CRISPR/Cas12f1 composition comprising the dCas12f1-expression regulating fusion protein.

From the viewpoint that the methods of the present disclosure all comprise introducing, delivering, administering, and/or injecting the engineered CRISPR/Cas12f1 composition into a target cell, common parts of the methods will be first described, and then the base editing method and the expression regulation method will be described separately.

### Target cell

In an embodiment, the target cell may be a prokaryotic cell. In an embodiment, the target cell may be a eukaryotic cell. Specifically, the eukaryotic cell may be, but is not limited to, a plant cell, an animal cell, and/or a human cell.

### Determination of target sequence

A target gene to be edited by a CRISPR base editing complex and/or a target gene whose expression is to be regulated by a CRISPR expression regulating complex may be determined in consideration of the purpose, an environment of a target cell, a PAM sequence recognized by an engineered Cas12f1 protein of the engineered CRISPR/Cas12f1 composition, and/or other variables. Here, there is no particular limitation on a method as long as it is capable of determining a target sequence of an appropriate length which is present in a target gene; and a technique known in the art may be used therefor.

### Determination of spacer sequence depending on target sequence

Once the target sequence is determined, a spacer sequence corresponding thereto is designed. The spacer sequence is designed as a sequence capable of binding complementarily to the target sequence. In an embodiment, the spacer sequence is designed as a sequence capable of binding complementarily to the target gene. In an embodiment, the spacer sequence is designed to be capable of binding complementarily to the target nucleic acid. In an embodiment, the spacer sequence is designed as a sequence complementary to a target sequence included in a target strand sequence of the target nucleic acid. In an embodiment, the spacer sequence is designed as an RNA sequence corresponding to a DNA sequence of a protospacer included in a non-target strand sequence of the target nucleic acid. Specifically, the spacer sequence is designed to have the same nucleotide sequence as the protospacer sequence, except that every thymidine included in the nucleotide sequence is replaced with uridine.

### Complementarity between target sequence and spacer sequence

In an embodiment, the spacer sequence may be complementary to the target sequence by 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. In an embodiment, the spacer sequence may be a sequence complementary to the target sequence within a numerical range selected from the immediately preceding sentence. As an example, the spacer sequence may be a sequence that is 60% to 90% complementary to the target sequence. As another example, the spacer sequence may be a sequence that is 90% to 100% complementary to the target sequence.

### Number of mismatches between target sequence and spacer sequence

In an embodiment, the spacer sequence may be a sequence that is complementary to the target sequence and has 0, 1, 2, 3, 4, 5, 6, 7, 8, 9, or 10 mismatches therewith. In an embodiment, the spacer sequence may have mismatches within a numerical range selected from the immediately preceding sentence. As an example, the spacer sequence may have 0, 1, 2, 3, 4, or 5 mismatches with the target sequence. As another example, the spacer sequence may have 6 to 10 mismatches with the target sequence.

### Delivery of respective components of engineered Cas 12f1-guide RNA complex into cell

The gene expression regulation method provided herein is on the premise that a CRISPR gene regulating complex comes in contact with a target gene in a target cell. Accordingly, in order to induce the CRISPR gene regulating complex to come in contact with the target gene, the gene expression regulation method comprises delivering respective components of the CRISPR gene regulating complex into the target cell.

In an embodiment, the gene expression regulation method may comprise delivering a guide RNA or a nucleic acid encoding the same and an engineered Cas12f1 protein or a nucleic acid encoding the same into a target cell. In an embodiment, the gene expression regulation method may comprise delivering a guide RNA and an engineered Cas12f1 protein into a target cell. In an embodiment, the gene expression regulation method may comprise delivering a nucleic acid encoding a guide RNA and an engineered Cas12f1 protein into a target cell. In an embodiment, the gene expression regulation method may comprise delivering a guide RNA and a nucleic acid encoding an engineered Cas12f1 protein into a target cell. In an embodiment, the gene expression regulation method may comprise delivering a nucleic acid encoding a guide RNA and a nucleic acid encoding an engineered Cas 12f1 protein into a target cell. The guide RNA or the nucleic acid encoding the same, and the engineered Cas12f1 protein or the nucleic acid encoding the same may be delivered into the target cell in various forms of delivery using various delivery methods.

### Form 1 of delivery - RNP

As the form of delivery, a ribonucleoprotein (RNP), in which a guide RNA and an engineered Cas12f1 protein are bound to each other, may be used. In an embodiment, the gene expression regulation method may comprise introducing a CRISPR gene regulating complex, in which a guide RNA and an engineered Cas12f1 protein are bound to each other, into a target cell.

### Form 2 of delivery - non-viral vector

As another form of delivery, a non-viral vector, which comprises a nucleotide sequence encoding a guide RNA and a nucleotide sequence encoding an engineered Cas12f1 protein, may be used. In an embodiment, the gene expression regulation method may comprise introducing, into a target cell, a non-viral vector that comprises a nucleotide sequence encoding a guide RNA and a nucleotide sequence encoding an engineered Cas12f1 protein. Specifically, the non-viral vector may be a plasmid, naked DNA, a DNA complex, or mRNA, but is not limited thereto. In another embodiment, the gene expression regulation method may comprise introducing, into a target cell, a first non-viral vector that comprises a nucleotide sequence encoding a guide RNA, and a second non-viral vector that comprises a nucleotide sequence encoding an engineered Cas12f1 protein. Specifically, each of the first non-viral vector and the second non-viral vector may be one selected from the group consisting of a plasmid, naked DNA, a DNA complex, and mRNA, but is not limited thereto.

### Form 3 of delivery - viral vector

As another form of delivery, a viral vector, which comprises a nucleotide sequence encoding a guide RNA and a nucleotide sequence encoding an engineered Cas12f1 protein, may be used. In an embodiment, the gene expression regulation method may comprise introducing, into a target cell, a viral vector that comprises a nucleotide sequence encoding a guide RNA and a nucleotide sequence encoding an engineered Cas12f1 protein. Specifically, the viral vector may be one selected from the group consisting of retrovirus, lentivirus, adenovirus, adeno-associated virus, vaccinia virus, poxvirus, and herpes simplex virus, but is not limited thereto. In an embodiment, the viral vector may be an adeno-associated virus.

In another embodiment, the gene expression regulation method may comprise introducing, into a target cell, a first viral vector that comprises a nucleotide sequence encoding a guide RNA, and a second viral vector that comprises a nucleotide sequence encoding an engineered Cas12f1 protein. Specifically, each of the first viral vector and the second viral vector may be one selected from the group consisting of retrovirus, lentivirus, adenovirus, adeno-associated virus, vaccinia virus, poxvirus, and herpes simplex virus, but is not limited thereto.

### Delivery method 1 - common means of delivery

The delivery method is not particularly limited as long as it is capable of delivering, into a cell, a guide RNA or a nucleic acid encoding the same, and an engineered Cas12f1 protein or a nucleic acid encoding the same in an appropriate form of delivery. In an embodiment, the delivery method may be electroporation, gene gun, sonoporation, magnetofection, and/or transient cell compression or squeezing.

### Delivery method 2 - nanoparticles

The delivery method may be delivering at least one component, which is included in the CRISPR gene regulation system, using nanoparticles. Here, the delivery method may be a method known in the art which can be appropriately selected by those skilled in the art. For example, the nanoparticle delivery method may be a method disclosed in WO 2019/089820 A1, but is not limited thereto.

In an embodiment, the delivery method may be delivering, using nanoparticles, an engineered Cas12f1 protein or a nucleic acid encoding the same and/or a guide RNA or a nucleic acid encoding the same. In an embodiment, the delivery method may be delivering, using nanoparticles, an engineered Cas12f1 protein or a nucleic acid encoding the same, a first guide RNA or a nucleic acid encoding the same, and/or a second guide RNA or a nucleic acid encoding the same. Here, the delivery method may be cationic liposome method, lithium acetate-dimethyl sulfoxide (DMSO), lipid-mediated transfection, calcium phosphate precipitation, lipofection, polyethyleneimine (PEI)-mediated transfection, diethylaminoethyl (DEAE)-dextran-mediated transfection, and/or nanoparticle-mediated nucleic acid delivery (see Panyam et., al Adv Drug Deliv Rev. 2012 Sep 13. pii: S0169-409X(12)00283-9. doi: 10.1016/j.addr.2012.09.023), but is not limited thereto. Here, components of the CRISPR gene regulation system may be in a form of an RNP, a non-viral vector, and/or a viral vector. For example, each of the components of the CRISPR gene regulation system may be in a form of mRNA encoding the same, but is not limited thereto.

### Combination of form and method of delivery

The gene expression regulation method comprises delivering, into a cell, a guide RNA or a nucleic acid encoding the same, and an engineered Cas12f1 protein or a nucleic acid encoding the same, wherein delivery forms and/or delivery methods of respective components may be the same as or different from each other. In an embodiment, the gene expression regulation method may comprise delivering a guide RNA or a nucleic acid encoding the same in a first form of delivery and delivering an engineered Cas12f1 protein or a nucleic acid encoding the same in a second form of delivery. Here, each of the first form of delivery and the second form of delivery may be any one of the above-described forms of delivery. In an embodiment, the gene expression regulation method may comprise delivering a guide RNA or a nucleic acid encoding the same in a first method of delivery, and delivering an engineered Cas12f1 protein or a nucleic acid encoding the same in a second method of delivery. Here, each of the first method of delivery and the second method of delivery may be any one of the above-described methods of delivery.

### Order of delivery

The gene expression regulation method comprises delivering, into a cell, a guide RNA or a nucleic acid encoding the same, and an engineered Cas12f1 protein or a nucleic acid encoding the same, wherein the components may be delivered into the cell simultaneously or sequentially with a time interval.

In an embodiment, the gene expression regulation method may comprise delivering a guide RNA or a nucleic acid encoding the same and an engineered Cas12f1 protein or a nucleic acid encoding the same simultaneously. In an embodiment, the gene expression regulation method may comprise delivering a guide RNA or a nucleic acid encoding the same into a cell, and then delivering an engineered Cas12f1 protein or a nucleic acid encoding the same into the cell at a time interval. In an embodiment, the gene expression regulation method may comprise delivering an engineered Cas12f1 protein or a nucleic acid encoding the same into a cell, and then delivering a guide RNA into the cell at a time interval. In an embodiment, the gene expression regulation method may comprise delivering a nucleic acid encoding an engineered Cas12f1 protein into a cell, and then delivering a guide RNA into the cell at a time interval.

### Bringing CRISPR gene regulating complex in contact with target nucleic acid

In the gene expression regulation method disclosed herein, regulation of expression of a target gene is performed as a CRISPR gene regulating complex comes in contact with the target gene. Accordingly, the gene expression regulation method may comprise bringing the CRISPR gene regulating complex in contact with a target cell or inducing the CRISPR gene regulating complex to come in contact therewith. In an embodiment, the gene expression regulation method may comprise bringing the CRISPR gene regulating complex with a target gene in the target cell. In an embodiment, the gene expression regulation method may comprise inducing the CRISPR gene regulating complex to come in contact with a target gene in the target cell. Here, the induction method is not particularly limited as long as it allows the CRISPR gene regulating complex to come in contact with a target gene in the cell. In an embodiment, the induction may be achieved by delivering, into a cell, a guide RNA or a nucleic acid encoding the same, and an engineered Cas12f1 protein or a nucleic acid encoding the same.

### Base editing method using engineered CRISPR/Cas12f1 composition

### Overview of base editing method

In the genetic methods described in the section "Gene regulation method using engineered CRISPR/Cas12f1 composition," the gene expression regulation method may be referred to as a base editing method using an engineered CRISPR/Cas 12f1 composition in a case where 1) a purpose of the method is to edit, replace, or change a specific base contained in a target gene to or with a base of interest, 2) the engineered Cas12f1 protein of the engineered CRISPR/Cas12f1 composition is described in the section "dCas12f1-base editing fusion protein," and 3) the target sequence is designed as a sequence adjacent to the specific base to be edited.

As a result of performing the base editing method, a specific base (for example, adenine or cytosine) in a target gene is edited to a base of interest (for example, guanine or thymine).

### Base editing mechanism 1 - meaning of base editing

The base editing method disclosed herein allows a specific base of a target gene in a cell to be edited to a base of interest. Hereinafter, a position of the base to be edited and an editing range therefor will be described in detail.

In a case where the target gene is double-stranded DNA, a strand having a target sequence is referred to as a target strand, and a strand having a PAM sequence and a protospacer sequence is referred to as a non-target strand. The base editing method allows a CRISPR base editing complex to be formed within the cell, in which the CRISPR base editing complex binds to a target sequence. Here, at least one specific base in the protospacer sequence (or a sequence adjacent thereto), which is a sequence complementary to the target sequence, is modified by the CRISPR base editing complex. Then, based on the above modification, the cell's own base repair mechanism causes nucleotide sequence modification in the sequence of the target gene, which is called base editing.

In an embodiment, the base editing may be such that at least one adenine in the protospacer sequence is modified into guanine. In another embodiment, the base editing may be such that at least one cytosine in the protospacer sequence is modified into thymine.

### Base editing mechanism 2 - base editing range

A range of positions where the base editing occurs may vary depending on the configuration and type of the dCas12f1-base editing fusion protein included in the CRISPR base editing complex. Here, the range of positions where the base editing can occur may be referred to as an editing window.

In the base editing method disclosed herein, unless otherwise stated, in a case where the base at the 5' end of the protospacer sequence is set as position 1 (first position), the editing window may be referred to as position 2 (second position), position 3 (third position), and position n (n^{th} position, n is a natural number) in a direction toward the 3' end (downstream direction). If necessary, in a case where the base at the 5' end of the protospacer sequence is set as position 1 (first position), the editing window may be referred to as position 0 (base at the 3' end of the PAM sequence), position -1, position -2, and position n (n is a negative number other than 0) in a direction toward the 5' end (upstream direction).

In an embodiment, as a result of performing the base editing method, in a case where the base at the 5' end of the protospacer sequence is set as position 1, the base at position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 in a direction toward the 3' end may be edited. Here, the base editing window may be a range of positions consisting of two numerical values selected from the immediately preceding sentence. For example, the base editing window may be a range of positions 2 to 8. In an embodiment, the base editing may be such that at least one adenine within the base editing window is modified into guanine. In another embodiment, the base editing may be such that at least one cytosine within the base editing window is modified into thymine.

### Base editing mechanism 3 - number of bases edited

The base editing is not limited to modification of one base, and two, three, four, or five or more bases may be modified simultaneously.

In an embodiment, the base editing may be such that based on the base at the 5' end of the protospacer sequence, at least one adenine within a base editing window from positions 2 to 8 in a direction toward 3' end is modified into guanine. In another embodiment, the base editing may be such that based on the base at the 5' end of the protospacer sequence, at least one cytosine within a base editing window from positions 3 to 5 in a direction toward 3' end is modified into thymine.

### Results obtained by performing base editing method

As a result of performing the base editing method provided herein, a specific base in a protospacer contained in a target gene, or a nucleic acid adjacent to the protospacer, and/or a nucleic acid adjacent to a target sequence is edited to, replaced with, or changed to a base of interest.

In an embodiment, the editing of the specific base to the base of interest may be editing of adenine to guanine. In an embodiment, the editing of the specific base to the target base may be editing of cytosine to thymine.

### Gene expression regulation method using engineered CRISPR/Cas12f1 composition

### Overview of gene expression regulation method

In the genetic method described in the section "Gene expression regulation method using engineered CRISPR/Cas12f1 composition," the gene expression regulation method may be referred to as a gene expression regulation method using an engineered CRISPR/Cas12f1 composition in a case where 1) a purpose of the method is to promote/activate or inhibit/deactivate expression of a target gene, 2) the engineered Cas12f1 protein of the engineered CRISPR/Cas12f1 composition is described in the section "dCas12f1-expresion regulating fusion protein," and 3) the target sequence is designed as a target gene whose expression is to be regulated, or a nucleic acid adjacent thereto.

As a result of performing the base editing method, expression of the target gene may be promoted (or increased) or suppressed (or inhibited).

### Results obtained by performing gene expression regulation method

As a result of performing the gene expression regulation method provided herein, expression of the target gene may be promoted (or increased) or suppressed (or inhibited). Here, the expression may mean transcription of the target gene into mRNA. Generally, in a case where expression of the target gene is promoted (or increased), an expression level of the mRNA of the gene is increased and production of the protein encoded by the gene is increased. In addition, in a case where expression of the target gene is suppressed (or inhibited), an expression level of the mRNA of the gene is decreased and production of the protein encoded by the gene is decreased. In an embodiment, as a result of performing the gene expression regulation method, production of the protein encoded by the target gene may be increased or decreased.

### Possible Embodiments of Invention

Hereinafter, possible embodiments provided by the present disclosure are described below. The following embodiments provided in this section are merely examples of the present disclosure. Therefore, the present disclosure should not be interpreted as limited to the following examples. In addition, the brief description provided along with the example number is only for convenience in distinguishing the respective examples and should not be construed as limiting the present disclosure.

### Dead Cas12f1 protein

### Example 1. Dead Cas12f1, WT-based

A dead Cas12f1 protein,
wherein the dead Cas12f1 protein is such that in a wild-type Cas12f1 represented by MAKNTITKTLKLRIVRPYNSAEVEKIVADEKNNREKIALEKNKDKVKEACSKH LKVAAYCTTQVERNACLFCKARKLDDKFYQKLRGQFPDAVFWQEISEIFRQLQ KQAAEIYNQSLIELYYEIFIKGKGIANASSVEHYLSDVCYTRAAELFKNAAIAS GLRSKIKSNFRLKELKNMKSGLPTTKSDNFPIPLUKQKGGQYTGFEISNHNSDF IIKIPFGRWQVKKEIDKYRPWEKFDFEQVQKSPKPISLLLSTQRRKRNKGWSK DEGTEAEIKKVMNGDYQTSYIEVKRGSKIGEKSAWMLNLSIDVPKIDKGVDPS IIGGIDVGVKSPLVCAINNAFSRYSISDNDLFHFNKKMFARRRILLKKNRHKRA GHGAKNKLKPITILTEKSERFRKKLIERWACEIADFFIKNKVGTVQMENLESMK RKEDSYFNIRLRGFWPYAEMQNKIEFKLKQYGIEIRKVAPNNTSKTCSKCGHL NNYFNFEYRKKNKFPHFKCEKCNFKENADYNAALNISNPKLKSTKEEP (SEQ ID NO: 1), at least one amino acid selected from the following is replaced with alanine, glutamine, leucine, tryptophan, or valine:
aspartic acid at position 326; glutamic acid at position 422; arginine at position 490; and aspartic acid at position 510.

### Example 2, WT-based, sequence defined

The dead Cas12f1 protein of Example 1, wherein the dead Cas12f1 protein has an amino acid sequence selected from: 
MAKNTITKTLKLRIVRPYNSAEVEKIVADEKNNREKIALEKNKDKVKE
ACSKHLKVAAYCTTQVERNACLFCKARKLDDKFYQKLRGQFPDAVFWQEISE
IFRQLQKQAAEIYNQSLIELYYEIFIKGKGIANASSVEHYLSDVCYTRAAELFK
NAAIASGLRSKIKSNFRLKELKNMKSGLPTTKSDNFPIPLVKQKGGQYTGFEIS
NHNSDFIIKIPFGRWQVKKEIDKYRPWEKFDFEQVQKSPKPISLLLSTQRRKRN
KGWSKDEGTEAEIKKVMNGDYQTSYIEVKRGSKIGEKSAWMLNLSIDVPKID
 
KGVDPSIIGGIAVGVKSPLVCAINNAFSRYSISDNDLFHFNKKMFARRRILLKKN
RHKRAGHGAKNKLKPITILTEKSERFRKKLIERWACEIADFFIKNKVGTVQME
NLESMKRKEDSYFNIRLRGFWPYAEMQNKIEFKLKQYGIEIRKVAPNNTSKTC
SKCGHLNNYFNFEYRKKNKFPHFKCEKCNFKENADYNAALNISNPKLKSTKE
EP (SEQ ID NO: 2),
 
MAKNTITKTLKLRIVRPYNSAEVEKIVADEKNNREKIALEKNKDKVKE
ACSKHLKVAAYCTTQVERNACLFCKARKLDDKFYQKLRGQFPDAVFWQEISE
IFRQLQKQAAEIYNQSLIELYYEIFIKGKGIANASSVEHYLSDVCYTRAAELFK
NAAIASGLRSKIKSNFRLKELKNMKSGLPTTKSDNFPIPLVKQKGGQYTGFEIS
NHNSDFIIKIPFGRWQVKKEIDKYRPWEKFDFEQVQKSPKPISLLLSTQRRKRN
KGWSKDEGTEAEIKKVMNGDYQTSYIEVKRGSKIGEKSAWMLNLSIDVPKID
KGVDPSIIGGIDVGVKSPLVCAINNAFSRYSISDNDLFHFNKKMFARRRILLKK
NRHKRAGHGAKNKLKPITILTEKSERFRKKLIERWACEIADFFIKNKVGTVQM
ANLESMKRKEDSYFNIRLRGFWPYAEMQNKIEFKLKQYGIEIRKVAPNNTSKT
CSKCGHLNNYFNFEYRKKNKFPHFKCEKCNFKENADYNAALNISNPKLKSTK
EEP (SEQ ID NO: 3),
 
MAKNTITKTLKLRIVRPYNSAEVEKIVADEKNNREKIALEKNKDKVKE
ACSKHLKVAAYCTTQVERNACLFCKARKLDDKFYQKLRGQFPDAVFWQEISE
IFRQLQKQAAEIYNQSLIELYYEIFIKGKGIANASSVEHYLSDVCYTRAAELFK
NAAIASGLRSKIKSNFRLKELKNMKSGLPTTKSDNFPIPLVKQKGGQYTGFEIS
NHNSDFIIKIPFGRWQVKKEIDKYRPWEKFDFEQVQKSPKPISLLLSTQRRKRN
KGWSKDEGTEAEIKKVMNGDYQTSYIEVKRGSKIGEKSAWMLNLSIDVPKID
KGVDPSIIGGIDVGVKSPLVCAINNAFSRYSISDNDLFHFNKKMFARRRILLKK
NRHKRAGHGAKNKLKPITILTEKSERFRKKLIERWACEIADFFIKNKVGTVQM
ENLESMKRKEDSYFNIRLRGFWPYAEMQNKIEFKLKQYGIEIRKVAPNNTSKT
CSKCGHLNNYFNFEYAKKNKFPHFKCEKCNFKENADYNAALNISNPKLKSTK
EEP (SEQ ID NO: 4),
 
MAKNTITKTLKLRIVRPYNSAEVEKIVADEKNNREKIALEKNKDKVKE
ACSKHLKVAAYCTTQVERNACLFCKARKLDDKFYQKLRGQFPDAVFWQEISE
 
IFRQLQKQAAEIYNQSLIELYYEIFIKGKGIANASSVEHYLSDVCYTRAAELFK
NAAIASGLRSKIKSNFRLKELKNMKSGLPTTKSDNFPIPLVKQKGGQYTGFEIS
NHNSDFIIKIPFGRWQVKKEIDKYRPWEKFDFEQVQKSPKPISLLLSTQRRKRN
KGWSKDEGTEAEIKKVMNGDYQTSYIEVKRGSKIGEKSAWMLNLSIDVPKID
KGVDPSIIGGIDVGVKSPLVCAINNAFSRYSISDNDLFHFNKKMFARRRILLKK
NRHKRAGHGAKNKLKPITILTEKSERFRKKLIERWACEIADFFIKNKVGTVQM
ENLESMKRKEDSYFNIRLRGFWPYAEMQNKIEFKLKQYGIEIRKVAPNNTSKT
CSKCGHLNNYFNFEYRKKNKFPHFKCEKCNFKENAAYNAALNISNPKLKSTK
EEP (SEQ ID NO: 5),
 
MAKNTITKTLKLRIVRPYNSAEVEKIVADEKNNREKIALEKNKDKVKE
ACSKHLKVAAYCTTQVERNACLFCKARKLDDKFYQKLRGQFPDAVFWQEISE
IFRQLQKQAAEIYNQSLIELYYEIFIKGKGWANASSVEHYLSDVCYTRAAELFK
NAAIASGLRSKIKSNFRLKELKNMKSGLPTTKSDNFPIPLVKQKGGQYTGFEIS
NHNSDFIIKIPFGRWQVKKEIDKYRPWEKFDFEQVQKSPKPISLLLSTQRRKRN
KGWSKDEGTEAEIKKVMNGDYQTSYIEVKRGSKIGEKSAWMLNLSIDVPKID
KGVDPSIIGGIDVGVKSPLVCAINNAFSRYSISDNDLFHFNKKMFARRRILLKK
NRHKRAGHGAKNKLKPITILTEKSERFRKKLIERWACEIADFFIKNKVGTVQM
ENLESMKRKEDSYFNIRLRGFWPYAEMQNKIEFKLKQYGIEIRKVAPNNTSKT
CSKCGHLNNYFNFEYRKKNKFPHFKCEKCNFKENAAYNAALNISNPKLKSTK
EEP (SEQ ID NO: 6),
 
MAKNTITKTLKLRIVRPYNSAEVEKIVADEKNNREKIALEKNKDKVKE
ACSKHLKVAAYCTTQVERNACLFCKARKLDDKFYQKLRGQFPDAVFWQEISE
IFRQLQKQAAEIYNQSLIELYYEIFIKGKGIANASYVEHYLSDVCYTRAAELFK
NAAIASGLRSKIKSNFRLKELKNMKSGLPTTKSDNFPIPLVKQKGGQYTGFEIS
NHNSDFIIKIPFGRWQVKKEIDKYRPWEKFDFEQVQKSPKPISLLLSTQRRKRN
KGWSKDEGTEAEIKKVMNGDYQTSYIEVKRGSKIGEKSAWMLNLSIDVPKID
KGVDPSIIGGIDVGVKSPLVCAINNAFSRYSISDNDLFHFNKKMFARRRILLKK
NRHKRAGHGAKNKLKPITILTEKSERFRKKLIERWACEIADFFIKNKVGTVQM
ENLESMKRKEDSYFNIRLRGFWPYAEMQNKIEFKLKQYGIEIRKVAPNNTSKT
CSKCGHLNNYFNFEYRKKNKFPHFKCEKCNFKENAAYNAALNISNPKLKSTK
 EEP (SEQ ID NO: 7), and

MAKNTITKTLKLRIVRPYNSAEVEKIVADEKNNREKIALEKNKDKVKE
ACSKHLKVAAYCTTQVERNACLFCKARKLDDKFYQKLRGQFPDAVFWQEISE
IFRQLQKQAAEIYNQSLIELYYEIFIKGKGWANASYVEHYLSDVCYTRAAELFK
NAAIASGLRSKIKSNFRLKELKNMKSGLPTTKSDNFPIPLVKQKGGQYTGFEIS
NHNSDFIIKIPFGRWQVKKEIDKYRPWEKFDFEQVQKSPKPISLLLSTQRRKRN
KGWSKDEGTEAEIKKVMNGDYQTSYIEVKRGSKIGEKSAWMLNLSIDVPKID
KGVDPSIIGGIDVGVKSPLVCAINNAFSRYSISDNDLFHFNKKMFARRRILLKK
NRHKRAGHGAKNKLKPITILTEKSERFRKKLIERWACEIADFFIKNKVGTVQM
ENLESMKRKEDSYFNIRLRGFWPYAEMQNKIEFKLKQYGIEIRKVAPNNTSKT
CSKCGHLNNYFNFEYRKKNKFPHFKCEKCNFKENAAYNAALNISNPKLKSTK
EEP (SEQ ID NO: 8).

### Example 3. Dead Cas12f1, variant 1-based

A dead Cas12f1 protein,
wherein the dead Cas12f1 protein is such that in a Cas12f1 variant protein represented by MGEKSSRRRRNGKSGAWTAAITSCVGGKMAKNTITKTLKLRIVRPYNSAEVE KIVADEKNNREKIALEKNKDKVKEACSKHLKVAAYCTTQVERNACLFCKARK LDDKFYQKLRGQFPDAVFWQEISEIFRQLQKQAAEIYNQSLIELYYEIFIKGKGI ANASSVEHYLSDVCYTRAAELFKNAAIASGLRSKIKSNFRLKELKNMKSGLPT TKSDNFPIPLVKQKGGQYTGFEISNHNSDFIIKIPFGRWQVKKEIDKYRPWEKF DFEQVQKSPKPISLLLSTQRRKRNKGWSKDEGTEAEIKKVMNGDYQTSYIEV KRGSKIGEKSAWMLNLSIDVPKIDKGVDPSIIGGIDVGVKSPLVCAINNAFSRY SISDNDLFHFNKKMFARRRILLKKNRHKRAGHGAKNKLKPITILTEKSERFRK KLIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCEK CNFKENADYNAALNISNPKLKSTKEEP (SEQ ID NO: 14), at least one amino acid selected from the following is replaced with alanine, glutamine, leucine, tryptophan, or valine:
aspartic acid at position 354; glutamic acid at position 450; arginine at position 518; and aspartic acid at position 538.

### Example 4. Variant 1-based, sequence defined

The dead Cas12f1 protein of Example 3, wherein the dead Cas12f1 protein has an amino acid sequence selected from:

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIAVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYRKKNKFPHFKC
EKCNFKENADYNAALNISNPKLKSTKEEP (SEQ ID NO: 15),

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMANLESMKRKEDSYFNIRLRGFWPYAE
MQNKIEFKLKQYGIEIRKVAPNNTSKTCSKCGHLNNYFNFEYRKKNKFPHFKC
EKCNFKENADYNAALNISNPKLKSTKEEP (SEQ ID NO: 16),

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC

KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYAKKNKFPHFKC
EKCNFKENADYNAALNISNPKLKSTKEEP (SEQ ID NO: 17),

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYRKKNKFPHFKC
EKCNFKENAAYNAALNISNPKLKSTKEEP (SEQ ID NO: 18),

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGWANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYRKKNKFPHFKC

EKCNFKENAAYNAALNISNPKLKSTKEEP (SEQ ID NO: 19),

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASYVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYRKKNKFPHFKC
EKCNFKENAAYNAALNISNPKLKSTKEEP (SEQ ID NO: 20),

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGWANASYVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYRKKNKFPHFKC
EKCNFKENAAYNAALNISNPKLKSTKEEP (SEQ ID NO: 21),

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT

SYIEVKRGSKIGEKSAWMLNLSIDVPKIDKGVDPSIIGGIAVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYRKKNKFPHFKC
EKCNFKENAAYNAALNISNPKLKSTKEEP (SEQ ID NO: 22),

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIAVGVKSPLVCAINNA
FSRYSISDNDLFHFNKKMFARRRILLKKNRHKRAGHGAKNKLKPITILTEKSER
FRKKLIERWACEIADFFIKNKVGTVQMANLESMKRKEDSYFNIRLRGFWPYAE
MQNKIEFKLKQYGIEIRKVAPNNTSKTCSKCGHLNNYFNFEYAKKNKFPHFKC
EKCNFKENADYNAALNISNPKLKSTKEEP (SEQ ID NO: 23), and

MGEKSSRRRRNGKSGAWTAAITSCVGGKMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIAVGVKSPLVCAINNA
FSRYSISDNDLFHFNKKMFARRRILLKKNRHKRAGHGAKNKLKPITILTEKSER
FRKKLIERWACEIADFFIKNKVGTVQMANLESMKRKEDSYFNIRLRGFWPYAE
MQNKIEFKLKQYGIEIRKVAPNNTSKTCSKCGHLNNYFNFEYAKKNKFPHFKC
EKCNFKENAAYNAALNISNPKLKSTKEEP (SEQ ID NO: 24).

### Example 5. Dead Cas12f1, variant 2-based

A dead Cas12f1 protein,
wherein the dead Cas12f1 protein is such that in a Cas12f1 variant protein represented by MAGGPGAGSAAPVSSTSSLPLAALNMRVMAKNTITKTLKLRIVRPYNSAEVE KIVADEKNNREKIALEKNKDKVKEACSKHLKVAAYCTTQVERNACLFCKARK LDDKFYQKLRGQFPDAVFWQEISEIFRQLQKQAAEIYNQSLIELYYEIFIKGKGI ANASSVEHYLSDVCYTRAAELFKNAAIASGLRSKIKSNFRLKELKNMKSGLPT TKSDNFPIPLVKQKGGQYTGFEISNHNSDFIIKIPFGRWQVKKEIDKYRPWEKF DFEQVQKSPKPISLLLSTQRRKRNKGWSKDEGTEAEIKKVMNGDYQTSYIEV KRGSKIGEKSAWMLNLSIDVPKIDKGVDPSIIGGIDVGVKSPLVCAINNAFSRY SISDNDLFHFNKKMFARRRILLKKNRHKRAGHGAKNKLKPITILTEKSERFRK KLIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCEK CNFKENADYNAALNISNPKLKSTKEEP (SEQ ID NO: 25), at least one amino acid selected from the following is replaced with alanine, glutamine, leucine, tryptophan, or valine:
aspartic acid at position 354; glutamic acid at position 450; arginine at position 518; and aspartic acid at position 538.

### Example 6. Variant 2-based, sequence defined

The dead Cas12f1 protein of Example 5, wherein the dead Cas12f1 protein has an amino acid sequence selected from:

MAGGPGAGSAAPVSSTSSLPLAALNMRVMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIAVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE

MQNKIEFKLKQYGIEIRKVAPNNTSKTCSKCGHLNNYFNFEYRKKNKFPHFKC
EKCNFKENADYNAALNISNPKLKSTKEEP (SEQ ID NO: 26),

MAGGPGAGSAAPVSSTSSLPLAALNMRVMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMANLESMKRKEDSYFNIRLRGFWPYAE
MQNKIEFKLKQYGIEIRKVAPNNTSKTCSKCGHLNNYFNFEYRKKNKFPHFKC
EKCNFKENADYNAALNISNPKLKSTKEEP (SEQ ID NO: 27),

MAGGPGAGSAAPVSSTSSLPLAALNMRVMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYAKKNKFPHFKC
EKCNFKENADYNAALNISNPKLKSTKEEP (SEQ ID NO: 28), and

MAGGPGAGSAAPVSSTSSLPLAALNMRVMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR

PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIDVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYRKKNKFPHFKC
EKCNFKENAAYNAALNISNPKLKSTKEEP (SEQ ID NO: 29).

### Example 7. Dead Cas12f1, variant 3-based

A dead Cas12f1 protein,
wherein the dead Cas12f1 protein is such that in a Cas12f1 variant protein represented by MAGGPGAGSAAPVSSTSSLPLAALNMMAKNTITKTLKLRIVRPYNSAEVEKIV ADEKNNREKIALEKNKDKVKEACSKHLKVAAYCTTQVERNACLFCKARKLD DKFYQKLRGQFPDAVFWQEISEIFRQLQKQAAEIYNQSLIELYYEIFIKGKGIAN ASSVEHYLSDVCYTRAAELFKNAAIASGLRSKIKSNFRLKELKNMKSGLPTTK SDNFPIPLVKQKGGQYTGFEISNHNSDFIIKIPFGRWQVKKEIDKYRPWEKFDF EQVQKSPKPISLLLSTQRRKRNKGWSKDEGTEAEIKKVMNGDYQTSYIEVKR GSKIGEKSAWMLNLSIDVPKIDKGVDPSIIGGIDVGVKSPLVCAINNAFSRYSIS DNDLFHFNKKMFARRRILLKKNRHKRAGHGAKNKLKPITILTEKSERFRKKLI ERWACEIADFFIKNKVGTVQMENLESMKRKEDSYFNIRLRGFWPYAEMQNKI EFKLKQYGIEIRKVAPNNTSKTCSKCGHLNNYFNFEYRKKNKFPHFKCEKCNF KENADYNAALNISNPKLKSTKEEP (SEQ ID NO: 30), at least one amino acid selected from the following is replaced with alanine, glutamine, leucine, tryptophan, or valine:
aspartic acid at position 352; glutamic acid at position 448; arginine at position 516; and aspartic acid at position 536.

### Example 8. Variant 3-based, sequence defined

The dead Cas12f1 protein of Example 7, wherein the dead Cas12f1 protein has an amino acid sequence selected from: 
MAGGPGAGSAAPVSSTSSLPLAALNMRVMAKNTITKTLKLRIVRPYNS
AEVEKIVADEKNNREKIALEKNKDKVKEACSKHLKVAAYCTTQVERNACLFC
KARKLDDKFYQKLRGQFPDAVFWQEISEIFRQLQKQAAEIYNQSLIELYYEIFI
KGKGIANASSVEHYLSDVCYTRAAELFKNAAIASGLRSKIKSNFRLKELKNM
KSGLPTTKSDNFPIPLVKQKGGQYTGFEISNHNSDFIIKIPFGRWQVKKEIDKYR
PWEKFDFEQVQKSPKPISLLLSTQRRKRNKGWSKDEGTEAEIKKVMNGDYQT
SYIEVKRGSKIGEKSAWMLNLSIDVPKIDKGVDPSIIGGIAVGVKSPLVCAINNA
FSRYSISDNDLFHFNKKMFARRRILLKKNRHKRAGHGAKNKLKPITILTEKSER
FRKKLIERWACEIADFFIKNKVGTVQMENLESMKRKEDSYFNIRLRGFWPYAE
MQNKIEFKLKQYGIEIRKVAPNNTSKTCSKCGHLNNYFNFEYRKKNKFPHFKC
EKCNFKENADYNAALNISNPKLKSTKEEP (SEQ ID NO: 31),
 
MAGGPGAGSAAPVSSTSSLPLAALNMMAKNTITKTLKLRIVRPYNSAE
VEKIVADEKNNREKIALEKNKDKVKEACSKHLKVAAYCTTQVERNACLFCKA
RKLDDKFYQKLRGQFPDAVFWQEISEIFRQLQKQAAEIYNQSLIELYYEIFIKG
KGIANASSVEHYLSDVCYTRAAELFKNAAIASGLRSKIKSNFRLKELKNMKSG
LPTTKSDNFPIPLVKQKGGQYTGFEISNHNSDFIIKIPFGRWQVKKEIDKYRPWE
KFDFEQVQKSPKPISLLLSTQRRKRNKGWSKDEGTEAEIKKVMNGDYQTSYIE
VKRGSKIGEKSAWMLNLSIDVPKIDKGVDPSIIGGIDVGVKSPLVCAINNAFSR
YSISDNDLFHFNKKMFARRRILLKKNRHKRAGHGAKNKLKPITILTEKSERFR
KKLIERWACEIADFFIKNKVGTVQMANLESMKRKEDSYFNIRLRGFWPYAEM
QNKIEFKLKQYGIEIRKVAPNNTSKTCSKCGHLNNYFNFEYRKKNKFPHFKCE
KCNFKENADYNAALNISNPKLKSTKEEP (SEQ ID NO: 32),
 
MAGGPGAGSAAPVSSTSSLPLAALNMMAKNTITKTLKLRIVRPYNSAE
VEKIVADEKNNREKIALEKNKDKVKEACSKHLKVAAYCTTQVERNACLFCKA
RKLDDKFYQKLRGQFPDAVFWQEISEIFRQLQKQAAEIYNQSLIELYYEIFIKG
KGIANASSVEHYLSDVCYTRAAELFKNAAIASGLRSKIKSNFRLKELKNMKSG
LPTTKSDNFPIPLVKQKGGQYTGFEISNHNSDFIIKIPFGRWQVKKEIDKYRPWE
KFDFEQVQKSPKPISLLLSTQRRKRNKGWSKDEGTEAEIKKVMNGDYQTSYIE
VKRGSKIGEKSAWMLNLSIDVPKIDKGVDPSIIGGIDVGVKSPLVCAINNAFSR
YSISDNDLFHFNKKMFARRRILLKKNRHKRAGHGAKNKLKPITILTEKSERFR
 
KKLIERWACEIADFFIKNKVGTVQMENLESMKRKEDSYFNIRLRGFWPYAEM
QNKIEFKLKQYGIEIRKVAPNNTSKTCSKCGHLNNYFNFEYAKKNKFPHFKCE
KCNFKENADYNAALNISNPKLKSTKEEP (SEQ ID NO: 33),
 and

MAGGPGAGSAAPVSSTSSLPLAALNMMAKNTITKTLKLRIVRPYNSAE
VEKIVADEKNNREKIALEKNKDKVKEACSKHLKVAAYCTTQVERNACLFCKA
RKLDDKFYQKLRGQFPDAVFWQEISEIFRQLQKQAAEIYNQSLIELYYEIFIKG
KGIANASSVEHYLSDVCYTRAAELFKNAAIASGLRSKIKSNFRLKELKNMKSG
LPTTKSDNFPIPLVKQKGGQYTGFEISNHNSDFIIKIPFGRWQVKKEIDKYRPWE
KFDFEQVQKSPKPISLLLSTQRRKRNKGWSKDEGTEAEIKKVMNGDYQTSYIE
VKRGSKIGEKSAWMLNLSIDVPKIDKGVDPSIIGGIDVGVKSPLVCAINNAFSR
YSISDNDLFHFNKKMFARRRILLKKNRHKRAGHGAKNKLKPITILTEKSERFR
KKLIERWACEIADFFIKNKVGTVQMENLESMKRKEDSYFNIRLRGFWPYAEM
QNKIEFKLKQYGIEIRKVAPNNTSKTCSKCGHLNNYFNFEYRKKNKFPHFKCE
KCNFKENAAYNAALNISNPKLKSTKEEP (SEQ ID NO: 34).

### Example 9. Dead Cas12f1, variant 4-based

A dead Cas12f1 protein,
wherein the dead Cas12f1 protein is such that in wild-type Cas12f1 represented by MEKRINKIRKKLSADNATKPVSRSGPMAKNTITKTLKLRIVRPYNSAEVEKIVA DEKNNREKIALEKNKDKVKEACSKHLKVAAYCTTQVERNACLFCKARKLDD KFYQKLRGQFPDAVFWQEISEIFRQLQKQAAEIYNQSLIELYYEIFIKGKGIANA SSVEHYLSDVCYTRAAELFKNAAIASGLRSKIKSNFRLKELKNMKSGLPTTKS DNFPIPLVKQKGGQYTGFEISNHNSDFIIKIPFGRWQVKKEIDKYRPWEKFDFE QVQKSPKPISLLLSTQRRKRNKGWSKDEGTEAEIKKVMNGDYQTSYIEVKRG SKIGEKSAWMLNLSIDVPKIDKGVDPSIIGGIDVGVKSPLVCAINNAFSRYSISD NDLFHFNKKMFARRRILLKKNRHKRAGHGAKNKLKPITILTEKSERFRKKLIE RWACEIADFFIKNKVGTVQMENLESMKRKEDSYFNIRLRGFWPYAEMQNKIE FKLKQYGIEIRKVAPNNTSKTCSKCGHLNNYFNFEYRKKNKFPHFKCEKCNFK ENADYNAALNISNPKLKSTKEEP (SEQ ID NO: 35), at least one amino acid selected from the following is replaced with alanine, glutamine, leucine, tryptophan, or valine:
aspartic acid at position 352; glutamic acid at position 448; arginine at position 516; and aspartic acid at position 536.

### Example 10. Variant 4-based, sequence defined

The dead Cas12f1 protein of Example 9, wherein the dead Cas12f1 protein has an amino acid sequence selected from: 
MEKRINKIRKKLSADNATKPVSRSGPMAKNTITKTLKLRIVRPYNSAEV
EKIVADEKNNREKIALEKNKDKVKEACSKHLKVAAYCTTQVERNACLFCKAR
KLDDKFYQKLRGQFPDAVFWQEISEIFRQLQKQAAEIYNQSLIELYYEIFIKGK
GIANASSVEHYLSDVCYTRAAELFKNAAIASGLRSKIKSNFRLKELKNMKSGL
PTTKSDNFPIPLVKQKGGQYTGFEISNHNSDFIIKIPFGRWQVKKEIDKYRPWE
KFDFEQVQKSPKPISLLLSTQRRKRNKGWSKDEGTEAEIKKVMNGDYQTSYIE
VKRGSKIGEKSAWMLNLSIDVPKIDKGVDPSIIGGIAVGVKSPLVCAINNAFSR
YSISDNDLFHFNKKMFARRRILLKKNRHKRAGHGAKNKLKPITILTEKSERFR
KKLIERWACEIADFFIKNKVGTVQMENLESMKRKEDSYFNIRLRGFWPYAEM
QNKIEFKLKQYGIEIRKVAPNNTSKTCSKCGHLNNYFNFEYRKKNKFPHFKCE
KCNFKENADYNAALNISNPKLKSTKEEP (SEQ ID NO: 36),
 
MEKRINKIRKKLSADNATKPVSRSGPMAKNTITKTLKLRIVRPYNSAEV
EKIVADEKNNREKIALEKNKDKVKEACSKHLKVAAYCTTQVERNACLFCKAR
KLDDKFYQKLRGQFPDAVFWQEISEIFRQLQKQAAEIYNQSLIELYYEIFIKGK
GIANASSVEHYLSDVCYTRAAELFKNAAIASGLRSKIKSNFRLKELKNMKSGL
PTTKSDNFPIPLVKQKGGQYTGFEISNHNSDFIIKIPFGRWQVKKEIDKYRPWE
KFDFEQVQKSPKPISLLLSTQRRKRNKGWSKDEGTEAEIKKVMNGDYQTSYIE
VKRGSKIGEKSAWMLNLSIDVPKIDKGVDPSIIGGIDVGVKSPLVCAINNAFSR
YSISDNDLFHFNKKMFARRRILLKKNRHKRAGHGAKNKLKPITILTEKSERFR
KKLIERWACEIADFFIKNKVGTVQMANLESMKRKEDSYFNIRLRGFWPYAEM
QNKIEFKLKQYGIEIRKVAPNNTSKTCSKCGHLNNYFNFEYRKKNKFPHFKCE
KCNFKENADYNAALNISNPKLKSTKEEP (SEQ ID NO: 37),
 
MEKRINKIRKKLSADNATKPVSRSGPMAKNTITKTLKLRIVRPYNSAEV
EKIVADEKNNREKIALEKNKDKVKEACSKHLKVAAYCTTQVERNACLFCKAR
KLDDKFYQKLRGQFPDAVFWQEISEIFRQLQKQAAEIYNQSLIELYYEIFIKGK
GIANASSVEHYLSDVCYTRAAELFKNAAIASGLRSKIKSNFRLKELKNMKSGL
PTTKSDNFPIPLVKQKGGQYTGFEISNHNSDFIIKIPFGRWQVKKEIDKYRPWE
KFDFEQVQKSPKPISLLLSTQRRKRNKGWSKDEGTEAEIKKVMNGDYQTSYIE
VKRGSKIGEKSAWMLNLSIDVPKIDKGVDPSIIGGIDVGVKSPLVCAINNAFSR
YSISDNDLFHFNKKMFARRRILLKKNRHKRAGHGAKNKLKPITILTEKSERFR
KKLIERWACEIADFFIKNKVGTVQMENLESMKRKEDSYFNIRLRGFWPYAEM
QNKIEFKLKQYGIEIRKVAPNNTSKTCSKCGHLNNYFNFEYAKKNKFPHFKCE
KCNFKENADYNAALNISNPKLKSTKEEP (SEQ ID NO: 38),
 and

MEKRINKIRKKLSADNATKPVSRSGPMAKNTITKTLKLRIVRPYNSAEV
EKIVADEKNNREKIALEKNKDKVKEACSKHLKVAAYCTTQVERNACLFCKAR
KLDDKFYQKLRGQFPDAVFWQEISEIFRQLQKQAAEIYNQSLIELYYEIFIKGK
GIANASSVEHYLSDVCYTRAAELFKNAAIASGLRSKIKSNFRLKELKNMKSGL
PTTKSDNFPIPLVKQKGGQYTGFEISNHNSDFIIKIPFGRWQVKKEIDKYRPWE
KFDFEQVQKSPKPISLLLSTQRRKRNKGWSKDEGTEAEIKKVMNGDYQTSYIE
VKRGSKIGEKSAWMLNLSIDVPKIDKGVDPSIIGGIDVGVKSPLVCAINNAFSR
YSISDNDLFHFNKKMFARRRILLKKNRHKRAGHGAKNKLKPITILTEKSERFR
KKLIERWACEIADFFIKNKVGTVQMENLESMKRKEDSYFNIRLRGFWPYAEM
QNKIEFKLKQYGIEIRKVAPNNTSKTCSKCGHLNNYFNFEYRKKNKFPHFKCE
KCNFKENAAYNAALNISNPKLKSTKEEP (SEQ ID NO: 39).

### Example 11. Dead Cas12f1, represented by structural formula

A dead Cas12f1, represented by the following sequence:

### N-[dummy sequence]-[dead Cas12f1]-C

wherein the dummy sequence is an amino acid sequence having a 1-mer, 2-mer, 3-mer, 4-mer, 5-mer, 6-mer, 7-mer, 8-mer, 9-mer, 10-mer, 11-mer, 12-mer, 13-mer, 14-mer, 15-mer, 16-mer, 17-mer, 18-mer, 19-mer, 20-mer, 21-mer, 22-mer, 23-mer, 24-mer, 25-mer, 26-mer, 27-mer, 28-mer, 29-mer, 30-mer, 31-mer, 32-mer, 33-mer, 34-mer, 35-mer, 36-mer, 37-mer, 38-mer, 39-mer, 40-mer, 41-mer, 42-mer, 43-mer, 44-mer, 45-mer, 46-mer, 47-mer, 48-mer, 49-mer, or 50-mer length, and
the dead Cas12f1 has an amino acid sequence of 
MAKNTITKTLKLRIVRPYNSAEVEKIVADEKNNREKIALEKNKDKVKEACSKH
LKVAAYCTTQVERNACLFCKARKLDDKFYQKLRGQFPDAVFWQEISEIFRQLQ
KQAAEIYNQSLIELYYEIFIKGKGX1ANASX2VEHYLSDVCYTRAAELFKNAAI
ASGLRSKIKSNFRLKELKNMKSGLPTTKSDNFPIPLVKQKGGQYTGFEISNHNS
DFIIKIPFGRWQVKKEIDKYRPWEKFDFEQVQKSPKPISLLLSTQRRKRNKGWS
KDEGTEAEIKKVMNGDYQTSYIEVKRGSKIGEKSAWMLNLSIDVPKIDKGVD
PSIIGGIX3VGVKSPLVCAINNAFSRYSISDNDLFHFNKKMFARRRILLKKNRHK
RAGHGAKNKLKPITILTEKSERFRKKLIERWACEIADFFIKNKVGTVQMX4NLE
SMKRKEDSYFNIRLRGFWPYAEMQNKIEFKLKQYGIEIRKVAPNNTSKTCSKC
GHLNNYFNFEYX5KKNKFPHFKCEKCNFKENAX6YNAALNISNPKLKSTKEEP
(SEQ ID NO: 9),
wherein X₁ is isoleucine or tryptophan, X₂ is serine or tyrosine,
X₃ is aspartic acid or alanine X₄ is glutamic acid or alanine, X₅ is arginine, alanine, glutamine, leucine, or tryptophan, and X₆ is aspartic acid, alanine, leucine, or valine, and
at least one of X₃, X₄, X₅, and X₆ is alanine, glutamine, leucine, tryptophan, or valine.

### Example 12. Dummy sequence defined

The dead Cas 12f1 of Example 11, wherein the dummy sequence is selected from MGEKSSRRRRNGKSGAWTAAITSCVGGK (SEQ ID NO: 10), MAGGPGAGSAAPVSSTSSLPLAALNMRV (SEQ ID NO: 11), MAGGPGAGSAAPVSSTSSLPLAALNM (SEQ ID NO: 12), and MEKRINKIRKKLSADNATKPVSRSGP (SEQ ID NO: 13).

### Base editing domain

### Example 13. Deaminase

A base editing domain, comprising 1, 2, 3, 4, or 5 or more domain proteins,
wherein the domain protein is each independently all or a part of a protein (polypeptide) selected from:
Escherichia coli (E. coli)-derived tRNA adenosine deaminase (TadA); Escherichia coli (E. coli)-derived tRNA adenosine deaminase (TadA) variant; human activation-induced cytidine deaminase (AID); human APOBEC3G; murine APOBEC1; APOBEC3A; APOBEC3B; CDA; AID; and lamprey PmCDA1.

### Example 14. Adenosine deaminase

The base editing domain of Example 13, wherein the domain protein is each independently all or a part of a protein (polypeptide) selected from:
Escherichia coli (E. coli)-derived tRNA adenosine deaminase (TadA); and Escherichia coli (E. coli)-derived tRNA adenosine deaminase (TadA) variant, and
the domain protein may be referred to as adenosine deaminase.

### Example 15. Adenosine deaminase, sequence defined

The base editing domain of Example 14, wherein the TadA and/or the TadA variant is represented by an amino acid sequence selected from the following:

MSEVEFSHEYWMRHALTLAKRAWDEREVPVGAVLVHNNRVIGEGWN
RPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFR
MRRQEIKAQKKAQSSTD (SEQ ID NO: 241);

MSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGVRNAKTGAAGSLMDVLHYPGMNHRVEITEGILADECAALLCYFFR
MPRQVFNAQKKAQSSTD (SEQ ID NO: 242);

MSEVEFSHEYWMRHALTLAKRAWDEREVPVGAVLVHNNRVIGEGWN

RPIGRHDPTAHAAIMALRQGGLVMQNYRLIDATLYVTLEPCVMCAGAMIHSRI
GRVVFGARDAKTGAAGSLMDVLHHPGMNHRVEITEGILADECAALLSDFFR
MRRQEIKAQKKAQSSTD (SEQ ID NO: 243);

MSEVEFSHEYWMRHALTLAKRARDEREVPVGAVLVLNNRVIGEGWN
RAIGLHDPTAHAAIMALRQGGLVMQNYRLIDATLYVTFEPCVMCAGAMIHSRI
GRVVFGVRNAKTGAAGSLMDVLHYPGMNHRVEITEGILADECAALLCYFFR
MPRQVFNAQKKAQSSTD (SEQ ID NO: 244);

MSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGWRNSKRGAAGSLMNVLNYPGMNHRVEITEGILADECAALLCDFYR
MPRQVFNAQKKAQSSIN (SEQ ID NO: 245);

MSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGWRQSKRGAAGSLMNVLNYPGMNHRVEITEGILADECAALLCDFYR
MPRQVFNAQKKAQSSIN (SEQ ID NO: 246);

MVEFSDEYWMRHALTLAKRARDEGEVPVGAVLVLNNRVIGEGWNRA
IGLHDPTAHAEIMALRQGGQVLQNYRLIDATLYVTFEPCVMCAGAMIHSRIKR
VVFGWRNSKRGAAGSLMNVLNHPGMNHRVEITEGVLADECAALLSDFFRMR
RQQKKAQKK (SEQ ID NO: 247);

MVEFSDEYWMRHALTLAKRARDEGEVPVGAVLVLNNQVIGEGWNRSI
SLHDPTAHAEIMALRQGGQVLQNYRLIDCTLYVTFEPCVMCAGAMIHSRIKRV
VFGWRNPKRGAAGSLMNVLNHPGMNHRIEITEGVLADECAAMLSDFFRMRR
QQKKAQKK (SEQ ID NO: 248);

MVEFSDEYWMRHALTLAKRARDEGEVPVGAVLVLNNQVIGEGWNRSI
THHDPTAHAEIMALRQGGQVLQNYRLIDCTLYVTFEPCVMCAGAMIHARIKR
VVFGWRNPKRGAAGSVMNVLNHPGMNHRIEITEGVLADECAAMLSDFFRMR
RQQKKAQKK (SEQ ID NO: 249);

MSEVEFSHEYWMRHALTLAKRARDEREVPVGAVLVLNNRVIGEGWN

RAIGLHDPTAHAEIMALRQGGLVMQNYRLIDATLYVTFEPCVMCAGAMIHSRI
GRVVFGVRNSKRGAAGSLMNVLNYPGMNHRVEITEGILADECAALLCDFYR
MPRQVFNAQKKAQSSIN (SEQ ID NO: 250);

MSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGWRNAKTGAAGSLMDVLHYPGMNHRVEITEGILADECAALLCYFFR
MPRQVFNAQKKAQSSTD (SEQ ID NO: 251);

MSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGWRQAKTGAAGSLMDVLHYPGMNHRVEITEGILADECAALLCYFFR
MPRQVFNAQKKAQSSTD (SEQ ID NO: 252);

MSEVEFSHEYWMRHALTLAKRARDEREVPVGAVLVLNNRVIGEGWN
RAIGLHDPTAHAEIMALRQGGLVMQNYRLYDATLYSTFEPCVMCAGAMIHSRI
GRVVFGVRNAKTGAAGSLMDVLHHPGMNHRVEITEGILADECAALLCRFFR
MPRRVFNAQKKAQSSTD (SEQ ID NO: 253);

MSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGVRQAKTGAAGSLMDVLHYPGMNHRVEITEGILADECAALLCYFFR
MPRQVFNAQKKAQSSTD (SEQ ID NO: 254);

MSEVEFSHEYWMRHALTLAKRAWDEREVPVGAVLVHNNRVIGEGWN
RPIGRHDPTAHAEIMALRQGGLVMQNYRLIDATLYVTLEPCVMCAGAMIHSRI
GRVVFGARDAKTGAAGSLMDVLHHPGMNHRVEITEGILADECAALLSDFFR
MRRQEIKAQKKAQSSTD (SEQ ID NO: 255);

MSEVEFSHEYWMRHALTLAKRARDEREVPVGAVLVLNNRVIGEGWN
RAIGLHDPTAHAEIMALRQGGLVMQNYRLIDATLYVTFEPCVMCAGAMIHSRI
GRVVFGVRNAKTGAAGSLMDVLHYPGMNHRVEITEGILADECAALLCYFFR
MPRQVFNAQKKAQSSTD (SEQ ID NO: 256).

### Example 16. Structure of adenosine deaminase, Markush type

A base editing domain (adenosine deaminase), represented by the following structure:

NH₂-[Tad₁]-[Linker]-[Tad₂]-COOH

wherein Tad₁ is a first domain protein having an amino acid sequence selected from SEQ ID NOS: 241 to 256,
the Linker is a linker or absent,
the Tad₂ is a second domain protein having an amino acid sequence selected from SEQ ID NOS: 241 to 256, or is absent, and
in a case where the Tad₂ is absent, the Linker is also absent.

### Example 17. Markush type, linker defined

The base editing domain of Example 16, wherein the linker is represented by an amino acid sequence selected from SEQ ID NO: 260 to 273.

### Example 18. Cytidine deaminase

The base editing domain of Example 13, wherein the domain protein is each independently all or a part of a protein (polypeptide) selected from:
human activation-induced cytidine deaminase (AID); human APOBEC3G; murine APOBEC1; APOBEC3A; APOBEC3B; CDA; AID; and lamprey PmCDA1; and
the domain protein may be referred to as cytidine deaminase.

### Example 19. Cytidine deaminase, sequence defined

The base editing domain of Example 18, wherein the domain protein has an amino acid sequence selected from the following: 


 


 (SEQ ID NO: 257);
 
MEASPASGPRHLMDPHIFTSNFNNGIGRHKTYLCYEVERLDNGTSVKM
DQHRGFLHNQAKNLLCGFYGRHAELRFLDLVPSLQLDPAQIYRVTWFISWSPC
FSWGCAGEVRAFLQENTHVRLRIFAARIYDYDPLYKEALQMLRDAGAQVSIM
TYDEFKHCWDTFVDHQGCPFQPWDGLDEHSQALSGRLRAILQNQGN (SEQ ID
NO: 258);
 and








 (SEQ ID NO: 259).

### Example 20. Both adenosine/cytidine deaminase included

The base editing domain of Example 13, wherein the base editing domain comprises two or more domain proteins, and
the base editing domain comprises at least one adenosine deaminase that is any one of Examples 14 to 17, and at least one cytidine deaminase that is any one of Examples 18 to 19.

### Example 21. CBE, defined by structure shown in Experimental Example

The base editing domain of Example 20, wherein the base editing domain has a structure selected from the following:

NH₂-[ABE]-[Linker]-[CBE]-COOH;

and

NH₂-[CBE]-[Linker]-[ABE]-COOH,

wherein the ABE is adenosine deaminase that is any one of Examples 14 to 17, the CBE is cytidine deaminase that is any one of Examples 18 to 19, and the Linker is a linker or absent.

### Example 22. Linker added

The base editing domain of any one of Examples 13 to 21, wherein the base editing domain further comprises at least one linker, and the linker links the respective domain proteins, which are included in the base editing domain, to each other.

### Gene expression regulatory domain

### Example 23. Gene expression regulatory domain

A gene expression regulatory domain, comprising 1, 2, 3, 4, or 5 or more domain proteins,
wherein the domain protein is each independently all or a part of a protein (polypeptide) selected from:
VP64; Sun Tag; VPR (VP64, p65, Rta); TV (TAL, VP64); KRAB; DNMT; MeCP2; HDAC; LSD; SRDX SALL1; and SDS3.

### Example 24. Domain details defined

The gene expression regulatory domain of Example 23, wherein the DNMT is selected from DNMT1, TRDMT1, and DNMT3; and/or the HDAC is selected from HDAC1, HDAC2, HDAC3, HDAC4, HDAC5, HDAC6, HDAC7, HDAC8, HDAC9, HDAC10 and HDAC11.

### Example 25. Sequence defined

The gene expression regulatory domain of Example 23, wherein the gene expression regulatory domain comprises one or more of the domain proteins, and the domain proteins each independently have an amino acid sequence selected from the following: 
MDALDDFDLDMLGSDALDDFDLDMLGSDALDDFDLDMLGSDALDDF
DLDML (SEQ ID NO: 329);
 
RTLVTFKDVFVDFTREEWKLLDTAQQIVYRNVMLENYKNLVSLGYQL
TKPDVILRLEKGEEPWLV (SEQ ID NO: 330);
 
VQVKRVLEKSPGKLLVKMPFQASPGGKGEGGGATTSAQVMVIKRPGR
KRKAEADPQAIPKKRGRKPGSVVAAAAAEAKKKAVKESSIRSVQETVLPIKKR
KTRETVSIEVKEVVKPLLVSTLGEKSGKGLKTCKSPGRKSKESSPKGRSSSASS
PPKKEHHHHHHHAESPKAPMPLLPPPPPPEPQSSEDPISPPEPQDLSSSICKEEK
MPRAGSLESDGCPKEPAKTQPMVAAAATTTTTTTTTVAEKYKHRGEGERKDI
VSSSMPRPNREEPVDSRTPVTERVS (SEQ ID NO: 331);
 
PSRLQMFFANNHDQEFDPPKVYPPVPAEKRKPIRVLSLFDGIATGLLVL
KDLGIQVDRYIASEVCEDSITVGMVRHQGKIMYVGDVRSVTQKHIQEWGPFD
LVIGGSPCNDLSIVNPARKGLYEGTGRLFFEFYRLLHDARPKEGDDRPFFWLFE
NVVAMGVSDKRDISRFLESNPVMIDAKEVSAAHRARYFWGNLPGMNRPLAST
VNDKLELQECLEHGRIAKFSKVRTITTRSNSIKQGKDQHFPVFMNEKEDILWC
TEMERVFGFPVHYTDVSNMSRLARQRLLGRSWSVPVIRHLFAPLKEYFACV
(SEQ ID NO: 332);
 and









 (SEQ ID NO: 333).

### Example 26. Linker/NLS included

The gene expression regulatory domain of any one of Examples 23 to 25, wherein the gene expression regulatory domain further comprises at least one linker and/or at least one nuclear localization signal (NLS).

### Example 27. Transcriptional promoting protein defined

The gene expression regulatory domain of any one of Examples 23 to 26, wherein the domain protein is selected from VP64, Sun Tag, VPR (VP64, p65, Rta), and TV (TAL, VP64).

### Example 28. Transcriptional inhibitor protein defined

The gene expression regulatory domain of any one of Examples 23 to 27, wherein the domain protein is selected from KRAB, DNMT, MeCP2, HDAC, LSD, SRDX SALL1, and SDS3.

### Other components

### Example 29. NLS

A nuclear localization signal, represented by an amino acid sequence selected from the following:
PKKKRKV (SEQ ID NO: 200); KRPAATKKAGQAKKKK (SEQ ID NO: 201); PAAKRVKLD (SEQ ID NO: 202); RQRRNELKRSP (SEQ ID NO: 203); NQSSNFGPMKGGNFGGRSSGPYGGGGQYFAKPRNQGGY (SEQ ID NO: 204); RMRIZFKNKGKDTAELRRRRVEVSVELRKAKKDEQILKRRNV (SEQ ID NO: 205); VSRKRPRP (SEQ ID NO: 206); PPKKARED (SEQ ID NO: 207); PQPKKKPL (SEQ ID NO: 208); SALIKKKKKMAP (SEQ ID NO: 209); DRLRR (SEQ ID NO: 210); PKQKKRK (SEQ ID NO: 211); RKLKKKIKKL (SEQ ID NO: 212); REKKKFLKRR (SEQ ID NO: 213); KRKGDEVDGVDEVAKKKSKK (SEQ ID NO: 214); and RKCLQAGMNLEARKTKK (SEQ ID NO: 215).

### Example 30. Amino acid linker

An amino acid linker that does not affect functions of targets to be linked.

### Example 31. Amino acid linker

The amino acid linker of Example 30, wherein the amino acid linker is represented by an amino acid sequence selected from:
SGGSSGGSSG (SEQ ID NO: 260); SGGSSGGSSGSETP (SEQ ID NO: 261); SGGSSGGSSGSETPGT (SEQ ID NO: 262); SGGSSGGSSGSETPGTSESA (SEQ ID NO: 263); SGGSSGGSSGSETPGTSESATPES (SEQ ID NO: 264); SGGSSGGSSGSETPGTSESATPESSGGS (SEQ ID NO: 265); SGGSSGGSSGSETPGTSESATPESSGGSSGGS (SEQ ID NO: 266); SGGSSGGSSGSETPGTSESATPESSGGSSGGSSGSE (SEQ ID NO: 267); SGGSSGGSSGSETPGTSESATPESSGGSSGGSSGSETPGT (SEQ ID NO: 268); GGGGS (SEQ ID NO: 269); EAAAK (SEQ ID NO: 270); SGSETPGTSESATPES (SEQ ID NO: 271); SGGS (SEQ ID NO: 272); and SGGSKRTADGSEFE (SEQ ID NO: 273).

### Example 32. Uracil glycosylase inhibitor

A uracil glycosylase inhibitor that has at least one uracil glycosylase inhibitor domain,
wherein the uracil glycosylase inhibitor domain is each independently an amino acid sequence selected from the following:

TNLSDIIEKETGKQLVIQESILMLPEEVEEVIGNKPESDILVHTAYDESTDENVM
LLTSDAPEYKPWALVIQDSNGENKIKML (SEQ ID NO: 461).

### Example 33. Two or more UGIs, linker included

The uracil glycosylase inhibitor of Example 32, wherein the uracil glycosylase inhibitor comprises two or more uracil glycosylase inhibitor domains, and the uracil glycosylase inhibitor domains are linked via a linker.

### Guide RNA

### Example 34. Engineered tracrRNA, naturally occurring sequence

An engineered tracrRNA, represented by a nucleotide sequence selected from the following: 
CUUCACUGAUAAAGUGGAGAACCGCUUCACCAAAAGCUGUCCCU
UAGGGGAUUAGAACUUGAGUGAAGGUGGGCUGCUUGCAUCAGCCUAAU
GUCGAGAAGUGCUUUCUUCGGAAAGUAACCCUCGAAACAAAUUCAUUU
UUCCUCUCCAAUUCUGCACAA (SEQ ID NO: 41);
 and

CUUCACUGAUAAAGUGGAGAACCGCUUCACCAAAAGCUGUCCCU
UAGGGGAUUAGAACUUGAGUGAAGGUGGGCUGCUUGCAUCAGCCUAAU
GUCGAGAAGUGCUUUCUUCGGAAAGUAACCCUCGAAACAAAUUCAUUU
(SEQ ID NO: 42).

### Example 35. Engineered tracrRNA, WT-based

An engineered tracrRNA, represented by the following sequence:
5'-[first sequence]-[second sequence]-[second sequence]-[second sequence]-[second sequence]-3'
wherein the first sequence has a nucleotide sequence selected from CAAAUUCANNNCNCCUCUCCAAUUCUGCACAA (SEQ ID NO: 45), CAAAUUCANNNCNCCUCUCCAAUUCUGCACA (SEQ ID NO: 46), CAAAUUCANNNCNCCUCUCCAAUUCUGCAC (SEQ ID NO: 47), CAAAUUCANNNCNCCUCUCCAAUUCUGCA (SEQ ID NO: 48), CAAAUUCANNNCNCCUCUCCAAUUCUGC (SEQ ID NO: 49), CAAAUUCANNNCNCCUCUCCAAUUCUG (SEQ ID NO: 50), CAAAUUCANNNCNCCUCUCCAAUUCU (SEQ ID NO: 51), CAAAUUCANNNCNCCUCUCCAAUUC (SEQ ID NO: 52), CAAAUUCANNNCNCCUCUCCAAUU (SEQ ID NO: 53), CAAAUUCANNNCNCCUCUCCAAU (SEQ ID NO: 54), CAAAUUCANNNCNCCUCUCCAA (SEQ ID NO: 55), CAAAUUCANNNCNCCUCUCCA (SEQ ID NO: 56), CAAAUUCANNNCNCCUCUCC (SEQ ID NO: 57), CAAAUUCANNNCNCCUCUC (SEQ ID NO: 58), CAAAUUCANNNCNCCUCU (SEQ ID NO: 59), CAAAUUCANNNCNCCUC (SEQ ID NO: 60), CAAAUUCANNNCNCCU (SEQ ID NO: 61), CAAAUUCANNNCNCC (SEQ ID NO: 62), CAAAUUCANNNCNC (SEQ ID NO: 63), and CAAAUUCANNNCN (SEQ ID NO: 64),
the second sequence has a nucleotide sequence selected from AUGUCGAGAAGUGCUUUCUUCGGAAAGUAACCCUCGAAA (SEQ ID NO: 65),
the third sequence has a nucleotide sequence selected from GGCUGCUUGCAUCAGCCUA (SEQ ID NO: 66),
the fourth sequence has a nucleotide sequence selected from 
CCGCUUCACCAAAAGCUGUCCCUUAGGGGAUUAGAACUUGAGUGAAGG
UG (SEQ ID NO: 67),
 CCGCUUCACCAAAAGCUGUCCUUAGGGAUUAGAACUUGAGUGAAGGUG (SEQ ID NO: 68), CCGCUUCACCAAAAGCUGUCUUAGGAUUAGAACUUGAGUGAAGGUG (SEQ ID NO: 69), CCGCUUCACCAAAAGCUGUUUAGAUUAGAACUUGAGUGAAGGUG (SEQ ID NO: 70), CCGCUUCACCAAAAGCUGUUAGUUAGAACUUGAGUGAAGGUG (SEQ ID NO: 71), CCGCUUCACCAAAAGCUGUUAGUAGAACUUGAGUGAAGGUG (SEQ ID NO: 72), CCGCUUCACCAAAAGCUUUAGAGAACUUGAGUGAAGGUG (SEQ ID NO: 73), CCGCUUCACCAAAAGCUUAGGAACUUGAGUGAAGGUG (SEQ ID NO: 74), CCGCUUCACCAAAAGUUAGAACUUGAGUGAAGGUG (SEQ ID NO: 75), CCGCUUCACCAAAAUUAGACUUGAGUGAAGGUG (SEQ ID NO: 76), CCGCUUCACCAAAUUAGCUUGAGUGAAGGUG (SEQ ID NO: 77), CCGCUUCACCAAUUAGUUGAGUGAAGGUG (SEQ ID NO: 78), CCGCUUCACCAUUAGUGAGUGAAGGUG (SEQ ID NO: 79), CCGCUUCACCUUAGGAGUGAAGGUG (SEQ ID NO: 80), CCGCUUCACUUAGAGUGAAGGUG (SEQ ID NO: 81), CCGCUUCACUUAGGUGAAGGUG (SEQ ID NO: 82), CCGCUUCAUUAGUGAAGGUG (SEQ ID NO: 83), CCGCUUCUUAGGAAGGUG (SEQ ID NO: 84), CCGCUUUUAGAAGGUG (SEQ ID NO: 85), CCGCUUUAGAGGUG (SEQ ID NO: 86), CCGCUUAGGGUG (SEQ ID NO: 87), and CCGUUAGGUG (SEQ ID NO: 88),
the fifth sequence has a nucleotide sequence selected from CUUCACUGAUAAAGUGGAGAA (SEQ ID NO: 89), AAGUGGAGAA (SEQ ID NO: 90), AAAGUGGAGAA (SEQ ID NO: 91), UAAAGUGGAGAA (SEQ ID NO: 92), AUAAAGUGGAGAA (SEQ ID NO: 93), GAUAAAGUGGAGAA (SEQ ID NO: 94), UGAUAAAGUGGAGAA (SEQ ID NO: 95), CUGAUAAAGUGGAGAA (SEQ ID NO: 96), ACUGAUAAAGUGGAGAA (SEQ ID NO: 97), CACUGAUAAAGUGGAGAA (SEQ ID NO: 98), UCACUGAUAAAGUGGAGAA (SEQ ID NO: 99), and UUCACUGAUAAAGUGGAGAA (SEQ ID NO: 100), and
the engineered tracrRNA is different from the nucleotide sequence of any one of SEQ ID NO: 41 to 42.

### Example 36. Linker sequence replacement

The tracrRNA scaffold sequence of Example 35, wherein 5'-UUAG-3' included in the fourth sequence is replaced with 5'-GAAA-3'.

### Example 37. Engineered tracrRNA, MF-based

An engineered tracrRNA scaffold, represented by the following sequence:
5'-[first region]-[second region]-[third region]-[fourth region]-3',
wherein the first region has a nucleotide sequence selected from AAGUGGAGAA (SEQ ID NO: 101), AAAGUGGAGAA (SEQ ID NO: 102), UAAAGUGGAGAA (SEQ ID NO: 103), AUAAAGUGGAGAA (SEQ ID NO: 104), GAUAAAGUGGAGAA (SEQ ID NO: 105), UGAUAAAGUGGAGAA (SEQ ID NO: 106), CUGAUAAAGUGGAGAA (SEQ ID NO: 107), ACUGAUAAAGUGGAGAA (SEQ ID NO: 108), CACUGAUAAAGUGGAGAA (SEQ ID NO: 109), UCACUGAUAAAGUGGAGAA (SEQ ID NO: 110), UUCACUGAUAAAGUGGAGAA (SEQ ID NO: 111), and CUUCACUGAUAAAGUGGAGAA (SEQ ID NO: 112),
the second region has a nucleotide sequence selected from CCUUAGGUGG (SEQ ID NO: 113), CCGUUAGGUGG (SEQ ID NO: 114), CCGCUUAGGGUGG (SEQ ID NO: 115), CCGCUUUAGAGGUGG (SEQ ID NO: 116), CCGCUUUUAGAAGGUGG (SEQ ID NO: 117), CCGCUUCUUAGGAAGGUGG (SEQ ID NO: 118), CCGCUUCAUUAGUGAAGGUGG (SEQ ID NO: 119), CCGCUUCACUUAGGUGAAGGUGG (SEQ ID NO: 120), CCGCUUCACUUAGAGUGAAGGUGG (SEQ ID NO: 121), CCGCUUCACCUUAGGAGUGAAGGUGG (SEQ ID NO: 122), CCGCUUCACCAUUAGUGAGUGAAGGUGG (SEQ ID NO: 123), CCGCUUCACCAAUUAGUUGAGUGAAGGUGG (SEQ ID NO: 124), CCGCUUCACCAAAUUAGCUUGAGUGAAGGUGG (SEQ ID NO: 125), CCGCUUCACCAAAAUUAGACUUGAGUGAAGGUGG (SEQ ID NO: 126), CCGCUUCACCAAAAGUUAGAACUUGAGUGAAGGUGG (SEQ ID NO: 127), CCGCUUCACCAAAAGCUUAGGAACUUGAGUGAAGGUGG (SEQ ID NO: 128), CCGCUUCACCAAAAGCUUUAGAGAACUUGAGUGAAGGUGG (SEQ ID NO: 129), CCGCUUCACCAAAAGCUGUUAGUAGAACUUGAGUGAAGGUGG (SEQ ID NO: 130), CCGCUUCACCAAAAGCUGUUAGUUAGAACUUGAGUGAAGGUGG (SEQ ID NO: 131), CCGCUUCACCAAAAGCUGUUUAGAUUAGAACUUGAGUGAAGGUGG (SEQ ID NO: 132), CCGCUUCACCAAAAGCUGUCUUAGGAUUAGAACUUGAGUGAAGGUGG (SEQ ID NO: 133), and 
CCGCUUCACCAAAAGCUGUCCCUUAGGGGAUUAGAACUUGAGUGAAGG
UGG (SEQ ID NO: 134),
the third region has a nucleotide sequence selected from CCUUAGGUGG (SEQ ID NO: 113), CCGUUAGGUGG (SEQ ID NO: 114), CCGCUUAGGGUGG (SEQ ID NO: 115), CCGCUUUAGAGGUGG (SEQ ID NO: 116), CCGCUUUUAGAAGGUGG (SEQ ID NO: 117), CCGCUUCUUAGGAAGGUGG (SEQ ID NO: 118), CCGCUUCAUUAGUGAAGGUGG (SEQ ID NO: 119), CCGCUUCACUUAGGUGAAGGUGG (SEQ ID NO: 120), CCGCUUCACUUAGAGUGAAGGUGG (SEQ ID NO: 121), CCGCUUCACCUUAGGAGUGAAGGUGG (SEQ ID NO: 122), CCGCUUCACCAUUAGUGAGUGAAGGUGG (SEQ ID NO: 123), CCGCUUCACCAAUUAGUUGAGUGAAGGUGG (SEQ ID NO: 124), CCGCUUCACCAAAUUAGCUUGAGUGAAGGUGG (SEQ ID NO: 125), CCGCUUCACCAAAAUUAGACUUGAGUGAAGGUGG (SEQ ID NO: 126), CCGCUUCACCAAAAGUUAGAACUUGAGUGAAGGUGG (SEQ ID NO: 127), CCGCUUCACCAAAAGCUUAGGAACUUGAGUGAAGGUGG (SEQ ID NO: 128), CCGCUUCACCAAAAGCUUUAGAGAACUUGAGUGAAGGUGG (SEQ ID NO: 129), CCGCUUCACCAAAAGCUGUUAGUAGAACUUGAGUGAAGGUGG (SEQ ID NO: 130), CCGCUUCACCAAAAGCUGUUAGUUAGAACUUGAGUGAAGGUGG (SEQ ID NO: 131), CCGCUUCACCAAAAGCUGUUUAGAUUAGAACUUGAGUGAAGGUGG (SEQ ID NO: 132), CCGCUUCACCAAAAGCUGUCUUAGGAUUAGAACUUGAGUGAAGGUGG (SEQ ID NO: 133), and 
CCGCUUCACCAAAAGCUGUCCCUUAGGGGAUUAGAACUUGAGUGAAGG
UGG (SEQ ID NO: 134),
the fourth region has a nucleotide sequence selected from is CCUUAGGUGG (SEQ ID NO: 113), CCGUUAGGUGG (SEQ ID NO: 114), CCGCUUAGGGUGG (SEQ ID NO: 115), CCGCUUUAGAGGUGG (SEQ ID NO: 116), CCGCUUUUAGAAGGUGG (SEQ ID NO: 117), CCGCUUCUUAGGAAGGUGG (SEQ ID NO: 118), CCGCUUCAUUAGUGAAGGUGG (SEQ ID NO: 119), CCGCUUCACUUAGGUGAAGGUGG (SEQ ID NO: 120), CCGCUUCACUUAGAGUGAAGGUGG (SEQ ID NO: 121), CCGCUUCACCUUAGGAGUGAAGGUGG (SEQ ID NO: 122), CCGCUUCACCAUUAGUGAGUGAAGGUGG (SEQ ID NO: 123), CCGCUUCACCAAUUAGUUGAGUGAAGGUGG (SEQ ID NO: 124), CCGCUUCACCAAAUUAGCUUGAGUGAAGGUGG (SEQ ID NO: 125), CCGCUUCACCAAAAUUAGACUUGAGUGAAGGUGG (SEQ ID NO: 126), CCGCUUCACCAAAAGUUAGAACUUGAGUGAAGGUGG (SEQ ID NO: 127), CCGCUUCACCAAAAGCUUAGGAACUUGAGUGAAGGUGG (SEQ ID NO: 128), CCGCUUCACCAAAAGCUUUAGAGAACUUGAGUGAAGGUGG (SEQ ID NO: 129), CCGCUUCACCAAAAGCUGUUAGUAGAACUUGAGUGAAGGUGG (SEQ ID NO: 130), CCGCUUCACCAAAAGCUGUUAGUUAGAACUUGAGUGAAGGUGG (SEQ ID NO: 131), CCGCUUCACCAAAAGCUGUUUAGAUUAGAACUUGAGUGAAGGUGG (SEQ ID NO: 132), CCGCUUCACCAAAAGCUGUCUUAGGAUUAGAACUUGAGUGAAGGUGG (SEQ ID NO: 133), and 
CCGCUUCACCAAAAGCUGUCCCUUAGGGGAUUAGAACUUGAGUGAAGG
UGG (SEQ ID NO: 134),
 and
the engineered tracrRNA is different from the nucleotide sequence of any one of SEQ ID NO: 41 to 42.

### Example 38. Engineered tracrRNA, similar sequences encompassed

A Cas12a protein, having a sequence that is identical with, the same as, matched with, and/or equivalent to the tracrRNA sequence of any one of Examples 34 to 37 by about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more,
wherein the term "about" has the same meaning as defined in the subsection "about" of the section "Definition of terms."

### Example 39. Engineered crRNA direct repeat

An engineered crRNA direct repeat, represented by a nucleotide sequence selected from the following:
GUUGCAGAACCCGAAUAGACGAAUGAAGGAAUGCAAC (SEQ ID NO: 43); and GAAUGAAGGAAUGCAAC (SEQ ID NO: 44).

### Example 40. Engineered crRNA direct repeat, WT-based

An engineered crRNA direct repeat, represented by the following sequence:
5'-[sixth sequence]-[seventh sequence]-3'
wherein the sixth sequence has a nucleotide sequence selected from GUUGCAGAACCCGAAUAGNBNNNUGAAGGA (SEQ ID NO: 158), GUUGCAGAACCCGAAUAGNGNNNUGAAGGA (SEQ ID NO: 159), UUGCAGAACCCGAAUAGNGNNNUGAAGGA (SEQ ID NO: 160), UGCAGAACCCGAAUAGNGNNNUGAAGGA (SEQ ID NO: 161), GCAGAACCCGAAUAGNGNNNUGAAGGA (SEQ ID NO: 162), CAGAACCCGAAUAGNGNNNUGAAGGA (SEQ ID NO: 163), AGAACCCGAAUAGNGNNNUGAAGGA (SEQ ID NO: 164), GAACCCGAAUAGNGNNNUGAAGGA (SEQ ID NO: 165), AACCCGAAUAGNGNNNUGAAGGA (SEQ ID NO: 166), ACCCGAAUAGNGNNNUGAAGGA (SEQ ID NO: 167), CCCGAAUAGNGNNNUGAAGGA (SEQ ID NO: 168), CCGAAUAGNGNNNUGAAGGA (SEQ ID NO: 169), CGAAUAGNGNNNUGAAGGA (SEQ ID NO: 170), GAAUAGNGNNNUGAAGGA (SEQ ID NO: 171), AAUAGNGNNNUGAAGGA (SEQ ID NO: 172), AUAGNGNNNUGAAGGA (SEQ ID NO: 173), UAGNGNNNUGAAGGA (SEQ ID NO: 174), AGNGNNNUGAAGGA (SEQ ID NO: 175), and NGNNNUGAAGGA (SEQ ID NO: 176), and
the seventh sequence is 5'-AUGCAAC-3',
wherein each N is independently A, C, G, or U.

### Example 41. Engineered crRNA direct repeat, MF-based

An engineered crRNA direct repeat, represented by the following sequence:
5'-[fifth region]-[sixth region]-3'
wherein the fifth region has a nucleotide sequence selected from GAAUGAAGGA (SEQ ID NO: 177), CGAAUGAAGGA (SEQ ID NO: 178), ACGAAUGAAGGA (SEQ ID NO: 179), GACGAAUGAAGGA (SEQ ID NO: 180), AGACGAAUGAAGGA (SEQ ID NO: 181), UAGACGAAUGAAGGA (SEQ ID NO: 182), AUAGACGAAUGAAGGA (SEQ ID NO: 183), AAUAGACGAAUGAAGGA (SEQ ID NO: 184), GAAUAGACGAAUGAAGGA (SEQ ID NO: 185), CGAAUAGACGAAUGAAGGA (SEQ ID NO: 186), CCGAAUAGACGAAUGAAGGA (SEQ ID NO: 187), CCCGAAUAGACGAAUGAAGGA (SEQ ID NO: 188), ACCCGAAUAGACGAAUGAAGGA (SEQ ID NO: 189), AACCCGAAUAGACGAAUGAAGGA (SEQ ID NO: 190), GAACCCGAAUAGACGAAUGAAGGA (SEQ ID NO: 191), AGAACCCGAAUAGACGAAUGAAGGA (SEQ ID NO: 192), CAGAACCCGAAUAGACGAAUGAAGGA (SEQ ID NO: 193), GCAGAACCCGAAUAGACGAAUGAAGGA (SEQ ID NO: 194), UGCAGAACCCGAAUAGACGAAUGAAGGA (SEQ ID NO: 195), and UUGCAGAACCCGAAUAGACGAAUGAAGGA (SEQ ID NO: 196),
the sixth region is 5'-AUGCAAC-3', and
the sequence is different from the nucleotide sequence of any one of SEQ ID NO: 43 to 44.

### Example 42. Similar sequences encompassed

A Cas12a protein, having a sequence that is identical with, the same as, matched with, and/or equivalent to the crRNA direct repeat sequence of any one of Examples 39 to 41 by about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more,
wherein the term "about" has the same meaning as defined in the subsection "about" of the section "Definition of terms."

### Example 43. U-rich tail

A U-rich tail, represented by a sequence selected from the following:

5'-(UₐN)_{d}Uₑ-3';

5'-UaVUaVUe-3';

and

5'-UaVUaVUaVUe-3',

wherein N is A, C, G or U, and each V is independently A, C, or G, and
a is an integer between 0 to 4 inclusive, d is an integer between 0 to 3 inclusive, and e is an integer between 1 to 10 inclusive.

### Example 44. Exemplary U-rich tail sequence

The U-rich tail of Example 43, wherein the U-rich tail has a nucleotide sequence selected from the following:
UUURUUU (SEQ ID NO: 216); UUURUUURUUU (SEQ ID NO: 217); UUUURU (SEQ ID NO: 218); UUUURUU (SEQ ID NO: 219); UUUURUUU (SEQ ID NO: 220); UUUURUUUU (SEQ ID NO: 221); UUUURUUUUU (SEQ ID NO: 222); UUUURUUUUUU (SEQ ID NO: 223); UUUAUUU (SEQ ID NO: 224); UUUAUUUAUUU (SEQ ID NO: 225); UUUUAU (SEQ ID NO: 226); UUUUAUU (SEQ ID NO: 227); UUUUAUUU (SEQ ID NO: 228); UUUUAUUUU (SEQ ID NO: 229); UUUUAUUUUU (SEQ ID NO: 230); UUUUAUUUUUU (SEQ ID NO: 231); UUUGUUU (SEQ ID NO: 232); UUUGUUUGUUU (SEQ ID NO: 233); UUUUGU (SEQ ID NO: 234); UUUUGUU (SEQ ID NO: 235); UUUUGUUU (SEQ ID NO: 236); UUUUGUUUU (SEQ ID NO: 237); UUUUGUUUUU (SEQ ID NO: 238); UUUUGUUUUUU (SEQ ID NO: 239); and UUUUUU (SEQ ID NO: 240).

### Example 45. Guide RNA

A guide RNA, comprising:
a scaffold comprising an engineered tracrRNA and an engineered crRNA direct repeat;
a spacer (guide domain); and
optionally, a U-rich tail,
wherein the scaffold may interact with the dead Cas12f1 protein of any one of the Examples, the dCas12f1-base editing fusion protein of any one of the Examples, and/or the dCas12f1-expression regulating fusion protein of any one of the Examples, thereby forming a complex,
the spacer is designed to bind complementarily to a predetermined target sequence,
the U-rich tail is a sequence that is rich in uridine (U), and
the scaffold, the spacer, and the U-rich tail are sequentially linked to each other in a 5' to 3' direction.

### Example 46. Scaffold, two molecules of nucleic acid

The guide RNA of Example 45,
wherein the engineered tracrRNA of the scaffold and the engineered crRNA direct repeat of the scaffold are distinct nucleic acid molecules,
a part of the engineered tracrRNA and the engineered crRNA direct repeat form complementary bonds with each other, and
the 3' end portion of the engineered crRNA direct repeat is linked to the 3' end portion of the spacer.

### Example 47. Scaffold, one molecule of nucleic acid

The guide RNA of Example 45,
wherein the 3' end of the engineered tracrRNA of the scaffold and the 3' end of the engineered crRNA direct repeat of the scaffold are linked via a linker, and
the 3' end portion of the engineered crRNA direct repeat is linked to the 3' end portion of the spacer.

### Example 48. Linker defined

### The guide RNA of Example 47, wherein the linker is 5'-GAAA-3'.

### Example 49. Engineered tracrRNA defined

The guide RNA of any one of Examples 45 to 48,
wherein the engineered tracrRNA is the tracrRNA of any one of Examples 34 to 38.

### Example 50. Engineered crRNA direct repeat defined

The guide RNA of any one of Examples 45 to 49,
wherein the engineered crRNA direct repeat is the crRNA direct repeat of any one of Examples 39 to 42.

### Example 51. U-rich tail defined

The guide RNA of any one of Examples 45 to 50,
wherein the U-rich tail is the U-rich tail of any one of Examples 43 to 44.

### Example 52. Similar sequences encompassed

A Cas12a protein, having a sequence that is identical with, the same as, matched with, and/or equivalent to the guide RNA sequence of any one of Examples 45 to 51 by about 70% or more, about 71% or more, about 72% or more, about 73% or more, about 74% or more, about 75% or more, about 76% or more, about 77% or more, about 78% or more, about 79% or more, about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more,
wherein the term "about" has the same meaning as defined in the subsection "about" of the section "Definition of terms."

### Example 53. DNA encoding guide RNA

A DNA that encodes the guide RNA of any one of Examples 45 to 52.
dCas12f1-base editing fusion protein

### Example 54. dCas12f1-base editing fusion protein

A dCas12f1-base editing fusion protein, comprising:
the dead Cas12f1 protein of any one of Examples 1 to 12; and
at least one base editing domain that is any one of Examples 13 to 22.

### Example 55. Adenine base editor (ABE)

The dCas12f1-base editing fusion protein of Example, wherein the base editing domain is the adenosine deaminase of any one of Examples 14 to 17.

### Example 56, ABE, structure defined

The dCas12f1-base editing fusion protein of Example 55, wherein the dCas12f1-base editing fusion protein has a structure selected from the following:

NHz-[dCas12f1]-[Linker]-[BE]-COOH;

and

NH₂-[BE]-[Linker]-[dCas12f1]-COOH,

[575] wherein the dCas12f1 is the dead Cas12f1 protein, the BE is the adenosine deaminase, and the Linker is a linker or absent.

### Example 57, ABE, sequence defined

The dCas12f1-base editing fusion protein of Example 56, wherein the dCas12f1-base editing fusion protein has an amino acid sequence selected from SEQ ID NOS: 284 to 324, and SEQ ID NOS: 418 to 442.

### Example 58. Cytidine base editor (CBE)

The dCas12f1-base editing fusion protein of Example 54, wherein the base editing domain is the cytidine deaminase of any one of Examples 18 to 22.

### Example 59. CBE, structure defined

The dCas12f1-base editing fusion protein of Example 58, wherein the dCas12f1 base editing fusion protein comprises the uracil glycosylase inhibitor of any one of Examples 32 to 33, and
the dCas12f1-base editing fusion protein has a structure selected from the following:

   NH₂-[UGI]-[Linker₂]-[dCas12f1]-[Linker₁]-[BE]-COOH;

   and

   NH₂-[BE]-[Linker₁]-[dCas12f1]-[Linker₂]-[UGI]-COOH,
wherein the dCas12f1 is the dead Cas12f1 protein, the BE is the adenosine deaminase, and the Linker₁ is a first linker or absent, and the Linker₂ is a second linker or absent.

### Example 60, CBE, sequence defined

The dCas12f1-base editing fusion protein of Example 59, wherein the dCas12f1-base editing fusion protein has an amino acid sequence selected from SEQ ID NOS: 325 to 328.

### Example 61. NLS additionally included

The dCas12f1-base editing fusion protein of any one of Examples 54 to 60,
wherein the dCas12f1-base editing fusion protein further comprises at least one NLS of Example 29,
the NLS is located at the N-terminus and/or C-terminus of the dCas12f1-base editing fusion protein, and
the dCas12f1-base editing fusion protein and the NLS are linked via a linker, or are directly linked to each other.

### dCas12f1-expression regulating fusion protein

### Example 62. dCas12f1-expression regulatory domain fusion protein

A dCas12f1-expression regulating fusion protein comprising:
the dead Cas12f1 protein of any one of Examples 1 to 12; and
at least one gene expression regulatory domain that is any one of Examples 23 to 28.

### Example 63. N terminal module

The dCas12f1-expression regulating fusion protein of Example 62, wherein the at least one gene expression regulatory domain is linked to the N terminus of the dead Cas12f1 protein.

### Example 64. N terminal, additional components

The dCas12f1-expression regulating fusion protein of Example 63,
wherein the dCas12f1-expression regulating fusion protein comprises at least one additional domain,
each additional component is independently selected from linker, NLS, and tag, and
the additional component is linked to at least one position selected from the following:
the N terminus of the dCas12f1-expression regulating fusion protein;
the C terminus of the dCas12f1-expression regulating fusion protein;
between the dead Cas12f1 protein and the gene expression regulatory domain; and
between the gene expression regulatory domain and another gene expression regulatory domain.

### Example 65. Module structure shown in Experimental Example

The dCas12f1-expression regulating fusion protein of Example 63, wherein the dCas12f1-expression regulating fusion protein is represented by a structure selected from the following:

NH₂-[NLS₁]-[Linker₁]-[Regulator₁]-[Linker₂]-[NLS₂]-[dCas12f1]-[NLS₃]-COOH;

NH₂-[NLS₁]-[Linker₁]-[FLAG]-[Linker₂]-[Regulator₁]-[Linker₃]-[NLS₂]-[Linker₄]-[dCas12f1]-[NLS₃]-COOH; and

NH₂-[NLS₁]-[Linker₁]-[Regulator₁]-[Linker₂]-[NLS₂]-[Regulator₂]-[NLS₃]-[Linker₃]-[dCas12f1]-[NLS₄]-COOH,

wherein the Linker₁, Linker₂, Linker₃, and Linker₄ are each independently the linker of any one of Examples 30 to 31 or absent,
the FLAG is DYKDDDDK (SEQ ID NO: 510) or absent,
the Regulator₁ and Regulator₂ are each independently the gene expression regulatory domain of any one of Examples 23 to 28,
the dCas12f1 is the dead Cas12f1 of any one of Examples 1 to 12, and
the NLS₁, NLS₂, NLS₃, and NLS₄ are each independently the NLS of Example 29 or absent.

### Example 66. Defined by Experimental Example

The dCas12f1-expression regulating fusion protein of Example 65, wherein the dCas12f1-expression regulating fusion protein is selected from the following:
the structure represented by NH₂-[NLS₁]-[Linker₁]-[Regulator₁]-[Linker₂]-[NLS_{2]}-[dCas12f1]-[NLS₃]-COOH,
wherein the Regulaton is KRAB, and the dCas12f1 is represented by an amino acid sequence selected from SEQ ID NOS: 17 to 18;
the structure represented by NH₂-[NLS₁]-[Linker₁]-[FLAG]-[Linker₂]-[Regulator₁]-[Linker₃]-[NLS₂]-[Linker₄]-[dCas12f1]-[NLS₃]-COOH,
wherein the Regulaton is DNMT3A, and the dCas12f1 is represented by an amino acid sequence selected from SEQ ID NOS: 17 to 18; and
the structure represented by NH₂-[NLS₁]-[Linker₁]-[Regulator₁]-[Linker₂]-[NLS₂]-[Regulator₂]-[NLS₃]-[Linker₃]-[dCas12f1]-[NLS₄]-COOH,
wherein the Regulaton is KRAB, the Regulator₂ is MeCP2, and the dCas12f1 is represented by an amino acid sequence selected from SEQ ID NOS: 17 to 18.

### Example 67. C terminal module

The dCas 12f1-expression regulating fusion protein of any one of Examples 62 to 66, wherein the at least one gene expression regulatory domain is linked to the C terminus of the dead Cas12f1 protein.

### Example 68. C terminal, additional components

The dCas12f1-expression regulating fusion protein of Example 67,
wherein the dCas12f1-expression regulating fusion protein comprises at least one additional domain,
each additional component is independently selected from linker, NLS, and tag,
the additional component is linked to at least one position selected from the following:
the N terminus of the dCas12f1-expression regulating fusion protein;
the C terminus of the dCas12f1-expression regulating fusion protein;
between the dead Cas12f1 protein and the gene expression regulatory domain; and
between the gene expression regulatory domain and another gene expression regulatory domain.

### Example 69. Module structure shown in Experimental Example

The dCas12f1-expression regulating fusion protein of Example 67, wherein the dCas12f1-expression regulating fusion protein is represented by a structure selected from the following:

NH₂-[NLS₁]-[Linker₁]-[dCas12f1]-[NLS₂]-[Linker₂]-[Regulator₁]-COOH;

NH₂-[NLS₁]-[Linker₁]-[dCas12f1]-[NLS₂]-[Linker₂]-[Regulator₁]-[Linker₃]-[NLS₃]-[Regulator₂]-COOH;

NH₂-[NLS₁]-[Linker₁]-[dCas12f1]-[NLS₂]-[Linker₂]-[Regulator₁]-[Linker₃]-[Regulator₂]-COOH; and

NH₂-[NLS₁]-[Linker₁]-[dCas12f1]-[NLS₂]-[Linker₂]-[Regulator₁]-[Linker₃]-[Regulator₂]-[Linker₄]-[NLS₃]-[Regulator₃]-COOH,

wherein the Linker₁, Linker₂, Linker₃, and Linker₄ are each independently the linker of any one of Examples 30 to 31 or absent,
the Regulators, Regulator₂, and Regulator₃ are each independently the gene expression regulatory domain of any one of Examples 23 to 28,
the dCas12f1 is the dead Cas12f1 of any one of Examples 1 to 12, and
the NLS₁, NLS₂, and NLS₃ are each independently the NLS of Example 29 or absent.

### Example 70. Defined by Experimental Example

The dCas12f1-expression regulating fusion protein of Example 69, wherein the dCas12f1-expression regulating fusion protein is selected from the following:
the structure represented by NH₂-[NLS₁]-[Linker₁]-[dCas12f1]-[NLS₂]-[Linker₂]-[Regulator₁]-COOH,
wherein the Regulator₁ is KRAB, and the dCas12f1 is represented by an amino acid sequence selected from SEQ ID NOS: 17 to 18;
the structure represented by NH₂-[NLS₁]-[Linker₁]-[dCas12f1]-[NLS₂]-[Linker₂]-[Regulator₁]-COOH,
wherein the Regulaton is hHDAC3, and the dCas12f1 is represented by an amino acid sequence selected from SEQ ID NOS: 17 to 18;
the structure represented by NH₂-[NLS₁]-[Linker₁]-[dCas12f1]-[NLS₂]-[Linker₂]-[Regulator₁]-[Linker₃]-[NLS₃]-[Regulator₂]-COOH,
wherein the Regulaton is KRAB, the Regulator₂ is MeCP2, and the dCas12f1 is represented by an amino acid sequence selected from SEQ ID NOS: 17 to 18;
the structure represented by NH₂-[NLS₁]-[Linker₁]-[dCas12f1]-[NLS₂]-[Linker₂]- [Regulator₁] - [Linker₃] - [Regulator₂] -COOH,
wherein the Regulator₁ and Regulator₂ are KRAB, and the dCas12f1 is represented by an amino acid sequence selected from SEQ ID NOS: 17 to 18; and
the structure represented by NH₂-[NLS₁]-[Linker₁]-[dCas12f1]-[NLS₂]-[Linker₂]- [Regulator₁] - [Linkers]-[Regulatorz]-[Linker4]-[NLSs]-[Regulators]-COOH,
wherein the Regulator₁ and Regulator₂ are KRAB, the Regulator₃ is MeCP2, and the dCas12f1 is represented by an amino acid sequence selected from SEQ ID NOS: 17 to 18.

### Example 71. N/C terminal module

The dCas12f1-expression regulating fusion protein of any one of Examples 62 to 70,
wherein the dCas12f1-expression regulating fusion protein comprises a first gene expression regulatory domain and a second gene expression regulatory domain,
the first gene expression regulatory domain is linked to the N terminus of the dead Cas12f1 protein, and
the second gene expression regulatory domain is linked to the C terminus of the dead Cas12f1 protein.

### Example 72. N/C terminal, additional components

The dCas12f1-expression regulating fusion protein of Example 71,
wherein the dCas12f1-expression regulating fusion protein comprises at least one additional domain,
each additional component is independently selected from linker, NLS, and tag, and
the additional component is linked to at least one position selected from the following:
   the N terminus of the dCas12f1-expression regulating fusion protein;
   the C terminus of the dCas12f1-expression regulating fusion protein;
   between the dead Cas12f1 protein and the gene expression regulatory domain; and
   between the gene expression regulatory domain and another gene expression regulatory domain.

### Example 73. Module structure shown in Experimental Example

The dCas12f1-expression regulating fusion protein of Example 71, wherein the dCas12f1-expression regulating fusion protein is represented by a structure selected from the following:

NH₂-[NLS₁]-[Linker₁]-[FLAG]-[Linker₂]-[Regulator₁]-[Linker₃]-[NLS₂]-[Linker₄]-[dCas12f1]-[NLS₃]-[Regulator₃]-COOH;

NH₂-[NLS₁]-[Linker₁]-[Regulator₁]-[Linker₂]-[NLS₂]-[Regulator₂]-[NLS₃]-[Linker₃]-[dCas12f1]-[NLS₄]-[Linker₄]-[Regulator₃]-COOH;

and

NH₂-[NLS₁]-[Regulator₁]-[NLS₂]-[Linker₁]-[dCas12f1]-[NLS₃]-[Linker₂]-[Regulators]-COOH,

wherein the Linker₁, Linker₂, Linker₃, and Linker₄ are each independently the linker of any one of Examples 30 to 31 or absent,
the FLAG is "FLAG" or absent,
the Regulators, Regulator₂, and Regulator₃ are each independently the gene expression regulatory domain of any one of Examples 23 to 28,
the dCas12f1 is the dead Cas12f1 of any one of Examples 1 to 12, and
the NLS₁, NLS₂, NLS₃, and NLS₄ are each independently the NLS of Example 29 or absent.

### Example 74. Defined by Experimental Example

The dCas12f1-expression regulating fusion protein of Example 73, wherein the dCas12f1-expression regulating fusion protein is selected from the following:
the structure represented by NH₂-[NLS₁]-[Linker₁]-[FLAG]-[Linker₂]-[Regulator₁]-[Linker₃]-[NLS₂]-[Linker₄]-[dCas12f1]-[NLS₃]-[Regulator₃]-COOH,
wherein the Regulator₁ is KRAB, the Regulator₂ is MeCP2, and the dCas12f1 is represented by an amino acid sequence selected from SEQ ID NOS: 17 to 18;
the structure represented by NH₂-[NLS₁]-[Linker₁]-[Regulator₁]-[Linker₂]-[NLS₂]-[Regulator₂]-[NLS₃]-[Linker₃]-[dCas12f1]-[NLS₄]-[Linker₄]-[Regulator₃]-COOH,
wherein the Regulator₁ and the Regulator₃ are KRAB, the Regulator₂ is MeCP2, and the dCas 12f1 is represented by an amino acid sequence selected from SEQ ID NOS: 17 to 18; and
the structure represented by NH₂-[NLS₁]-[Regulator₁]-[NLS₂]-[Linker₁]-[dCas12f1]-[NLS₃]-[Linker₂]-[Regulator₃]-COOH,
wherein the Regulator₁ is MeCP2, the Regulator₂ is KRAB, and the dCas12f1 is represented by an amino acid sequence selected from SEQ ID NOS: 17 to 18.

### Example 7J. Transcriptional promoting protein

The dCas12f1-expression regulating fusion protein of any one of Examples 62 to 74, wherein the expression regulatory domain is the gene expression regulatory domain of Example 27.

### Example 76. Transcriptional inhibitor protein

The dCas 12f1-expression regulating fusion protein of any one of Examples 62 to 75, wherein the expression regulatory domain is the gene expression regulatory domain of Example 28.

### Example 77. Structure shown in drawings

A dCas12f1-expression regulating fusion protein that has a structure shown in any one of FIGS. 39 to 49,
wherein dTnpB in the drawing is the dead Cas12f1 of any one of Examples 1 to 12.

### Engineered Cas12f1-guide RNA complex

### Example 78. Engineered Cas12f1-guide RNA complex

An engineered Cas12f1-guide RNA complex, comprising:
an engineered Cas12f1 protein selected from the dead Cas12f1 protein of any one of Examples 1 to 12, the dCas12f1-base editing protein of any one of Examples 54 to 61, and the dCas12f1-expression regulating protein of any one of Examples 62 to 77; and
the guide RNA of any one of Examples 45 to 52,
wherein the scaffold of the guide RNA is capable of interacting with the engineered Cas12f1 protein to form a complex, and
the spacer of the guide RNA is designed to target a predetermined target sequence.

### Example 79. Dead Cas12f1 complex

The engineered Cas12f1-guide RNA complex of Example 78,
wherein the engineered Cas12f1 protein is the dead Cas12f1 protein of any one of Examples 1 to 12.

### Example 80. Base editing complex

The engineered Cas12f1-guide RNA complex of Example 78,
wherein the engineered Cas12f1 protein is the dCas12f1-base editing protein of any one of Examples 54 to 61, and
the engineered Cas12f1 protein-guide RNA complex may be referred to as a base editing complex.

### Example 81. Expression regulating complex

The engineered Cas12f1-guide RNA complex of Example 78,
wherein the engineered Cas12f1 protein is the dCas12f1-expression regulating protein of any one of Examples 62 to 77, and
the engineered Cas12f1 protein-guide RNA complex may be referred to as an expression regulating complex.

### Vector encoding engineered Cas12f1 and guide RNA

### Example 82. Vector that encodes engineered Cas12f1 and guide RNA, one type of guide

A vector that encodes an engineered Cas12f1 and a guide RNA, comprising:
a nucleic acid encoding an engineered Cas12f1 protein that is selected from the dead Cas12f1 protein of any one of Examples 1 to 12, the dCas12f1-base editing protein of any one of Examples 54 to 61, and the dCas12f1-expression regulating protein of any one of Examples 62 to 77; and
a nucleic acid encoding the guide RNA of any one of Examples 45 to 52,
wherein the scaffold of the guide RNA is capable of interacting with the engineered Cas12f1 protein to form a complex, and
the spacer of the guide RNA is designed to target a predetermined target sequence.

### Example 83. Vector that encodes engineered Cas12f1 and guide RNA, two types of guides

A vector that encodes an engineered Cas12f1 and a guide RNA, comprising:
a nucleic acid encoding an engineered Cas12f1 protein that is selected from the dead Cas12f1 protein of any one of Examples 1 to 12, the dCas12f1-base editing protein of any one of Examples 54 to 61, and the dCas12f1-expression regulating protein of any one of Examples 62 to 77;
a nucleic acid encoding a first guide RNA selected from any one of Examples 45 to 52, and
a nucleic acid encoding a second guide RNA selected from any one of Examples 45 to 52,
wherein the scaffold of each of the first guide RNA and the second guide RNA is capable of interacting with the engineered Cas12f1 protein to form a complex, and
the spacer of each of the first guide RNA and the second guide RNA is designed to target a predetermined target sequence.

### Example 84. Vector number

The vector of any one of Examples 82 to 83, wherein both the nucleic acid encoding the engineered Cas12f1 protein and the nucleic acid encoding the guide RNA are loaded onto one vector (one unit).

### Example 85. Vector type

The vector of Example, wherein the vector is a viral vector or a non-viral vector.

### Example 86. Viral vector type

The vector of Example 85, wherein the vector is a viral vector selected from retrovirus, lentivirus, adenovirus, adeno-associated virus, vaccinia virus, poxvirus, and herpes simplex virus.

### Example 87. Non-viral vector type

The vector of Example 85, wherein the vector is a non-viral vector selected from plasmid, phage, naked DNA, DNA complex, PCR amplicon, and mRNA,
in which, optionally, the plasmid is selected from pcDNA series, pS456, pG1806, pACYC177, ColE1, pKT230, pME290, pBR322, pUC8/9, pUC6, pBD9, pHC79, pIJ61, pLAFR1, pHV14, pGEX series, pET series, and pUC19, and
optionally, the phage is selected from λgt4λB, λ-Charon, λΔz1, and M13.

### Engineered CRISPR/Cas12f1 composition

### Example 88. Engineered CRISPR/Cas12f1 Composition

An engineered CRISPR/Cas12f1 composition, comprising:
an engineered Cas12f1 protein, which is selected from the dead Cas12f1 protein of any one of Examples 1 to 12, the dCas12f1-base editing protein of any one of Examples 54 to 61, and the dCas12f1-expression regulating protein of any one of Examples 62 to 77, or a nucleic acid encoding the engineered Cas12f1 protein; and
at least one (one unit, one kind, or one type of) guide RNA that is any one of Examples 45 to 52, or a nucleic acid encoding the guide RNA,
wherein the scaffold of each guide RNA is capable of interacting with the engineered Cas12f1 protein to form a complex, and
the spacer of each guide RNA is designed to target a predetermined target sequence.

### Example 89. Ribonucleoprotein encompassed

The engineered CRISPR/Cas12f1 composition of Example 88,
wherein the engineered CRISPR/Cas12f1 composition comprises the engineered Cas12f1 protein and the one guide RNA in a form of ribonucleoprotein (RNP).

### Example 90. Coding nucleic acids encompassed

The engineered CRISPR/Cas12f1 composition of Example 88,
wherein the engineered CRISPR/Cas12f1 composition comprises a nucleic acid encoding the engineered Cas12f1 protein and a nucleic acid encoding the at least one guide RNA.

### Example 91. Vector encompassed

The engineered CRISPR/Cas12f1 composition of Example 90, wherein the engineered CRISPR/Cas12f1 composition comprises the nucleic acid encoding the engineered Cas12f1 protein and the nucleic acid encoding the at least one guide RNA in a form of the vector of any one of Examples 82 to 87.

### Example 92. Base editing composition

The engineered CRISPR/Cas12f1 composition of any one of Examples 88 to 91,
wherein the engineered Cas12f1 protein is the dCas12f1-base editing fusion protein of any one of Examples 54 to 61, and
the engineered CRISPR/Cas12f1 composition may be referred to as a base editing composition.

### Example 93. ABE composition

The engineered CRISPR/Cas12f1 composition of Example 92,
wherein the engineered Cas12f1 protein is the adenosine deaminase of any one of Examples 55 to 57, and
the engineered CRISPR/Cas12f1 composition may be referred to as an adenine base editor (ABE) composition.

### Example 94. CBE composition

The engineered CRISPR/Cas12f1 composition of Example 92,
wherein the engineered Cas12f1 protein is the cytosine deaminase of any one of Examples 58 to 61, and
the engineered CRISPR/Cas12f1 composition may be referred to as a cytosine base editor (CBE) composition.

### Example 95. Expression regulating composition

The engineered CRISPR/Cas12f1 composition of any one of Examples 88 to 94,
wherein the engineered Cas12f1 protein is the dCas12f1-expression regulating fusion protein of any one of Examples 62 to 77, and
the engineered CRISPR/Cas12f1 composition may be referred to as an expression regulating composition.

### Pharmaceutical composition comprising engineered CRISPR/Cas12f1 system

### Example 96. Pharmaceutical composition, general

A pharmaceutical composition for treating cancer, a genetic disease, or an infectious disease, comprising:
the CRISPR/Cas12f1 composition of any one of Examples 88 to 95; and
a pharmaceutically acceptable carrier.

### Example 97. Carrier defined

The pharmaceutical composition of Example 96, wherein the pharmaceutically acceptable carrier is at least one selected from the following:
a binder such as lactose, saccharose, sorbitol, mannitol, starch, amylopectin, cellulose, or gelatin; an excipient such as dicalcium phosphate; a disintegrant such as corn starch or sweet potato starch; a lubricant such as magnesium stearate, calcium stearate, sodium stearyl fumarate, or polyethylene glycol wax; a sweetener; a fragrance; syrup; a liquid carrier such as fatty oil; a sterile aqueous solution; propylene glycol; polyethylene glycol; injectable ester such as ethyl oleate; a suspension; an emulsion; a freeze-dried preparation; a topical preparation; a stabilizer; a buffering agent; animal oil; vegetable oil; wax; paraffin; starch; tragacanth; cellulose derivative; polyethylene glycol; silicon; bentonite; silica; talc; zinc oxide.

### Base editing method for target gene

### Example 98. Base editing method 1

A method for editing, correcting, or replacing a base contained in a target gene of a cell, comprising:
delivering, injecting, or administering the base editing composition of any one of Examples 92 to 94 to the cell,
wherein the spacer of the guide RNA in the base editing composition is capable of targeting, recognizing, or hybridizing with a target sequence contained in the target gene of the cell.

### Example 99. Base editing method 2

A method for editing a base contained in a target gene of a cell, comprising:
inducing the base editing complex of Example 80 and the target gene to come in contact with each other in the cell,
wherein the spacer of the guide RNA in the base editing composition is capable of targeting, recognizing, or hybridizing with a target sequence contained in the target gene of the cell.

### Example 100. Mechanism specified

The method of any one of Examples 98 to 99, wherein the target gene of the cell is double-stranded DNA and comprises a target strand and a non-target strand,
the target strand has the target sequence,
the non-target strand has a protospacer adjacent motif (PAM) and a protospacer,
the protospacer has a sequence complementary to the target sequence,
the spacer of the guide RNA is capable of targeting, recognizing, or hybridizing with the target sequence contained in the target gene of the cell, and
the method causes at least one adenine contained in or adjacent to the protospacer to be edited to, corrected to, or replaced with guanine, and/or at least one cytosine contained in or adjacent to the protospacer to be edited to, corrected to, or replaced with thymine.

### Example 101. ABE method

The method of any one of Examples 98 to 100, wherein the base editing composition is the ABE composition of Example 93, and
the method causes at least one adenine contained in the protospacer to be edited to, corrected to, or replaced with guanine.

### Example 102. ABE method, base editing window defined

The method of Example 101,
wherein the protospacer of the target gene contains at least one adenosine at 1st, 2nd, 3rd, 4th, 5th, 6th, 7th, 8th, 9th, 10th, 11th, 12th, 13th, 14th, 15th, 16th, 17th, 18th, 19th, 20th, 21st, 22nd, 23rd, 24th, 25th, 26th, 27th, 28th, 29th, or 30th position from the 5' end, and
the method causes the at least one adenine contained in the protospacer to be edited to, corrected to, or replaced with guanine.

### Example 103. CBE method

The method of any one of Examples 98 to 100, wherein the base editing composition is the CBE composition of Example 94, and
the method causes at least one cytosine contained in the protospacer to be edited to, corrected to, or replaced with thymine.

### Example 104. CBE method, base editing window defined

The method of Example 101,
wherein the protospacer of the target gene contains at least one cytosine at 1st, 2nd, 3rd, 4th, 5th, 6th, 7th, 8th, 9th, 10th, 11th, 12th, 13th, 14th, 15th, 16th, 17th, 18th, 19th, 20th, 21st, 22nd, 23rd, 24th, 25th, 26th, 27th, 28th, 29th, or 30th position from the 5' end, and
the method causes the at least one cytosine contained in the protospacer to be edited to, corrected to, or replaced with thymine.

### Method for regulating expression of target gene

### Example 105. Expression regulating method

A method of regulating expression of a target gene in a cell, comprising:
delivering, injecting, or administering the expression regulation composition of any one of Examples to the cell,
wherein the spacer of the guide RNA in the expression regulating composition is capable of targeting, recognizing, or hybridizing with a target sequence contained in the target gene of the cell.

### Example 106. Result obtained by performing method

The method of Example 105, wherein introduction of the expression regulating composition causes formation of a CRISPR expression regulating complex in the cell, and
the CRISPR expression regulating complex regulates expression of the target gene.

### Example 107. Method for promoting gene expression

The method of Example 106,
wherein the expression regulating composition comprises the dCas12f1-expression regulating fusion protein of Example 75,
the CRISPR expression regulating complex is a CRISPR activation complex, and
the CRISPR activation complex promotes expression of the target gene.

### Example 108. Method for inhibiting gene expression

The method of Example 106,
wherein the expression regulating composition comprises the dCas12f1-expression regulating fusion protein of Example 76,
the CRISPR expression regulating complex is a CRISPR interference complex, and
the CRISPR interference complex inhibits expression of the target gene.

### Use of engineered Cas12f1 protein

### Example 109. Use of engineered Cas12f1 protein

A use of an engineered Cas12f1 protein, which is selected from the dead Cas12f1 protein of any one of Examples 1 to 12, the dCas12f1-base editing protein of any one of Examples 54 to 61, and the dCas12f1-expression regulating protein of any one of Examples 62 to 77, for use in a method for regulating gene expression.

### Example 110. Base editing use defined

The use of Example 109, wherein the engineered Cas12f1 protein is selected from the dCas12f1-base editing protein of any one of Examples 54 to 61, and
the method for regulating gene expression is the method of base editing for a target gene of any one of Examples 98 to 104.

### Example 111. Expression regulating use defined

The use of Example 109, wherein the engineered Cas12f1 protein is selected from the dCas12f1-expression regulating protein of any one of Examples 62 to 77, and
the method for regulating gene expression is the method of regulating expression of a target gene of any one of Examples 105 to 108.

### Mode for Carrying Out Invention

Hereinafter, the present disclosure will be described in more detail by way of experimental examples and examples. These examples are merely for illustrating embodiments disclosed by the present specification, and it will be obvious to those skilled in the art that a scope of the embodiments disclosed by the present specification is not to be construed as being limited by these examples.

### Experimental Example 1. Experimental method and material

### Experimental Example 1.1. Preparation of Cas12f1 protein

For expression in human cells, the Cas12f1 gene was codon-optimized (SEQ ID NO: 460), and the optimized sequence was synthesized for vector construction. Finally, to the sequence encoding the Cas12f1 protein are added a chicken β-actin promoter, 5'- and 3'-terminal nuclear localization signal sequences, and a sequence encoding eGFP linked by a self-cleaving T2A peptide.

A template DNA encoding (engineered) Cas12f1 guide RNA was synthesized and cloned into the pTwist Amp plasmid vector (Twist Bioscience). If necessary, the vector was used as a template for amplifying the guide RNA-encoding sequence using a U6-complementary forward primer and a protospacer-complementary reverse primer. Using a Gibson assembly, an oligonucleotide encoding the engineered Cas12f1 guide RNA was cloned into the vector comprising the codon-optimized Cas12f1 gene, so that a vector for an engineered CRISPR/Cas12f1 system was constructed.

### Experimental Example 1.2, Preparation of dead Cas12f1 variant protein

The Cas12f1-expressing vector constructed in Experimental Example 1.1 was subjected to mutagenesis so that Cas12f1 was modified into its dead form, and various dead-Cas12f1 proteins were prepared which could be used in respective experiments. An appropriate primer was used depending on each of the dead forms (see each experimental example).

### Experimental Example 1.3. Preparation of base editing domain fusion protein

A nucleotide sequence encoding the base editing domain fusion protein to be used in each experiment was specified, and then the base editing domain fusion protein was prepared in the following manner:

The coding nucleotide sequence was cloned into a pMAL-c2 plasmid vector for replication. The plasmid vector was used to transform BL21(DE3) E. coli. The colonies of the transformed E. coli were grown in LB broth at 37°C until their optical density reached 0.7. The transformed E. coli were incubated overnight at 18°C in the presence of 0.1 mM isopropylthio-β-D-galactoside.

Then, the transformed E. coli were centrifuged at 3,500 g for 30 minutes and collected. The collected transformed E. coli were resuspended in 20mM Tris-HCl (pH 7.6), 500mM NaCl, 5mM β-mercaptoethanol, 5% glycerol. The resuspended E. coli were lysed and disrupted through sonication. Each sample containing the disrupted E. coli was centrifuged at 15,000 g for 30 minutes, and then the supernatant was filtered through a 0.45 µm syringe filter (Millipore). The dCas12f1 protein bound to adenosine deaminase or cytidine deaminase, which was present in the filtered supernatant, was loaded onto a Ni²⁺-affinity column using an FPLC purification system (KTA Purifier, GE Healthcare). The loaded dCas12f1 protein was eluted in a gradient of 80 to 400 mM imidazole, 20 mM Tris-HCl (pH 7.5).

The eluted protein was treated with TEV protease for 16 hours. The isolated protein was purified on a heparin column with a linear concentration gradient of 0.15 to 1.6 M NaCl. The recombinant Cas12f1 protein purified by the heparin column was dialyzed against 20 mM Tris pH 7.6, 150 mM NaCl, 5 mM β-mercaptoethanol, 5% glycerol. The dialyzed protein was purified by passing through an MBP column and then re-purified on a monoS column (GE Healthcare) or EnrichS with a linear gradient of 0.5 to 1.2 M NaCl. The re-purified proteins were collected and dialyzed against 20 mM Tris pH 7.6, 150 mM NaCl, 5 mM β-mercaptoethanol, 5% glycerol so that a hypercompact base editing construct was purified. A concentration of the produced protein was quantified by Bradford quantification using bovine serum albumin (BSA) as a standard and measured electropheromerically on a Coomassie blue-stained SDS-PAGE gel.

### Experimental Example 1.4. Preparation of gene expression regulatory domain fusion protein

Into the N-terminus and/or C-terminus of the dead Cas12f1 prepared in Experimental Example 1.2 was cloned each of KRAB, MeCP2, and DNMT3A, or a combination thereof (FIGS. 39 to 49). The vector and insert fragment were amplified with KOD-one (TOYOBO) using templates, and then ligation was performed according to the protocol using Gibson Assembly^{®} Master Mix (NEB). The template DNA and primers used to produce each of the vector and the insert fragment are shown in Tables 1 and 2.

**[Table 1]**

| Clone | Donor | | Primer | Primser sequence (5' to 3') |
|---|---|---|---|---|
| dCas12f-KRAB | Vector | dCas9-KRAB | dCas9_KRAB_R | AAGGGTTCGATCCTCTAGAGTC (SEQ ID NO:462) |
| | | (Addgene,#110820) | dCas9_KRAB_F | AGTGGTGGAGGAAGTGGC (SEQ ID NO:463) |
| | Insert | px458-dCas12f1- | NLS_dCas14_KRAB_F | GATCCAGCCTCCGGACTCTAGAGGATCGAAC CCTTGCCACCATGGCCCCAAAGAAGAAGCG (SEQ ID NO:464) |
| | | dual-v4.1(-2) | NLS_dCas14_KRAB_R | TCGCGTCCATCGACCCTGACCCGCCACTTCCT CCACCACTCTTTTTCTTTTTTGCCTGGC (SEQ ID NO:465) |
| KRAB-dCas12f1 | Vector | KRAB-dCas9 | dCas9_KRAB_R | AAGGGTTCGATCCTCTAGAGTC AAGGGTTCGATCCTCTAGAGTC (SEQ ID NO:466) |
| | | (Addgene,#112195) | dCas9_KRAB_F | AGTGGTGGAGGAAGTGGC (SEQ ID NO:467) |
| | Insert | px458-dCas12f1- | NLS_KRAB_dCas14_Gib_F | GCGGCGGTTCTGGTGGCTCAGGCGGCAGCGG CCGGCCACCAAAGAAGAAGCGGAAGGTCG (SEQ ID NO:468) |
| | | dual-v4.1(-2) | NLS_KRAB_dCas14_Gib_R | TTTAAACTCATTACTAACCGGTAGGGATCGA ACCCTTTCACTTTTTCTTTTTTGCCTGGC (SEQ ID NO:469) |
| dCas12f1-KRAB-MeCP2 | Vector | dCas9-KRAB-M eCP2 | dCas9_KRAB_R | AAGGGTTCGATCCTCTAGAGTC (SEQ ID NO:470) |
| | | (Addgene,#110821) | dCas9_KRAB_F | AGTGGTGGAGGAAGTGGC (SEQ ID NO:471) |
| | Insert | px458-dCas12f1- | NLS_dCas14_KRAB_F | GATCCAGCCTCCGGACTCTAGAGGATCGAAC CCTTGCCACCATGGCCCCAAAGAAGAAGCG (SEQ ID NO:472) |
| | | dual-v4.1(-2) | NLS_dCas14_KRAB_R | TCGCGTCCATCGACCCTGACCCGCCACTTCCT CCACCACTCTTTTTCTTTTTTGCCTGGC (SEQ ID NO:473) |
| KRAB-MeCP2-dCas 12f1 | Vector | KRAB-dCas12f1 | KRAB_meCP2_dCas12f1_KRAB_Vector_R | GGCCATGGTGGCAAGGGTTC (SEQ ID NO:474) |
| | | | KRAB_MeCP2_dCas12f1_KRAB_Vector_F | CCAAAGAAGAAGCGGAAGGTC (SEQ ID NO:475) |
| | Insert | dCas9-KRAB-MeCP2 | KRAB_MeCP2_dCas12f1_KRAB_Gibson_F | CCTCCGGACTCTAGAGGATCGAACCCTTGCCA CCATGGCCCCCAAGAAGAAGAGGAAGGT (SEQ ID NO:476) |
| | | | KRAB_MeCP2_dCas12f1_KRAB_Gibson_R | CTGGGACTCCGTGGATACCGACCTTCCGCTTC TTCTTTGGTGAGACTCTCTCAGTCACGG (SEQ ID NO:477) |
| dCas12f1-DNM T3A | Vector | dCaS12f1-KRAB | dCas12f1_DNMT3A_Vector_F | TAGAAGAATTCGATCCCTACCGG (SEQ ID NO:478) |
| | | | dCas12f1_DNMT3A_Vector_R | CTTTTTCTTTTTTGCCTGGCCG (SEQ ID NO:479) |
| | Insert | pdCas9-DNM T3A-EGFP (Addgene, #71666) | dCas12f1_DNMT3A_Gibson_F | CCAAAAGGCCGGCGGCCACGAAAAAGGCCGG CCAGGCAAAAAAGAAAAAGCTCGAGGGCGGA GGCGGGAGCGGA (SEQ ID NO:480) |
| | | | dCas12f1_DNMT3A_Gibson_R | TTTAAACTCATTACTAACCGGTAGGGATCGA ATTCTTCTACACACACGCAAAATACTCCT (SEQ ID NO:481) |
| DNM T3A-dCas12f1 | Vector | DNM T3A-dCas9 | DNMT3A_dCas12f1_Vector_F | TGAAAGGGTTCGATCCCTACC (SEQ ID NO:482) |
| | | (A ddgene, # 100090) | DNMT3A_dCas12f1_Vector_R | TGGCCGGCCGCTGCCGCCTGAG (SEQ ID NO:483) |
| | Insert | dCas12f1-KRAB | DNMT3A_dCas12f1_Gibson_F | AGCGGCGGTTCTGGTGGCTCAGGCGGCAGCG GCCGGCCACCAAAGAAGAAGCGGAAGGTC (SEQ ID NO:484) |
| | | | DNMT3A_dCas12f1_Gibson_R | TTAAACTCATTACTAACCGGTAGGGATCGAA CCCTTTCACTTTTTCTTTTTTGCCTGGCC (SEQ ID NO:485) |

**[Table 2]**

| Clone | Donor | | Primer | Primser sequence (5'-) |
|---|---|---|---|---|
| d C as 12f I - hHDAC3 | Vector | dCas12f1-KRAB | dCas12f1_hHDAC3_Vector_F | AAGAATTCGATCCCTACCGGTT (SEQ ID NO:486) |
| | | | dCas12f1_hHDAC3_Vector_R | CTTTTTCTTTTTTGCCTGGCCGG (SEQ ID NO:487) |
| | Insert | dCas9-hHDAC3 (Addgene,#98591) | dCas12f1_hHDAC3_Gibson_F | GGCGGCCACGAAAAAGGCCGGCCAGGCAAAA AAGAAAAAGGCGGCCGCTGGATCCATGGC (SEQ ID NO:488) |
| | | | dCas12f1_hHDAC3_Gibson_R | CCGTTTAAACTCATTACTAACCGGTAGGGAT CGAATTCTTTTAAATCTCCACATCGCTTT (SEQ ID NO:489) |
| dCas12f1-KRAB-KRAB | Vector | dCas12f1-KRAB-M eCP2 | dCas12f1_KRAB_KRAB_Vector_F | TAGAAGAATTCGATCCCTACCGG (SEQ ID NO:490) |
| | | | dCas12f1_KRAB_KRAB_Vector_R | TGAGGCTTCCACCTTCCGTTTC (SEQ ID NO:491) |
| | Insert | dCas12f1-KRAB | dCas12f1_KRAB_KRAB_Gbson_F | GGAGGTGGTTCGGGTGGCTCTGGATCAAGCC CAAAGAAGAAACGGAAGGTGGAAGCCTCAAG AACACTGGTTACGTTCAA (SEQ ID NO:492) |
| | | | dCas14_KRAB_KRAB_Ginson_R | GTTTCAGTTAGCCTCCCCCGTTTAAACTCATT ACTAACCGGTAGGGATCGAATTCTTCTATAC CAGCCAAGGTTCTTCCC (SEQ ID NO:493) |
| d C as 12f I - KRAB-KRAB-M eCP2 | Vector | dCas12f1-KRAB-M eCP2 | dCas12f1_K_K_MeCP2_Vector_F | AGTGGTGGAGGAAGTGGC (SEQ ID NO:494) |
| | | | dCas12f1_K_K_MeCP2_Vector_R | CTTTTTCTTTTTTGCCTGGCCG (SEQ ID NO:495) |
| | Insert | dCas12f1-KRAB-M eCP2 | dCas12f1_K_K_MeCP2_Gibson_F | GGCGGCCACGAAAAAGGCCGGCCAGGCAAAA AAGAAAAAGAGTGGTGGAGGAAGTGGCGG (SEQ ID NO:496) |
| | | | dCas12f1_K_K_MeCP2_Gibson_R | TCGCGTCCATCGACCCTGACCCGCCACTTCCT CCACCACTTACCAGCCAAGGTTCTTCCC (SEQ ID NO:497) |
| KRAB-dCas12f1-M eCP2 | Vector | KRAB-dCas12f1 | KRAB_dCas12f1_MeCP2_Vector_R | CTTTTTCTTTTTTGCCTGGCCG (SEQ ID NO:498) |
| | | | KRAB_dCas12f1_MeCP2_Vector_F | TGAAAGGGTTCGATCCCTACC (SEQ ID NO:499) |
| | Insert | dCas12f1-KRAB-M eCP2 | KRAB_dCas12f1_MeCP2_Gibson_F | GGCGGCCACGAAAAAGGCCGGCCAGGCAAAA AAGAAAAAGGAAGCCTCAGTGCAGGTGAA (SEQ ID NO:500) |
| | | | KRAB_dCas12f1_MeCP2_Gibson_R | TTTAAACTCATTACTAACCGGTAGGGATCGA ACCCTTTCATGAGACTCTCTCAGTCACGG (SEQ ID NO:501) |
| KRAB-MeCP2-dCas12f1-KRAB | Vector | dCas12f1-KRAB | KRAB_MeCP2_dCas14_KRAB_Vector_R | GGCCATGGTGGCAAGGGTTC (SEQ ID NO:502) |
| | | | KRAB_MeCP2_dCas14_KRAB_Vector_F | CCAAAGAAGAAGCGGAAGGTC (SEQ ID NO:503) |
| | Insert | KRAB-MeCP2-dCas12f1 | KRAB_MeCP2_dCas14_KRAB_Gibson_F | CCTCCGGACTCTAGAGGATCGAACCCTTGCCA CCATGGCCCCCAAGAAGAAGAGGAAGGT (SEQ ID NO:504) |
| | | | KRAB_MeCP2_dCas14_KRAB_Gibson_R | CTGGGACTCCGTGGATACCGACCTTCCGCTTC TTCTTTGGTGAGACTCTCTCAGTCACGG (SEQ ID NO:505) |
| MeCP2-d C as 12f-KRAB | Vector | dCas12f1-KRAB | MeCP2_dCas14_KRAB_Vector_F | CCAAAGAAGAAGCGGAAGGTCGG (SEQ ID NO:506) |
| | | | MeCP2_dCas14_KRAB_Vector_R | GGCCATGGTGGCAAGGGTTC (SEQ ID NO:507) |
| | Insert | KRAB-MeCP2-dCas12f1 | MeCP2_dCas14_KRAB_Gibson_F | CCTCCGGACTCTAGAGGATCGAACCCTTGCCA CCATGGCCCCAAAGAAGAAACGGAAGGT (SEQ ID NO:508) |
| | | | MeCP2_dCas14_KRAB_Gibson_R | CTGGGACTCCGTGGATACCGACCTTCCGCTTC TTCTTTGGTGAGACTCTCTCAGTCACGG (SEQ ID NO:509) |

### Experimental Example 1.4. Preparation of guide RNA

A nucleotide sequence of the guide RNA to be used in each experiment was specified, and then the guide RNA was prepared in the following manner:
To prepare the designed Cas12f1 sgRNA, a guide RNA was prepared by chemically synthesizing the pre-designed guide RNA therefor, and then a PCR amplicon was prepared which comprises the pre-designed guide RNA sequence and the T7 promoter sequence.

Ligation of a U-rich tail to the 3'-end of the engineered Cas12f1 single guide RNA was performed using Pfu PCR Master Mix5 (Biofact) in the presence of sequence-modified primers and a plasmid vector for the Cas12f1 guide RNA. The PCR amplicon was purified using HiGene^{™} Gel & PCR Purification System (Biofact).

In addition, modification of the second, fourth, and fifth regions in the engineered scaffold region of the engineered Cas12f1 single guide RNA was performed by cloning synthetic oligonucleotides containing modified sequences into the guide RNA-encoding vector that had been linearized using Apol and BamHI restriction enzymes.

Modification of the first region in the engineered scaffold region of the engineered Cas12f1 single guide RNA was performed by PCR amplification of a canonical or engineered template plasmid vector using a forward primer that targets the 5'-end portion of tracrRNA and a reverse primer that targets the U6 promoter region.

The PCR amplification was performed by Q5 Hot Start high-fidelity DNA polymerase (NEB), and the PCR products were ligated using KLD Enzyme Mix (NEB). The ligated PCR products were transformed into DH5α E.coli cells. Mutagenesis was identified by Sanger sequencing.

The modified plasmid vector was purified using the NucleoBond ^{®}Xtra Midi EF kit (MN). One microgram of the purified plasmid was used as a template for synthesis of mRNA using T7 RNA polymerase (NEB) and NTPs (Jena Bioscience). The thus prepared engineered Cas12f1 guide RNA was purified using the Monarch^{®}RNA cleanup kit (NEB), aliquoted into cryogenic vials, and stored in liquid nitrogen.

To prepare amplicons of the guide RNA and the engineered guide RNA, a template DNA plasmid for the canonical guide RNA and a template DNA plasmid for the engineered guide RNA were subjected to PCR amplification with KAPA HiFi HotStart DNA polymerase (Roche) or Pfu DNA polymerase (Biofact) using a U6-complementary forward primer and a protospacer sequence-complementary reverse primer. The resulting products of PCR amplification were purified using the Higene^{™} Gel & PCR purification system (Biofact) to obtain the amplicons of the guide RNA and the engineered guide RNA.

Using the PCR amplicon as a template, in vitro transcription was performed with NEB T7 polymerase. The resulting products of in vitro transcription were treated with NEB DNase I, and the purified using the Monarch RNA Cleanup Kit (NEB) to obtain the guide RNA. Then, a plasmid vector containing the pre-designed guide RNA sequence and the T7 promoter sequence was prepared according to the Tblunt plasmid cloning method.

The vector was purified by double-cutting both ends of the guide RNA sequence that contains the T7 promoter sequence, and then the resulting products were subjected to in vitro transcription using NEB T7 polymerase. The resulting products of in vitro transcription were treated with NEB DNase I, and then purified using the Monarch RNA Cleanup Kit (NEB) to obtain the guide RNA.

The sequences of the guide RNAs used in the following experimental examples are as shown in Table 3.

**[Table 3]**

| Label | Scaffold sequence (5' to 3') | SEQ ID NO | spacer | U-richtail (5' to 3') | SEQ ID NO |
|---|---|---|---|---|---|
| v3.0 | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAA CCCUCGAAACAAAUUCAGUGCUCCUCUCCAAUUC UGCACAAGAAAGUUGCAGAACCCGAAUAGAGCA AUGAAGGAAUGCAAC | 197 | Depend on target sequence, 10nt to SOot | UUUUAUUUUUU | 231 |
| v4.0 | ACCGCUUCACCAAAAGCUGUCCCUUAGGGGAUUA GAACUUGAGUGAAGGUGGGCUGCUUGCAUCAGC CUAAUGUCGAGAAGUGCUUUCUUCGGAAAGUAA CCCUCGAAACAAAGAAAGGAAUGCAAC | 198 | Depend on target sequence, 10nt to SOnt | UUUUAUUUUUU | 231 |
| v4.1 | ACCGCUUCACUUAGAGUGAAGGUGGGCUGCUUGC AUCAGCCUAAUGUCGAGAAGUGCUUUCUUCGGA AAGUAACCCUCGAAACAAAGAAAGGAAUGCAAC | 199 | Depend on target sequence, 10nt to 50nt | UUUUAUUUUUU | 231 |

Here, each guide RNA has a structure of 5'-scaffold-spacer-U-rich tail-3', and the spacer is designed as an RNA sequence (T in the DNA sequence is replaced with U) equivalent to the proto spacer in each experimental example.

### Experimental Example 1.5. Preparation of ribonucleoprotein (RNP)particles

A combination of the engineered Cas12f1 protein (dead Cas12f1 protein, dCas12f1-base editing fusion protein, and/or dCas12f1-expression regulating fusion protein) and the guide RNA to be used in each experiment was specified, and then RNP particles were prepared in the following manner:

300 nM of the engineered Cas12f1 protein prepared in Experimental Examples 1.1 to 1.3 and 900 nM of the guide RNA prepared in Experimental Example 1.4 were incubated at room temperature for 10 minutes to prepare ribonucleoprotein particles (RNPs).

### Experimental Example 1.6. Design and construction of plasmid, rAAV vector (AAV inverted terminal repeat vector)

The engineered Cas12f1 protein (dead Cas12f1 protein, dCas12f1-base editing fusion protein, and/or dCas12f1-expression regulating composition), the guide RNA, and other vector component(s) to be used in each experiment were specified, and then a plasmid vector was designed and constructed in the following manner.

The engineered Cas12f1 protein was human codon-optimized for expression in human cells, and a polynucleotide containing the codon-optimized nucleotide sequence was synthesized.

The codon-optimized polynucleotide encoding the engineered Cas12f1 protein was cloned by being operably linked to a plasmid that comprises a chicken β-actin (CBA) promoter, 5'- and 3'-terminal nuclear localization signal (NLS) sequences, and a sequence encoding eGFP linked by a self-cleaving T2A peptide, or a plasmid that comprises a CMV enhancer, a CMV promoter, 5'- and 3'-terminal nuclear localization signal (NLS) sequences.

In addition, the template DNA for the specified guide RNA was synthesized (Twist Bioscience), and cloned into the pTwist Amp plasmid vector for replication. The template DNA for the engineered guide RNA was prepared using an enzyme cloning technique, and was cloned into the pTwist Amp plasmid for replication. In addition, the amplicons of the guide RNA and the engineered guide RNA were prepared with the plasmids as templates using a U6-complementary forward primer and a protospacer sequence-complementary reverse primer. If necessary, the prepared amplicon was cloned into the T-blunt plasmid (Biofact) for replication.

In addition, to prepare an engineered dual guide RNA, oligonucleotides encoding the engineered tracrRNA and the engineered crRNA were cleaved with the restriction enzymes BamHI and HindIII (New England Biolabs) and cloned into pSilencer 2.0 (ThermoFisher Scientific) for replication.

A template DNA encoding the specified guide RNA was synthesized, and cloned into the pTwist Amp plasmid vector (Twist Bioscience). If necessary, the vector was used as a template for amplification of the guide RNA-encoding sequence with a U6-complementary forward primer and a proto spacer-complementary reverse primer.

Using a Gibson assembly, the polynucleotide encoding the guide RNA was cloned into the vector that contains the codon-optimized polynucleotide encoding the engineered Cas12f1 protein, thereby constructing a vector that expresses the respective components of the engineered Cas12f1 system.

Specifically, adeno-associated virus inverted terminal repeat plasmid vectors (AAV inverted terminal repeat vectors) and vectors were constructed in which 1) a chicken β-actin (CBA) promoter, 5'- and 3'-terminal nuclear localization signal sequences, and a sequence encoding eGFP linked by a self-cleaving T2A peptide, or 1)-1 a CMV enhancer, a CMV promoter, and 5'- or 3'-terminal nuclear localization signal (NLS) sequences, 2) the codon-optimized polynucleotide encoding the engineered Cas12f1 protein, and 3) the guide RNA were operably linked.

Here, transcription of the engineered Cas12f1 protein and the guide RNA was driven by the chicken β-actin(CBA) promoter and U6 promoter; or the CMV promoter and U6 promoter, respectively. In addition, the vector and AAV vector may be appropriately changed depending on the purpose of gene editing, such as eGFP, number of guide RNAs, and/or addition of an effector protein.

For mass-production of the AAV, the AAV vector, a helper plasmid, and an RC plasmid were transformed into HEK 293T cells, and the transformed HEK 293T cells were cultured in DMEM medium containing 2% FBS. Recombinant pseudotyped AAV vector stocks were generated with PEIpro (Polyplus-transfection) and PEI coprecipitation using triple-transfection with plasmids at equal molar ratios. After 72 hours of incubation, the cells were lysed and the lysates were subjected to iodixanol (Sigma-Aldrich) step-gradient ultracentrifugation so that the AAV was purified.

### Experimental Example 1.7. Cell culture and transfection

HEK 293T (ATCC CRL-11268), HeLa (ATCC CLL-2), U-2OS (ATCC HTB-96), and K-562 (ATCC CCL-243) cells were cultured in DMEM medium supplemented with 10% heat-inactivated FBS, 1% penicillin/streptomycin, and 0.1 mM non-essential amino acids at 37°C with 5% CO₂.

For cell transfection with the vector constructed in Experimental Example 1.6, 1.0 × 10⁵ HEK 293T cells were seeded 1 day before transfection. The cell transfection was performed by electroporation or lipofection.

For the electroporation, 2 to 5 µg each of the plasmid vector encoding the engineered Cas12f1 protein and the DNA encoding the guide RNA were transfected into 4 × 10⁵ HEK 293 T cells using the Neon transfection system (Invitrogen). The electroporation was performed under the conditions of 1300 V, 10 mA, and 3 pulses.

For the lipofection, 6 to 15 µl of FuGene reagent (Promega) was mixed for 15 minutes with 2 to 5 µg of the plasmid vector encoding the engineered Cas12f1 protein and 1.5 to 5 µg of the PCR amplicon. The mixture (300 µl) was added to 1.5 ml of DMEM medium plated with 1 × 10⁶ cells 1 day prior to transfection. The cells were cultured in the presence of the mixture for 1 to 10 days. After the culture, the cells were collected, and their genomic DNA was isolated manually using the PureHelixTM genomic DNA preparation kit (NanoHelix) or the Maxwell RSC Cultured cells DNA Kit (Promega).

For cell transfection with the AAV vector constructed in Experimental Example 1.6, human HEK 293T cells were transfected with the AAV vector at different multiplicities of infection (MOI) of 1, 5, 10, 50, 100, 100, 1000, 10000, 50000, and 100000 as determined by quantitative PCR. The transfected HEK 293T cells were cultured in DMEM medium containing 2% FBS. The cells were collected for isolation of genomic DNA at different time points, for example, days 1, 3, 5, 7, and 9.

In addition, transfection of the ribonucleoprotein particles (RNP) prepared in Experimental Example 1.5 was performed using electroporation or lipofection, and 1 day later, transfection of the guide RNA prepared in Experimental Example 1.4 was performed using electroporation.

### Experimental Example 1.8. Analysis of gene editing efficiency

In the genomic DNA isolated from the HEK 293T cells, a region containing the protospacer was subjected to PCR in the presence of KAPA HiFi HotStart DNA polymerase (Roche) using target-specific primers. The amplification method was performed according to the manufacturer's instructions.

The PCR amplicon, which resulted from the amplification and contains Illumina TruSeq HT dual indexes, was subjected to 150bp paired-end sequencing using Illumina iSeq 100. Indel frequency was calculated using MAUND. The MAUND is available at https://github.com/ibscge/maund.

PCR products were obtained using BioFACT^{™} Lamp Pfu DNA polymerase. The PCR products (100 to 300 µg) were allowed to react with 10 units of T7E1 enzyme (NewEngland Biolabs) in 25 µg of the reaction mixture at 37°C for 30 minutes. 20 µl of the reaction mixture was loaded directly onto a 10% acrylamide gel, and the cleaved PCR products were run in a TBE buffer system. Gel images were stained with ethidium bromide solution, and then digitized using the Printgraph 2 M gel imaging system (Atto). The digitized results were analyzed to evaluate gene editing efficiency. In addition, to identify unwanted indels, analysis was performed through NGS.

### Experimental Example 1.9. Analysis of base editing activity in cell

Deamination analysis of adenine (A) base or cytosine (C) base located at a target site of a target nucleic acid or target gene in a cell was performed as follows.

A vector capable of expressing the engineered Cas12f1 system was transfected into cells according to Experimental Example 1.7. After 3, 5, and 7 days, genomic DNA was obtained from the transfected HEK 293T cells, and purified using a Genomic DNA prep kit (QIAGEN, catalog #: 69504). In the purified products, the target site of the target nucleic acid or target gene was amplified by PCR, and then the final PCR products were analyzed using targeted deep sequencing.

The target site was amplified for generation of a library using the KAPA HiFi HotStart PCR kit (KAPA Biosystems #: KK2501). This library was sequenced using MiniSeq with the TruSeq HT Dual Index system (Illumina).

### Experimental Example 1.10. Analysis of gene expression suppression effect in cell

To suppress gene expression in a cell, the dCas12f1-gene expression inhibiting fusion protein and the guide RNA to be used therewith were specified, and then a gene expression suppression effect thereof in a cell was analyzed using the following method:

1.5 µg of the engineered Cas12f1 expression vector constructed in Experimental Example 1.6 and 0.5 µg of guide RNA cassette were each transfected into HEK293T-lentiX cells according to Experimental Example 1.7. The cells were harvested 96 hours after transfection. RNA was extracted therefrom using the Maxwell^{®} RSC miRNA Tissue Kit (Promega). cDNA was synthesized from 1 µg of RNA using SuperScript IV Reverse Transcriptase (Invitrogen) according to the protocol.

### Experimental Example 1.11. Analysis of gene expression promoting effect in cell

To promote gene expression in a cell, the dCas12f1-gene expression promoting protein and the guide RNA to be used therewith were specified, and then a gene expression promoting effect thereof in a cell was analyzed using the following method:

The dCas12f1-gene expression promoting fusion protein expression vector constructed in Experimental Example 1.6 and the guide RNA expression vector were transfected into HEK293T cells using FugeneHD (Promega) reagent. The transfection method was performed according to the protocol for the reagent.

72 hours after transfection, the cells were harvested and RNA was extracted therefrom. cDNA was synthesized from the extracted RNA using the SuperScript iV (Invitrogen) kit. Changes in expression were analyzed by qPCR using the cDNA as a template.

### Experimental Example 1.12, Statistical analysis

Statistical significance verification by a two-tailed Student's t-test was performed by Sigma Plot software (ver. 14.0). A p-value of less than 0.05 was considered statistically significant, and the p-value is shown in each drawing. Error bars for all data were plotted using Sigma plot and mean the standard deviation value of each data. The sample size was not pre-determined based on statistical methods. The experiment for each experimental example was performed three times, and the average of the respective values was used for analysis.

For the dead Cas12f1 variants prepared in Experimental Example 1.2, their gene cleavage activity in a cell was identified by Experimental Examples 1.7 and 1.9. The examples used in the experiment are shown in the table below, and Target-3 was used as the target protospacer sequence.

**[Table 4] Dead Cas12f1 variant**

| No | Label | dCas12f1 | guide RNA |
|---|---|---|---|
| 1 | TnpB(D354A) | SEQ ID NO: 15 | v4.1 |
| 2 | TnpB(E450A) | SEQ ID NO: 16 | v4.1 |
| 3 | TnpB(R518A) | SEQ ID NO: 17 | v4.1 |
| 4 | TnpB(D538A) | SEQ ID NO: 18 | v4.1 |
| 5 | wt TnpB | SEQ ID NO: 14 | v4.1 |
| 6 | TnpB(D354A, D538A) | SEQ ID NO: 22 | v4.1 |
| 7 | TnpB(D354A, E450A, R518A) | SEQ ID NO: 23 | v4.1 |
| 8 | TnpB(D354A, E450A, R518A, D538A) | SEQ ID NO: 24 | v4.1 |

It was identified that the prepared variants were dead forms that did not generate indels (FIG. 5).

### Experimental Example 3. Base editing effect of base editing domain fusion protein

### Experimental Example 3.1. Selection of target sequence

To investigate whether the dCas12f1-base editing fusion protein disclosed herein has base editing activity against a target nucleic acid or gene in a cell, human endogenous DNA target sites, which contain the PAM sequence of Cas12f1 and in which a plurality of adenines (A) are contained within the editing window range, were identified.

The protospacer sequences used in the experiment are as shown in the tables below.

**[Table 5] Base editing target sequence 1**

| Label | PAM (5' to 3') | Protospacer (5' to 3') | SEQ ID NO |
|---|---|---|---|
| Target-1 | TTTG | CACACACACAGTGGGCTACC | 334 |
| Target-2 | TTTG | CATCCCCAGGACACACACAC | 335 |
| Target-3 | TTTA | CAAAGACACTCACCCTGTTG | 336 |
| Target-4 | TTTA | AAGAAAGCTACAGGAAAGCA | 337 |
| Target-5 | TTTA | CAAAACCCAACTGATTCACC | 338 |
| Target-6 | TTTA | CAAAAGCTACCACACATAGC | 339 |
| Target-7 | TTTA | CAAAACTGTGGCCAATACAG | 340 |
| Target-8 | TTTG | GAAAACTGCAGGCAAGATTC | 341 |
| Target-9 | TTTG | CAAAACTGTACACGTGGGCC | 342 |
| Target-10 | TTTG | CAAAACGTGCACAATGTGCA | 343 |

**[Table 6] Base editing target sequence 2**

| Label | PAM (5' to 3') | Protospacer (5' to 3') | SEQ ID NO |
|---|---|---|---|
| Target-11 | TTTA | CCCCCACAGGATTGTAATAA | 344 |
| Target-12 | TTTA | GGCCAAGTGCGAAGTCAGAG | 345 |
| Target-13 | TTTA | CTAGGACACTCACCCTGTTG | 346 |
| Target-14 | TTTG | CTAGCACACAGTGGGCAGAG | 347 |
| Target-15 | TTTA | CTAGGACACTCACCCTGGAG | 348 |
| Target-16 | TTTA | CTAGTCGGCCATTCAGAGAG | 349 |
| Target-17 | TTTA | GCAGTCGGAATGGCGGATGG | 350 |
| Target-18 | TTTA | AGAACACATACCCCTGGGCC | 351 |
| Target-19 | TTTG | CAGTGTGTGCAGGAACGGAG | 352 |
| Target-20 | TTTA | ATACAGAAATCCTAAATGGT | 353 |

**[Table 7] Base editing target sequence 3**

| Label | PAM (5' to 3') | Protospacer (5' to 3') | SEQ ID NO |
|---|---|---|---|
| Target-21 (LOC105370393) | TTTA | AAGAAAGCTACAGGAAAGCA | 354 |
| Target-22 (ZNF10) | TTTA | AATAAGTCTTACCACGTGTC | 355 |
| Target-23 (FUS) | TTTA | ACAAAGAAACCAGCAGTGGC | 356 |
| Target-24 (OR4K17) | TTTA | ACAAGTTCAGAATCACCTTA | 357 |
| Target-25 (POLRMT) | TTTA | AGGACTATGTGTGGCCAGTG | 358 |
| Target-26 (LOC100128398) | TTTA | CAAAGAAATGTACTGCCTTA | 359 |
| Target-27 (CARS) | TTTA | CAACAGCCTCACCAGGAACA | 360 |
| Target-28 (RPH3AL) | TTTA | CACAAGGGATCTGAGACTTG | 361 |
| Target-29 (P2RX5-TAX1BP3) | TTTA | CACATAGGCCATTCAGAAAC | 362 |
| Target-30 (GAK) | TTTA | CAGAGTCCCGGGAACAAGCC | 363 |

**[Table 8] Base editing target sequence 4**

| Label | PAM (5' to 3') | Protospacer (5' to 3') | SEQ ID NO |
|---|---|---|---|
| Target-31 (Intergene) | TTTA | CATACAGGGCTCTGTACCCA | 364 |
| Target-32 (Intergene) | TTTA | CTGAGATTTGCGAAGAGTTA | 3δ5 |
| Target-33 (Intergene) | TTTA | CTTAGTAGTCTCAGAACCAA | 366 |
| Target-34 (Intergene) | TTTA | GAAATATGACTGGAAGTAAA | 367 |
| Target-35 (KLHL29) | TTTA | GAGAGACCGCTCAGGCTGGA | 368 |
| Target-36 (LOC105370393) | TTTA | GCAGTACACCTGAGGGAACA | 369 |
| Target-37 (OSBPL5) | TTTA | GCATTAAGGCCAGCGCTGGG | 370 |
| Target-38 (LOC105369597) | TTTA | GCCATGGTGAAGGTGAAATC | 371 |
| Target-39 (CCDC127) | TTTA | GGCAAGGGTCTTGATGCATC | 372 |
| Target -40 (ZMYM2) | TTTA | GTAGGCTGCTGTTGGACAGA | 373 |
| Target-41 (Intergene) | TTTA | GTCAAATAAAGAAAAATACG | 374 |
| Target-42 (INIP) | TTTA | AGAGCAGCGATTGTAAGGAG | 375 |

### Experimental Example 3.2. Base editing effect 1 of adenosine deaminase fusion protein

An effect of the dCas12f1-base editing fusion protein comprising adenosine deaminase, according to Table 9, was identified in the following manner:

**[Table 9] dCas12f1-base editing fusion protein comprising adenosine deaminase**

| Label | Structure (N to C) | dCas t 2f1 | Deaminase | Linker (SEQ ID) | NLS (SEQ ID) | SEQ ID NO (full sequence) | guide RNA |
|---|---|---|---|---|---|---|---|
| dCas12f1-ABE-N1 | [Deaminase]-[Linker]-[dCas12f1]-[NLS] | dCas12f1(D326A) (SEQ ID NO: 2) | TadA-eTadA1 (SEQ ID NO: 274) | 266 | 201 | 284 | v4.1 |
| dCas12f1-ABE-N2 | [Deaminase]-[Linker]-[dCas12f1]-[NLS] | dCas12f1(D326A) (SEQ ID NO: 2) | eTadA1-TadA (SEQ ID NO: 275) | 266 | 201 | 285 | v4.1 |
| dCas12f1-ABE-C1 | [dCas12f1]-[Linker]-[Deaminase]-[NLS] | dCas12f1(D326A) (SEQ ID NO: 2) | eTadA1-TadA (SEQ ID NO: 275) | 266 | 201 | 286 | v4.1 |
| dCas12f1-ABE-C2 | [dCas12f1]-[Linker]-[Deaminase]-[NLS] | dCas12f1(D326A) (SEQ ID NO: 2) | TadA-eTadA1 (SEQ ID NO: 274) | 266 | 201 | 287 | v4.1 |
| dTnpB-ABE-N1 | [Deaminase]-[Linker]-[dCas12f1]-[NLS] | dCas12f1 variant 1(D354A) (SEQ ID NO: 15) | TadA-eTadA1 (SEQ ID NO: 274) | 266 | 201 | 288 | v4.1 |
| dTnpB-ABE-N2 | [Deaminase]-[Linker]-[dCas12f1]-[NLS] | dCas12f1 variant 1(D354A) (SEQ ID NO: 15) | eTadA1-TadA (SEQ ID NO: 275) | 266 | 201 | 289 | v4.1 |
| dTnpB-ABE-C1 | [dCas12f1]-[Linker]-[Deaminase]-[NLS] | dCas12f1 variant 1(D354A) (SEQ ID NO: 15) | eTadA1-TadA (SEQ ID NO: 275) | 266 | 201 | 290 | v4.1 |
| dTnpB-ABE-C1 | [dCas12f1]-[Linker]-[Deaminase]-[NLS] | dCas12f1 variant 1(D354A) (SEQ ID NO: 15) | TadA-eTadA1 (SEQ ID NO: 274) | 266 | 201 | 291 | v4.1 |

Here, the protospacer sequences used to design the spacer of the guide RNA are Target-1, Target-2, and Target-5 to Target-10.

A vector capable of expressing the engineered Cas12f1 protein and a vector capable of expressing the guide RNA, according to the table above, were constructed according to Experimental Example 1.6. The constructed vectors were transfected into HEK 293T cells according to Experimental Example 1.7, and genomic DNA was isolated therefrom 3 days later.

Then, base editing efficiency for target sequence sites, according to Table 9, in the genomic DNA was analyzed according to Experimental Example 1.9. Specifically, for the 10 target sites shown in Table te3-01, it was analyzed whether replacement of any base with another base occurred at respective nucleotides ranging from N1 to N20 (the 1st nucleotide to the 20th nucleotide following the PAM sequence) within the guide RNA-binding region of the 5'-end target site which is the expected editing window range.

As a result, as illustrated in FIGS. 51 to 57, it was identified that the engineered Cas12f1 system, which comprises the dCas12f1-base editing fusion protein comprising the adenosine deaminase, effectively edited adenine (A) to guanine (G) within the editing window range. In particular, the best efficiency was observed for replacement of adenine bases at positions A3 and A4 with guanine bases.

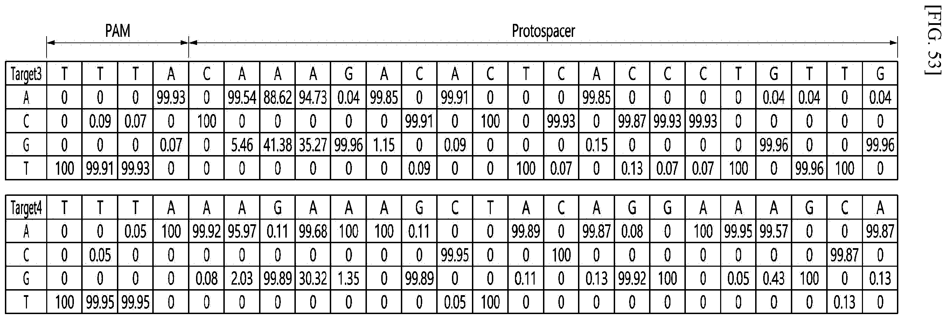

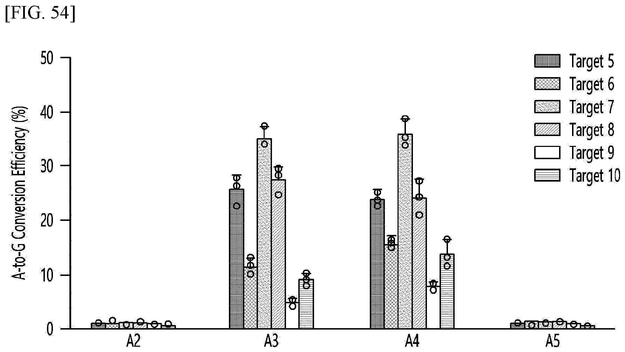

For dCas12f1-ABE-N1 containing dCas12f1 (D326A), it was identified that the frequency of editing the A2 base of Target-1 to guanine was about 15% (FIG. 53), and the frequencies of editing the A3 and A4 bases of Target-3 to guanines were about 41% and about 35%, respectively (FIG. 53). In addition, it was identified that the A3 and A4 bases of Target-5, Target-7, and Target-8 were simultaneously edited to guanines at a frequency of 30% to 40% (FIG. 54).

For dCas12f1.v1-ABE-C2 containing dCas12f1 variant type 1 (D354A), the frequency of editing the A4 base of Target-1 to guanine was about 14% (FIGS. 55 and 56). In addition, the frequencies of editing the A3 and A4 bases of Target-3 to guanines were identified to be about 47% and about 40%, respectively, and the frequency of editing the A4 base of Target-4 with guanine was about 37% that is very high (FIG. 57).

### Experimental Example 3.3. Base editing effect 2 of adenosine deaminase fusion protein

Using the method disclosed in Experimental Example 3.2, an effect of each dCas12f1-base editing fusion protein according to Table 10 was identified:

**[Table 10] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| D326A | [dCas12f1]-[Linker]-[Deaminase]-[NLS] | dCas12f1(D326A) (SEQ ID NO: 2) | TadA-eTadA1 (SEQ ID NO: 274) | 266 | 201 | 287 | v4.1 |
| E422A | [dCas12f1]-[Linker]-[Deaminase]-[NLS] | dCas 12f1 (E422A) (SEQ ID NO: 3) | TadA-eTadA1 (SEQ ID NO: 274) | 266 | 201 | 292 | v4.1 |
| R490A | [dCas12fl]-[Linker]-[Deaminase]-[NLS] | dCas12f1(R490A) (SEQ ID NO: 4) | TadA-eTadA1 (SEQ ID NO: 274) | 266 | 201 | 293 | v4.1 |
| D510A | [dCas12f1]-[Linker]-[Deaminase][NLS] | dCas12f1(D510A) (SEQ ID NO: 5) | TadA-eTadA1 (SEQ ID NO: 274) | 266 | 201 | 294 | v4.1 |
| D354A | [dCas12f1]-[Linker]-[Deaminase]-[NLS] | dCas12f1 variant 1(D354A) (SEQ ID NO: 15) | TadA-eTadA1 (SEQ ID NO: 274) | 266 | 201 | 291 | v4.1 |
| E450A | [dCas12f1]-[Linker]-[Deaminase]-[NLS] | dCas12f1 variant 1(D354A) (SEQ ID NO: 16) | TadA-eTadA1 (SEQ ID NO: 274) | 266 | 201 | 295 | v4.1 |
| R518A | [dCas12f1]-[Linker]-[Deaminase]-[NLS] | dCas12f1 variant 1(0354A) (SEQ ID NO: 17) | TadA-eTadA1 (SEQ ID NO: 274) | 266 | 201 | 296 | v4.1 |
| D538A | [dCas12f1]-[Linker]-[Deaminase]-[NLS] | dCas12f1 variant 1(D354A) (SEQ ID NO: 18) | TadA-eTadA1 (SEQ ID NO: 274) | 266 | 201 | 297 | v4.1 |

Here, the protospacer sequence used to design the spacer of the guide RNA is Target-3.

From the experimental results, as illustrated in FIGS. 6 and 7, dCas12f1 (D326A), dCas12f1 (E422A), dCas12f1 (R490A), and dCas12f1 (D510A) exhibited base editing rates of about 9%, about 13%, about 3%, and about 5%, respectively, for 3A of Target-3, and exhibited base editing rates of about 4%, about 5%, about 2%, and about 7%, respectively, for 4A of Target-3.

dTnpB (D354A), dTnpB (E450A), dTnpB (R518A), and dTnpB (D538A) exhibited base editing rates of about 10%, about 14%, about 11%, and about 17%, respectively, for 2A of Target-3, exhibited base editing rates of about 22%, about 25%, about 17%, and about 33%, respectively, for 3A of Target-3, and exhibited base editing rates of about 20%, about 22%, about 15%, and about 26%, respectively, for 4A of Target-3.

As a result, it was identified that dTnpB has better base editing efficiency than dCas12f1.

### Experimental Example 3.4. Base editing effect 3 of adenosine deaminase fusion protein

In order to identify whether the base editing efficiency of the dCas12f1-base editing system is associated with the type of promoter included in the expression vector, the base editing efficiency of each base editing system according to Table 11 was checked with reference to Experimental Example 3.2.

**[Table 11] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| v4.0 CMV | [dCas12f1]-[Linker]-[Deaminase]-[NLS] | dCas12f1 variant 1(D354A) (SEQ ID NO: 15) | TadA-eTadA1 (SEQ ID NO: 274) | 266 | 201 | 291 | v4.0 |
| v4.0 CBA | [dCas12f1]-[Linker]-[Deaminase]-[NLS] | dCas12f1 variant 1(D354A) (SEQ ID NO: 15) | TadA-eTadA1 (SEQ ID NO: 274) | 266 | 201 | 291 | v4.0 |
| v4.1 CMV | [dCas12f1]-[Linker]-[Deaminase]-[NLS] | dCas12f1 variant 1(0354A) (SEQ ID NO: 15) | TadA-eTadA1 (SEQ ID NO: 274) | 266 | 201 | 291 | v4.1 |
| v4.1 CBA | [dCas12f1]-[Linker]-[Deaminase]-[NLS] | dCas12f1 variant 1(D354A) (SEQ ID NO: 15) | TadA-eTadA1 (SEQ ID NO: 274) | 266 | 201 | 291 | v4.1 |

Here, the protospacer sequences used to design the spacer of the guide RNA are Target-1 and Target-3.

Here, for the same dCas12f1-base editing protein, the experiments were performed using different promoters for the expression vector.

As a result, both the CBA promoter and the CMV promoter used showed excellent base editing efficiency, and in particular, the CBV promoter showed the best activity in a case of being combined with gRNA Ver4.1 (FIG. 8).

### Experimental Example 3.5. Base editing effect 4 of adenosine deaminase fusion protein

In order to identify whether the base editing efficiency of the dCas12f1-base editing system is associated with the length of the linker that links the base editing domain to the dead Cas12f1 protein, base editing efficiency was checked for each base editing system according to Tables 12 to 14 with reference to Experimental Example 3.2.

**[Table 12] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| 10aa (Fig. 9) | [dCas12f1]-[Linker]-[Deaminase]-[NLS] | dCas12f1 variant 1(D354A) (SEQ ID NO: 15) | TadA-eTadA1 (SEQ ID NO: 274) | **260** | 201 | 418 | v4.1 |
| 14aa (Fig. 9) | [dCas12f1]-[Linker]-[Deaminase]-[NLS] | dCas12f1 variant 1(D354A) (SEQ ID NO: 15) | TadA-eTadA1 (SEQ ID NO: 274) | **261** | 201 | 419 | v4.1 |
| 16aa (Fig. 9) | [dCas12f1]-[Linker]-[Deaminase]-[NLS] | dCas12f1 variant 1(D354A) (SEQ ID NO: 15) | TadA-eTadA1 (SEQ ID NO: 274) | **262** | 201 | 420 | v4.1 |
| 20aa (Fig. 9) | [dCas12f1]-[Linker]-[Deaminase]-[NLS] | dCas12f1 variant 1(D354A) (SEQ ID NO: 15) | TadA-eTadA1 (SEQ ID NO: 274) | **263** | 201 | 421 | v4.1 |
| 24aa (Fig. 9) | [dCas12f1]-[Linker]-[Deaminase]-[NLS] | dCas12f1 variant 1(D354A) (SEQ ID NO: 15) | TadA-eTadA1 (SEQ ID NO: 274) | **264** | 201 | 422 | v4.1 |
| 28aa (Fig. 9) | [dCas12f1]-[Linker]-[Deaminase]-[NLS] | dCas12f1 variant 1(0354A) (SEQ ID NO: 15) | TadA-eTadA1 (SEQ ID NO: 274) | **265** | 201 | 423 | v4.1 |
| 32aa (Fig. 9) | [dCas12f1]-[Linker]-[Deaminase]-[NLS] | dCas12f1 variant 1(0354A) (SEQ ID NO: 15) | TadA-eTadA1 (SEQ ID NO: 274) | **266** | 201 | 291 | v4.1 |
| 36aa (Fig. 9) | [dCas12f1]-[Linker]-[Deaminase]-[NLS] | dCas12f1 variant 1(0354A) (SEQ ID NO: 15) | TadA-eTadA1 (SEQ ID NO: 274) | **267** | 201 | 424 | v4.1 |
| 40aa (Fig. 9) | [dCas12f1]-[Linker]-[Deaminase]-[NLS] | dCas12f1 variant 1(D354A) (SEQ ID NO: 15) | TadA-eTadA1 (SEQ ID NO: 274) | **268** | 201 | 425 | v4.1 |

**[Table 13] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| 10aa (Fig. 10) | [dCas12f1]-[Linker]-[Deaminase]-[NLS] | dCas12f1 variant 1(D354A) (SEQ ID NO: 15) | TadA-eTadA3 (SEQ ID NO: 276) | **260** | 201 | 426 | v4.1 |
| 14aa (Fig. 10) | [dCas12f1]-[Linker]-[Deaminase]-[NLS] | dCas12f1 variant 1(D354A) (SEQ ID NO: 15) | TadA-eTadA3 (SEQ ID NO: 276) | **261** | 201 | 427 | v4.1 |
| 16aa (Fig. 10) | [dCas12fl]-[Linker]-[Deaminase]-[NLS] | dCas12f1 variant 1(D354A) (SEQ ID NO: 15) | TadA-eTadA3 (SEQ ID NO: 276) | **262** | 201 | 428 | v4.1 |
| 20aa (Fig. 10) | [dCas12f1]-[Linker]-[Deaminase]-[NLS] | dCas12f1 variant 1(D354A) (SEQ ID NO: 15) | TadA-eTadA3 (SEQ ID NO: 276) | **263** | 201 | 429 | v4.1 |
| 24aa (Fig. 10) | [dCas12f1]-[Linker]-[Deaminase]-[NLS] | dCas12f1 variant 1(D354A) (SEQ ID NO: 15) | TadA-eTadA3 (SEQ ID NO: 276) | **264** | 201 | 430 | v4.1 |
| 28aa (Fig. 10) | [dCas12f1]-[Linker]-[Deaminase]-[NLS] | dCas12f1 variant 1(D354A) (SEQ ID NO: 15) | TadA-eTadA3 (SEQ ID NO: 276) | **265** | 201 | 431 | v4.1 |
| 32aa (Fig. 10) | [dCas12f1]-[Linker]-[Deaminase]-[NLS] | dCas12f1 variant 1(0354A) (SEQ ID NO: 15) | TadA-eTadA3 (SEQ ID NO: 276) | **266** | 201 | 299 | v4.1 |
| 36aa (Fig. 10) | [dCas12f1]-[Linker]-[Deaminase]-[NLS] | dCas12f1 variant 1(0354A) (SEQ ID NO: 15) | TadA-eTadA3 (SEQ ID NO: 276) | **267** | 201 | 432 | v4.1 |
| 40aa (Fig. 10) | [dCas12f1]-[Linker]-[Deaminase] -[NLS] | dCas12f1 variant 1(D354A) (SEQ ID NO: 15) | TadA-eTadA3 (SEQ ID NO: 276) | **268** | 201 | 433 | v4.1 |

**[Table 14] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| 10aa (Fig. 11) | [dCas12f1]-[Linker]-[Deaminase]-[NLS] | dCas12f1(D326A) (SEQ ID NO: 2) | TadA-eTadA1 (SEQ ID NO: 274) | **260** | 201 | 434 | v4.1 |
| 14aa (Fig. 11) | [dCas12f1]-[Linker]-[Deaminase]-[NLS] | dCas12f1(D326A) (SEQ ID NO: 2) | TadA-eTadA1 (SEQ ID NO: 274) | **261** | 201 | 435 | v4.1 |
| 16aa (Fig. 11) | [dCas12f1]-[Linker]-[Deaminase]-[NLS] | dCas12f1(D326A) (SEQ ID NO: 2) | TadA-eTadA1 (SEQ ID NO: 274) | **262** | 201 | 436 | v4.1 |
| 20aa (Fig. 11) | [dCas12f1]-[Linker]-[Deaminase]-[NLS] | dCas12f1(0326A) (SEQ ID NO: 2) | TadA-eTadA1 (SEQ ID NO: 274) | **263** | 201 | 437 | v4.1 |
| 24aa (Fig. 11) | [dCas12f1]-[Linker]-[Deaminase]-[NLS] | dCas12f1(D326A) (SEQ ID NO: 2) | TadA-eTadA1 (SEQ ID NO: 274) | **264** | 201 | 438 | v4.1 |
| 28aa (Fig. 11) | [dCas12f1]-[Linker]-[Deaminase]-[NLS] | dCas12f1(D326A) (SEQ ID NO: 2) | TadA-eTadA1 (SEQ ID NO: 274) | **265** | 201 | 439 | v4.1 |
| 32aa (Fig. 11) | [dCas12f1]-[Linker]-[Deaminase]-[NLS] | dCas12f1(D326A) (SEQ ID NO: 2) | TadA-eTadA1 (SEQ ID NO: 274) | **266** | 201 | 287 | v4.1 |
| 36aa (Fig. 11) | [dCas12f1]-[Linker]-[Deaminase]-[NLS] | dCas12f1(D326A) (SEQ ID NO: 2) | TadA-eTadA1 (SEQ ID NO: 274) | **267** | 201 | 440 | v4.1 |
| 40aa (Fig. 11) | [dCas12f1]-[Linker]-[Deaminase]-[NLS] | dCas12f1(D326A) (SEQ ID NO: 2) | TadA-eTadA1 (SEQ ID NO: 274) | **268** | 201 | 441 | v4.1 |

Here, the protospacer sequence used to design the spacer of the guide RNA is Target-3.

Referring to the experimental results, all linkers showed similar activity even in a case where their length varied from 10 to 40 amino acids (FIGS. 9 to 11). Accordingly, it was found that the length of the linker, which links the dead Cas12f1 protein to the base editing domain, did not have a significant effect on base editing efficiency.

### Experimental Example 3.6. Base editing effect 5 of adenosine deaminase fusion protein

In order to investigate base editing efficiency of various dead Cas12f1 variant-based dCas12f1-base editing proteins, base editing efficiency was checked for each base editing system according to Table 15 with reference to Experimental Example 3.2.

Here, the protospacer sequence used to design the spacer of the guide RNA is Target-3.

**[Table 15] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| D510A | [dCas12f1]-[Linker]-[Deaminase]-[NLS] | dCast2f1(D510A) (SEQ ID NO: 5) | TadA-eTadA1 (SEQ ID NO: 274) | 266 | 201 | 294 | v4.1 |
| DS10A,I131W | [dCas12f1]-[Linker]-[Deaminase] -[NLS] | dCas12f1(D510A, I131W) (SEQ ID NO: 6) | TadA-eTadA1 (SEQ ID NO: 274) | 266 | 201 | 300 | v4.1 |
| D510A, S136Y | [dCas12f1]-[Linker]-[Deaminase]-[NLS] | dCas12f1(D510A, S136Y) (SEQ ID NO: 7) | TadA-eTadA1 (SEQ ID NO: 274) | 266 | 201 | 301 | v4.1 |
| D510A, I131W, S136Y | [dCas12f1]-[Linker]-[Deaminase]-[NLS] | dCas12f1 (D510A, 1131W, S136Y) (SEQ ID NO: 8) | TadA-eTadA1 (SEQ ID NO: 274) | 266 | 201 | 302 | v4.1 |
| D538A | [dCas12f1]-[Linker]-[Deaminase]-[NLS] | dCas12f1 variant 1(D538A) (SEQ ID NO: 18) | TadA-eTadA1 (SEQ ID NO: 274) | 266 | 201 | 297 | v4.1 |
| D538A, I159W | [dCas12f1]-[Linker]-[Deaminase] -[NLS] | dCas12f1 variant 1 (D538A, I159W) (SEQ ID NO: 19) | TadA-eTadA1 (SEQ ID NO: 274) | 266 | 201 | 303 | v4.1 |
| D538A, S164Y | [dCas12f1]-[Linker]-[Deaminase]-[NLS] | dCas12f1 variant 1 (D438A, S164Y) (SEQ ID NO: 20) | TadA-eTadA1 (SEQ ID NO: 274) | 266 | 201 | 304 | v4.1 |
| D538A, I159W, S164Y | [dCas12f1]-[Linker]-[Deaminase]-[NLS] | dCas12f1 variant 1 (D538A, I159W, S164Y) (SEQ ID NO: 21) | TadA-eTadA1 (SEQ ID NO: 274) | 266 | 201 | 305 | v4.1 |

Referring to the experimental results, the tested variants showed similar base editing rates for 3A and 4A of Target-3. The I159W and/or S164Y variants, which were expected to affect a base editing window, showed significantly increased base editing rates for 6A of Target-3 as compared with the wild type (FIGS. 12 and 13).

### Experimental Example 3.7. Base editing effect 6 of adenosine deaminase fusion protein

In order to investigate base editing efficiency of various dead Cas12f1 variant-based dCas12f1-base editing proteins, base editing efficiency was checked for each base editing system according to Tables 16 and 17 with reference to Experimental Example 3.2.

**[Table 16] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| TadAeTadA1-dCas12f1 | [Deaminase]-[Linker]-[dCas12f1]-[NLS] | dCas12f1(D326A) (SEQ ID NO: 2) | TadA-eTadA1 (SEQ ID NO: 274) | 266 | 201 | 284 | v4.1 |
| eTadA1TadA-dCas12f1 | [Deaminase]-[Linker]-[dCas12f1]-[NLS] | dCas12f1(D326A) (SEQ ID NO: 2) | eTadA1-TadA1 (SEQ ID NO: 275) | 266 | 201 | 285 | v4.1 |
| dCas12f1-eTadAlTadA | [dCas12f1]-[Linker]-[Deaminase]-[NLS] | dCas12f1(D326A) (SEQ ID NO: 2) | eTadA-TadAl (SEQ ID NO: 275) | 266 | 201 | 286 | v4.1 |
| dCas12f1-TadAeTadA1 | [dCas12f1]-[Linker]-[Deaminase]-[NLS] | dCas12f1(D326A) (SEQ ID NO: 2) | TadA-eTadA1 (SEQ ID NO: 274) | 266 | 201 | 287 | v4.1 |
| dCas12f1(I159W)-TadAeTadA1 | [dCas12f1]-[Linker]-[Deaminase]-[NLS] | dCas12f1(D510A, I131W) (SEQ ID NO: 6) | TadA-eTadA1 (SEQ ID NO: 274) | 266 | 201 | 300 | v4.1 |
| dCas12f1-TadAeTadA2 | [dCas12f1]-[Linker]-[Deaminase]-[NLS] | dCas12f1(D326A) (SEQ ID NO: 2) | TadA-eTadA2 (SEQ ID NO: 277) | 266 | 201 | 308 | v4.1 |
| dCas12f1-TadAeTadA3 | [dCas12f1]-[Linker]-[Deaminase]-[NLS] | dCas12f1(D326A) (SEQ ID NO: 2) | TadA-eTadA3 (SEQ ID NO: 276) | 266 | 201 | 313 | v4.1 |

**[Table 17] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| TadAeTadA1-dTnpB | [Deaminase]-[Linker]-[dCas12f1]-[NLS] | dCas12f1 variant 1(D354A) (SEQ ID NO: 15) | TadA-eTadA1 (SEQ ID NO: 274) | 266 | 201 | 288 | v4.1 |
| eTadAlTadA-dTnpB | [Deaminase]-[Linker]-[dCas12f1] -[NLS] | dCas12f1 variant 1(D354A) (SEQ ID NO: 15) | eTadA1-TadA1 (SEQ ID NO: 275) | 266 | 201 | 289 | v4.1 |
| dTnpB-eTadA1TadA | [dCas12f1]-[Linker]-[Deaminase]-[NLS] | dCas12f1 variant 1(D354A) (SEQ ID NO: 15) | eTadA-TadA1 (SEQ ID NO: 275) | 266 | 201 | 290 | v4.1 |
| dTnpB-TadAeTadA1 | [dCas12f1]-[Linker]-[Deaminase]-[NLS] | dCas12f1 variant 1(D354A) (SEQ ID NO: 15) | TadA-eTadA1 (SEQ ID NO: 274) | 266 | 201 | 291 | v4.1 |
| dTnpB(1159W)-TadAeTadA1 | [dCas12f1]-[Linker]-[Deaminase]-[NLS] | dCas12f1 variant 1 (D538A, I159W) (SEQ ID NO: 19) | TadA-eTadA1 (SEQ ID NO: 274) | 266 | 201 | 303 | v4.1 |
| dTnpB-TadAeTadA2 | [dCas12f1]-[Linker]-[Deaminase]-[NLS] | dCas12f1 variant 1(D354A) (SEQ ID NO: 15) | TadA-eTadA2 (SEQ ID NO: 277) | 266 | 201 | 316 | v4.1 |
| dTnpB(I159W)-TadAeTadA3 | [dCas12f1]-[Linker]-[Deaminase]-[NLS] | dCas12f1 variant 1 (D538A,1159W) (SEQ ID NO: 19) | TadA-eTadA3 (SEQ ID NO: 276) | 266 | 201 | 442 | v4.1 |

Here, the protospacer sequence used to design the spacer of the guide RNA is Target-1.

From the experimental results, it was found that in a case of using various dead Cas12f1 variant-based dCas12f1-base editing proteins, the base editing window extended from N2 to N18 for target sequence 1 (FIGS. 15 to 18).

### Experimental Example 3.8. Base editing effect 7 of adenosine deaminase fusion protein

In order to investigate base editing efficiency of the dCas12f1-base editing protein for various target sequences, base editing efficiency was checked, with reference to Experimental Example 3.2, for the base editing system that comprises the dCas12f1-base editing fusion protein of SEQ ID NO: 299 and the guide RNA of v4.0 or v4.1.

Here, the protospacer sequences used to design the spacer of the guide RNA are as shown in Tables 7 and 8.

In addition, as an embodiment, the base editing system using TadAeTadA3 was verified on various target sequences. As a result, it was identified that the available base editing window ranged from A2 to A8 and/or A15 to A20 for 25 different target sequences (FIGS. 19 to 24).

### Experimental Example 3.9. Base editing effect 8 of adenosine deaminase fusion protein

In order to investigate base editing efficiency of the dCas12f1-base editing protein depending on the type of base editing domain included therein, base editing efficiency was checked for each base editing system according to Tables 18 and 19 with reference to Experimental Example 3.2.

**[Table 18] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| TadAeTadA1 (Fig. 25 to Fig. 27) | [dCas12f1]-[Linker]-[Deaminase]-[NLS] | dCas12f1(D326A) (SEQ ID NO: 2) | TadAeTadA1 (SEQ ID NO: 274) | 266 | 201 | 287 | v3.0, v4.0, v4.1 |
| dTadAeTadA1 (Fig. 25 to Fig. 27) | [dCas12f1]-[Linker]-[Deaminase]-[NLS] | dCas12f1(D326A) (SEQ ID NO: 2) | dTadAeTadA1 (SEQ ID NO: 278) | 266 | 201 | 306 | v3.0, v4.0, v4.1 |
| TadAdeTadA1 (Fig. 25 to Fig. 27) | [dCas12f1]-[Linker]-[Deaminase]-[NLS] | dCas12f1(D326A) (SEQ ID NO: 2) | TadAdeTadA1 (SEQ ID NO: 279) | 266 | 201 | 307 | v3.0, v4.0, v4.1 |
| TadAeTadA2 (Fig. 25 to Fig. 27) | [dCas12f1]-[Linker]-[Deaminase]-[NLS] | dCas12f1(D326A) (SEQ ID NO: 2) | TadAeTadA2 (SEQ ID NO: 277) | 266 | 201 | 308 | v3.0, v4.0, v4.1 |
| eTadA2 (Fig. 25 to Fig. 27) | [dCas12f1]-[Linker]-[Deaminase]-[NLS] | dCas12f1(D326A) (SEQ ID NO: 2) | eTadA2 (SEQ ID NO: 245) | 266 | 201 | 309 | v3.0, v4.0, v4.1 |
| eTadA4 (Fig. 25 to Fig. 27) | [dCas12f1]-[Linkerj-[Deaminase]-[NLS] | dCas12f1(D326A) (SEQ ID NO: 2) | eTadA4 (SEQ ID NO: 247) | 266 | 201 | 310 | v3.0, v4.0, v4.1 |
| eTadA5 (Fig. 25 to Fig. 27) | [dCas12f1]-[Linker]-[Deaminase]-[NLS] | dCas12f1(D326A) (SEQ ID NO: 2) | eTadA5 (SEQ ID NO: 248) | 266 | 201 | 311 | v3.0, v4.0, v4.1 |
| eTadA6 (Fig. 25 to Fig. 27) | [dCas12f1]-[Linker]-[Deaminase]-[NLS] | dCas12f1(D326A) (SEQ ID NO: 2) | eTadA6 (SEQ ID NO: 249) | 266 | 201 | 312 | v3.0, v4.0, v4.1 |
| TadAeTadA3 (Fig. 25 to Fig. 27) | [dCas12f1]-[Linker]-[Deaminase]-[NLS] | dCas12f1(D326A) (SEQ ID NO: 2) | TadAeTadA3 (SEQ ID NO: 276) | 266 | 201 | 313 | v3.0, v4.0, v4.1 |

**[Table 19] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| TadAeTadA1 (Fig. 28 to Fig. 30) | [dCas12f1]-[Linker]-[Deaminase]-[NLS] | dCas12f1 variant 1(D354A) (SEQ ID NO: 15) | TadAeTadA1 (SEQ ID NO: 274) | 266 | 201 | 291 | v3.0, v4.0, v4.1 |
| dTadAeTadA1 (Fig. 28 to Fig. 30) | [dCas12f1]-[Linker]-[Deaminase]-[NLS] | dCas12f1 variant 1(D354A) (SEQ ID NO: 15) | dTadAeTadA1 (SEQ ID NO: 278) | 266 | 201 | 314 | v3.0, v4.0, v4.1 |
| TadAdeTadA1 (Fig. 28 to Fig. 30) | [dCas12f1]-[Linker]-[Deaminase]-[NLS] | dCas12f1 variant 1(D354A) (SEQ ID NO: 15) | TadAdeTadA1 (SEQ ID NO: 279) | 266 | 201 | 315 | v3.0, v4.0, v4.1 |
| TadAeTadA2 (Fig. 28 to Fig. 30) | [dCas12f1]-[Linker]-[Deaminase]-[NLS] | dCas12f1 variant 1(D354A) (SEQ ID NO: 15) | TadAeTadA2 (SEQ ID NO: 277) | 266 | 201 | 316 | v3.0, v4.0, v4.1 |
| eTadA2 (Fig. 28 to Fig. 30) | [dCas12f1]-[Linker]-[Deaminase]-[NLS] | dCas12f1 variant 1(D354A) (SEQ ID NO: 15) | eTadA2 (SEQ ID NO: 245) | 266 | 201 | 317 | v3.0, v4.0, v4.1 |
| eTadA4 (Fig. 28 to Fig. 30) | [dCas12f1]-[Linker]-[Deaminase] -[NLS] | dCas12f1 variant 1(D354A) (SEQ ID NO: 15) | eTadA4 (SEQ ID NO: 247) | 266 | 201 | 318 | v3.0, v4.0, v4.1 |
| eTadA5 (Fig. 28 to Fig. 30) | [dCas12f1]-[Linker]-[Deaminase]-[NLS] | dCas12f1 variant 1(0354A) (SEQ ID NO: 15) | eTadAS (SEQ ID NO: 248) | 266 | 201 | 319 | v3.0, v4.0, v4.1 |
| eTadA6 (Fig. 28 to Fig. 30) | [dCas12f1]-[Linker]-[Deaminase]-[NLS] | dCas12f1 variant 1(D354A) (SEQ ID NO: 15) | eTadA6 (SEQ ID NO: 249) | 266 | 201 | 320 | v3.0, v4.0, v4.1 |
| TadAeTadA3 (Fig. 28 to Fig. 30) | [dCas12f1]-[linker]-[Deaminase]-[NLS] | dCas12f1 variant 1(D354A) (SEQ ID NO: 15) | TadAeTadA3 (SEQ ID NO: 276) | 266 | 201 | 299 | v3.0, v4.0, v4.1 |

Here, the protospacer sequence used to design the spacer of the guide RNA is Target-3.

From the experimental results, as illustrated in FIGS. 25 to 30, TadA-eTadA2, eTadA2, and TadA-eTadA1 showed excellent base editing efficiency together with dCas12f1 or dTnpB. The results indicate that Tad is an important factor affecting base editing efficiency.

### Experimental Example 3.10. Base editing effect 9 of adenosine deaminase fusion protein

In order to investigate base editing efficiency of the dCas12f1-base editing protein depending on the type of Cas12f1 variant protein, which is the basis of the dead Cas12f1 protein included in the dCas12f1-base editing protein, for each base editing system according to Table 20, base editing efficiency thereof was checked with reference to Experimental Example 3.2.

**[Table 20] dCas12f1-base editing fusion protein comprising adenosine deaminase**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| dead Cas12f1 | [dCas12f1]-[Linker]-[Deaminase]-[NLS] | dCas12f1(D326A) (SEQ ID NO: 2) | TadA-eTadA1 (SEQ ID NO: 274) | 266 | 201 | 287 | v4.0, v4.1 |
| dead Cas12f1 variant 1 | [dCas12f1]-[Linker]-[Deaminase]-[NLS] | dCas12f1 variant 1(D354A) (SEQ ID NO: 15) | TadA-eTadA1 (SEQ ID NO: 274) | 266 | 201 | 291 | v4.0, v4.1 |
| dead Cas12f1 variant 2 | [dCas12f1]-[Linker]-[Deaminase]-[NLS] | dCas12f1 variant 2(D354A) (SEQ ID NO: 26) | TadA-eTadA1 (SEQ ID NO: 274) | 266 | 201 | 321 | v4.0, v4.1 |
| dead Cas12f1 variant 3 | [dCas!2f1]-[Linker]-[Deaminase]-[NLS] | dCas12f1 variant 3(D352A) (SEQ ID NO: 31) | TadA-eTadA1 (SEQ ID NO: 274) | 266 | 201 | 322 | v4.0, v4.1 |
| deadCas12f1 variant 4 | [dCas12f1]-[Linker]-[Deaminase]-[NLS] | dCas12f1 variant 4(D352A) (SEQ ID NO: 36) | TadA-eTadA1 (SEQ ID NO: 274) | 266 | 201 | 323 | v4.0, v4.1 |

The base editing effect of each of the thus produced hypercompact base editing systems is shown in Table 21.

**[Table 21] Base editing effect depending on type of base editing system**

| Label | Target-1 | | | | Target-3 | | | | |
|---|---|---|---|---|---|---|---|---|---|
| | 2A | 4A | 6A | 8A | 2A | 3A | 4A | 6A | 8A |
| dead Cas12f1 + v4.0 | 2.22 | 4.74 | 0.6 | 0.73 | 2.4 | 19.92 | 13.49 | 2.95 | 0.57 |
| dead Cas12f1 + v4.1 | 2.08 | 12.12 | 0.69 | 0.59 | 3.35 | 30.29 | 18.29 | 2.95 | 0.6 |
| dead Cas12f1 variant 1 + v4.0 | 1.2 | 22.36 | 0.6 | 0.83 | 5.33 | 56.88 | 34.52 | 3.73 | 0.53 |
| dead Cas12f1 variant 1 + v4.1 | 2.47 | 23.86 | 0.68 | 0.73 | 5.69 | 59.29 | 26.71 | 4.08 | 0.55 |
| dead Cas12f1 variant 2 + v4.0 | 2.57 | 14.58 | 0.57 | 0.56 | 4.68 | 38.92 | 25.48 | 3.64 | 0.52 |
| dead Cas12f1 variant 2 + v4.1 | 2.87 | 32.98 | 0.64 | 0.56 | 5.59 | 53.2 | 27.88 | 3.49 | 0.56 |
| dead Cas12f1 variant 3 + v4.0 | 2.63 | 10.98 | 0.73 | 0.76 | 3.75 | 29.85 | 19.67 | 3.25 | 0.55 |
| dead Cas12f1 variant 3 + v4.1 | 2.52 | 30.44 | 0.69 | 0.66 | 3.32 | 40.15 | 21.24 | 3.4 | 0.48 |
| dead Cas12f1 variant 4 + v4.0 | 2.37 | 16.3 | 0.51 | 0.64 | 3.96 | 38.87 | 23.11 | 3.19 | 0.59 |
| dead Cas12f1 variant 4 + v4.1 | 3.03 | 30.12 | 0.66 | 0.64 | 3.15 | 46.81 | 22.08 | 3.63 | 0.53 |
| Control | 0.49 | 0.37 | 0.4 | 0.6 | 0.3 | 0.36 | 0.39 | 0.5 | 0.3 |

Here, the protospacer sequences used to design the spacer of the guide RNA are Target-1 and Target-3.

From the experimental results, it was found that the base editing efficiency of the dCas12f1-base editing protein that comprises the Cas12f1 variant protein-based dead Cas12f1 protein was overall very high as compared with the base editing efficiency of the dCas12f1-base editing protein that comprises the wild-type Cas12f1 protein-based dead Cas12f1, and in particular, Cas12f1 variant 1 of SEQ ID NO: 15 showed high base editing efficiency.

### Experimental Example 3.11. Base editing effect 10 of adenosine deaminase fusion protein

In order to compare base editing efficiency between various dead Cas12f1 variant-based dCas12f1-base editing proteins and the existing Cas9-based base editing systems, base editing efficiency was checked, with reference to Experimental Example 3.2, for each base editing system according to Table 22 and the Cas9-based ABEs 7.10, 8e, and 9 as comparative examples.

**[Table 22]**

| Label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| ABEMINI | [Deaminase]-[Linker]-[dCas12f1]-[NLS] | dCas12f1(D326A) (SEQ ID NO: 2) | TadA-eTadA1 (SEQ ID NO: 274) | 266 | 201 | 284 | v4.1 |
| ABE-C3 | [dCas12f1]-[Linker]-[Deaminase]-[NLS] | dCas12f1 variant 1(D354A) (SEQ ID NO: 15) | TadA-eTadA3 (SEQ ID NO: 276) | 266 | 201 | 299 | v4.1 |

From the experimental results, as illustrated in FIG. 31, dead Cas12f1 variant 1-based ABE-C3 (SEQ ID NO: 299) showed an excellent base editing rate at positions A3 and A4 as compared with dead Cas12f1-based ABEMINI (SEQ ID NO: 284) or Cas9-based ABEs 7.10, 8e, and 9. The results indicate that the dead Cas12f1 variant 1-based base editing system shows the best ABE activity.

### Experimental Example 3.12. Base editing effect 11 of adenosine deaminase fusion protein

Comparison was performed in terms of base editing activity between the CRISPR base editing system disclosed herein and the adenine base editor miniABEmax having a size that allows delivery by an existing AAV vector.

For this purpose, an AAV vector that comprises a nucleic acid encoding the dCas12f1-base editing protein (ABE-C2) according to SEQ ID NO: 287, an AAV vector that comprises a nucleic acid encoding the ABE-C2 and a guide RNA (ABE-C2+sgRNA), and an AAV vector that comprises an auxiliary target sequence (Auxiliary) polynucleotide in addition to the ABE-C2+sgRNA were constructed.

In addition, for comparison in terms of intracelluar base editing efficiency with the hypercompact base editing system according to the present disclosure, miniABEmax, which is an existing spCas9n-based adenine base editor AAV vector, was prepared.

The prepared vectors were transfected into HEK 293T cells, and 3 days later, genomic DNA was obtained from the transfected HEK 293T cells. The PCR products thereof were analyzed to determine whether the adenine (A) base was replaced with the guanine (G) base, using targeted deep sequencing.

As a result of the analysis, as illustrated in FIG. 32, the hypercompact base editing systems according to the present disclosure, ABE-C2+sgRNA and ABE-C2+sgRNA+Auxillary, showed base editing rates, at which the adenine (A) base is replaced with the guanine (G) base, of 30% and 40%, respectively.

On the other hand, the existing SpCas9n-based adenine base editor miniABEmax showed low adenine base editing specificity of 5% or lower.

From the above results, it was found that the hypercompact base editing system comprising dCas12f1 or a functional analog thereof according to the present disclosure not only had the advantage of a wide range of gene editing applications due to its small size as described above, but also exhibited significantly increased base editing efficiency as compared with the currently used base editor on which the most research had been conducted.

### Experimental Example 3.13. Base editing effect 1 of cytidine deaminase fusion protein

Experiments were performed on whether the dCas12f1-base editing fusion protein provided herein, which comprises cytidine deaminase as a base editing domain, has cytosine base editing activity. To identify this, human endogenous DNA target sites, in which a plurality of cytosines are contained within the editing window range of the cytosine base editing composition, were selected. Specifically, the protospacer sequences of the selected targets are the same as Target-2, Target-11, and Target-12 shown in Tables 5 and 6.

In order to identify the cytosine editing activity, base editing efficiency was checked for each cytidine base editing system according to Table 23.

**[Table 23] dCas12f1-base editing fusion protein comprising cytidine deaminase**

| Label | Structure (N to C) | dCas12f1 | Deaminase | UGI (SEQ ID) | Linker (SEQ ID) | NLS (SEQ ID) | SEQ ID NO (full sequence) | guide RNA |
|---|---|---|---|---|---|---|---|---|
| CBE_N1 | [Deaminase]-[Linker1]-[dCas12f1]-[Linker2]-[UGI]-[NLS] | dCas12f1(D326A) (SEQ ID NO: 2) | APOBEC1-TadA (SEQ ID NO: 280) | 461 | 266 | 201 | 325 | v4.1 |
| CBE_N2 | [Deaminase]-[Linker1]-[dCas12f1]-[Linker2]-[UGI]-[NLS] | dCas12f1(D326A) (SEQ ID NO: 2) | APOBEC1-eTadA (SEQ ID NO: 281) | 461 | 266 | 201 | 326 | v4.1 |
| CBE_C1 | [UGI]-[Linker1]-[dCas12f1]-[Linker2]-[Deaminase]-[NLS] | dCas12f1 variant 1(D354A) | eTadA-APOBEC1 (SEQ ID NO: 282) | 461 | 266 | 201 | 327 | v4.1 |
| CBE_C2 | [UGI]-[Linker1]-[dCas12f1]-[Linker2]-[Deaminase]-[NLS] | dCas12f1 variant 1 (D354A) | TadA-APOBEC1 (SEQ ID NO: 283) | 461 | 266 | 201 | 328 | v4.1 |

Here, the protospacer sequences used to design the spacer of the guide RNA are Target-2, Target-11, and Target-12.

A vector capable of expressing the engineered Cas12f1 protein and a vector capable of expressing the guide RNA, according to the table above, were constructed according to Experimental Example 1.6. The constructed vectors were transfected into HEK 293T cells according to Experimental Example 1.7, and genomic DNA was isolated therefrom 3 days later.

Then, base editing efficiency for target sequence sites, according to Table te3-01, in the genomic DNA was analyzed according to Experimental Example 1.9. Specifically, for the 10 target sites shown in Table 5, it was analyzed whether replacement of any base with another base occurred at respective nucleotides ranging from N1 to N20 (the 1st nucleotide to the 20th nucleotide following the PAM sequence) within the guide RNA-binding region of the 5'-end target site which is the expected editing window range.

From the experimental results, it was identified that the base editing system edited cytosine to thymine within the editing window range (FIG. 33).

For Target-2, the cytidine base editing systems designated as CBE-C1 and CBE-C2 had the highest frequency of replacing cytosine bases at positions C4 and C5 with thymine bases (FIG. 33). In addition, it was identified that the cytidine base editing system designated as CBE-C2 had frequencies of editing the C3 and C4 bases of Target-12 to thymines at about 20% and about 16%, respectively (FIG. 33).

From the above results, it was identified that the dCas12f1-base editing fusion protein comprising the cytidine deaminase, of the present disclosure, was capable of editing the two cytosines, which are located consecutively at C3 to C5 following the PAM sequence, that is, C3 and C4; or C4 and C5, to thymines at the same time.

From the above results, it was identified that the hypercompact base editing system for cytosine base editing, according to the present disclosure, exhibited a narrow editing window ranging from C3 to C5 and was capable of editing two consecutive cytosine bases within that range to thymine bases at the same time.

These results indicate that like the adenosine base editing system as described above, the cytidine base editing system is a new base editor from the viewpoint that it is also capable of solving the problem related to base editing that causes a silent mutation and has the advantage of overcoming the problem that for the stop codon of UAA, no base editing effect occurs since the stop codon is still UAG even in a case where the third adenine is edited to guanine.

### Experimental Example 3.14. Identification on whether CRISPR base editing complex generates unwanted indels

Meanwhile, the existing adenine base-editors (ABEs) or cytosine base-editors (CBEs), in which adenosine deaminase or cytidine deaminase is bound to dCas9 or nCas9 protein, were problematic in that they caused "unwanted indels," which are deletions or additions of bases within a target nucleic acid due to double-stranded DNA breaks therein, in addition to base editing.

Accordingly, it was identified whether the base editing system comprising the dCas12f1-base editing protein causes unwanted indels during the process of editing bases in a cell.

To identify this, generation of unwanted indels was checked for each base editing system of Table 24 according to Experimental Example 1.8. Here, the dCas9- or nCas9-based adenine base editors (ABEs) ABE7.10 and ABE8e, and the cytosine base editors (CBEs) BE4 and BE4-GAM were used as comparison groups.

**[Table 24] dCas12f1-base editing fusion protein**

| Label | Structure (N to C) | dCas12f1 | Deaminase | UGI (SEQ ID) | Linker (SEQ ID) | NLS (SEQ ID) | SEQ ID NO (full sequence) | guide RNA |
|---|---|---|---|---|---|---|---|---|
| ABE-C2 | [dCas12f1]-[Linker]-[Deaminase]-[NLS] | dCas12f1(D326A) (SEQ ID NO: 2) | TadA-eTadA1 (SEQ ID NO: 274) | - | 266 | 201 | 287 | v4.1 |
| CBE-C2 | [UGI]-[Linker1]-[dCas12f1]-[Linker2]-[Deaminase]-[NLS] | dCas12f1 variant 1(D354A) | TadA-APOBEC1 (SEQ ID NO: 283) | 461 | 266 | 201 | 328 | v4.1 |

As a result, as illustrated in FIG. 34, it was identified that both the adenine base editing system designated as ABE-C2 and the cytosine base editing system designated as CBE-C2 rarely generate unwanted indels.

However, it was identified that each of the existing dCas9 or nCas9 protein-based adenine base editors (ABEs) ABE7.10 and ABE8e and cytosine base editors (CBEs) BE4 and BE4-Gam generated 10-fold or higher unwanted indels as respectively compared with the ABE-C2 and CBE-C2 according to the present disclosure.

The above results are in sharp contrast to showing similar base editing activity such as editing adenine (A) to guanine (G) or cytosine (C) to thymine (T). This suggests that the base editing system disclosed herein is a very useful base editor in situations where only editing of a particular base is required without generating indels.

Furthermore, the above results have newly identified that the CRISPR base editing complex disclosed herein, which is a hypercompact base editing system, allows unlimited selection of a desired gene editing method such as indel, base editing, or prime editing, and allows such a method to be performed effectively without adverse effects.

### Experimental Example 3.15. Identification 1 of base editing effect of AAV vector encoding respective components of CRISPR base editing complex

In order to identify a base editing effect of the AAV vector, which encodes respective components of the CRISPR base editing complex provided herein, on a target gene in a cell, rAAV vector was transfected into cells to identify base editing efficiency thereof.

Specifically, to identify the base editing efficiency, a transfected cell line was produced using the following method, in which the cell line normally expresses eGFP and fails to express mRuby gene due to the stop codon in front of it. Plasmid vectors were constructed in which 1) a chicken β-actin (CBA) promoter, 5'- and 3'-terminal nuclear localization signal sequences, and a sequence encoding eGFP linked by a self-cleaving T2A peptide; 2) a polynucleotide for a target sequence (Target) and/or auxiliary target sequence (Auxiliary) which can be recognized by the codon-optimized hypercompact base editing structure according to the present disclosure and has a stop codon (TAG); and 3) a sequence of mRuby gene are operably linked. Each vector was transfected into HEK 293T cells. HEK 293T cells with the plasmid vector inserted into its chromosome normally express eGFP and fail to express the mRuby gene due to the stop codon in front of it.

The structure of each plasmid vector and the target sequence are shown in detail in FIGS. 35 and 36.

Following HEK 293T cells into which the plasmid vector is inserted, rAAV vector was constructed according to Experimental Example 1.6. The CRISPR base editing system loaded onto the vector is shown in Table 25.

**[Table 25] CRISPR base editing system loaded onto rAAVvector**

| label | Structure (N to C) | dCas12f1 | Deaminase | Linker (SEQ ID) | NLS (SEQ ID) | SEQ ID NO (full sequence) | guide RNA |
|---|---|---|---|---|---|---|---|
| ABE-C2 | [dCas12f1]-[Linker]-[Deaminase]-[NLS] | dCas12f1(D326A) (SEQ ID NO: 2) | TadA-eTadA1 (SEQ ID NO: 274) | 266 | 201 | 287 | v4.1 |
| TaRGET-ABE | [dCas12f1]-[Linker]-[Deaminase]-[NLS] | dCas12f1 variant 1(D354A) (SEQ ID NO: 15) | TadA-eTadA1 (SEQ ID NO: 274) | 266 | 201 | 291 | v4.1 |

Here, the spacer of the guide RNA was designed from the proto spacer sequences of Target-14, Target-15, Target-16, and Target-43, respectively. The structures of the vectors are shown in detail in FIGS. 35 and 36, respectively.

The thus constructed rAAV vector was transfected into the produced cells according to Experimental Example 1.7, and the expression of the mRuby gene was checked to identify whether the CRISPR base editing system performed its base editing function as intended. Here, spCas9-ABE split AAV as a control was transfected into the produced cells, and identification was performed.

The reason for checking the expression of mRuby is that the mRuby gene will be normally expressed because in a case where the vector is delivered into the transfected HEK 293T cells, recognizes the target sequence, and is located at that site so that the adenine (A) base of the stop codon TAG present in the target sequence is replaced with the guanine (G) base, the stop codon in front of the mRuby gene is changed to TGG (Trp) and thus the stop codon disappears.

From the experimental results, as illustrated in FIG. 37, it was identified that the CRISPR base editing system normally expressed the mRuby gene in the transfected HEK 293T cells. The base editing rate was found to be 25.2%. In addition, it was identified that the CRISPR base editing system showed a base editing rate of about 40% on day 6 and 60% or higher on day 9 after transfection, and caused the mRuby gene to be normally expressed in HEK 293T cells (FIG. 37).

The rAAV vector loaded with the CRISPR base editing system provided herein exhibited much better base editing efficiency than spCas9-ABE split AAV, and such an effect appeared to become more pronounced over time after transfection.

### Experimental Example 3.16. Identification 2 of base editing effect of AAV vector encoding base editing domain fusion protein

The biggest advantage of the CRISPR gene regulation system disclosed herein is that it is not restricted by the limitation on the deliverable size of AAV. Moreover, the base editing system developed through the present disclosure allows for additional space within the deliverable limits of AAV in a case where one guide RNA is loaded. By adding an additional element such as guide RNA or shRNA into this additional space, the base editing system has the advantage of being able to edit multiple genes simultaneously.

To prove this, as illustrated in FIG. 38, AAV2s each loaded with different guide RNAs and AAV2 loaded with both guide RNAs were produced with reference to Experimental Example 1.6. Then, with reference to Experimental Example 1.7, HEK293T cells were transfected with each AAV2 comprising a nucleic acid encoding the dCas12f1-base editing fusion protein of SEQ ID NO: 299 at the same multiplicity of infection (MOI).

Each of the prepared AAV2 vectors was transfected into HEK 293T cells, and 10 days later, genomic DNA was obtained from the transfected HEK 293T cells. Then, with reference to Experimental Example 1.9, the PCR products were analyzed to determine whether the adenine (A) base was replaced with the guanine (G) base, using targeted deep sequencing.

As a result of the analysis, as illustrated in FIG. 38, efficiency of adenine base editing for both targets was checked for AAV2 loaded with two types of guide RNA. The efficiency for each target was similar to that observed in a case of being loaded with one type of guide RNA. From the results, it was found that multi-target base editing could be achieved in a case of using an AAV vector loaded with a multi-target CRISPR gene regulation system that comprises two or more types of guide RNA.

### Experimental Example 4. Expression regulation effect of CRISPR expression regulating system

### Experimental Example 4.1. Target gene expression inhibition effect of CRISPR interference system

In order to identify a target gene expression inhibition effect of the CRISPR interference system disclosed herein, by setting BRCA1 gene as a target, a protospacer target therefor was selected, and the spacer sequence of the guide RNA was designed accordingly. The spacer sequences are shown in Table 26:

**[Table 26]**

| Label | Spacer sequence (5' to 3') | SEQ ID NO |
|---|---|---|
| gRNA_1 | ATCAGATAGGATCGTCCGAT | 445 |
| gRNA_2 | GGGAGGGCGAGGCCGAAACC | 446 |
| gRNA_3 | GGAGTTTTTTCTTCCCTCTG | 447 |
| gRNA_4 | GAAGGCTGCCAGGTTAAGGC | 448 |
| gRNA_5 | TGCACCCTGCACACTGACCT | 449 |

Here, the comparison group used 18s as a target, and the primers used for qPCR of the comparison group are as shown in Table 27:

**[Table 27]**

| Name | Sequence (5'-3') | SEQ ID NO |
|---|---|---|
| 18s_rRNA_F | TCAACTTTCGATGGTAGTCGCC | 458 |
| 18s_rRNA_R | GGCCTCGAAAGAGTCCTGTATTGT | 459 |

A vector capable of expressing the CRISPR interference system, which comprises the following dCas12f1-expression regulating fusion protein and the guide RNA targeting Table 26, was constructed according to Experimental Example 1.4, and the constructed vector was transfected into HEK293T-lentiX cells according to Experimental Example 1.10 to analyze its gene expression suppression effect in the cells.

From the experimental results, as illustrated in FIGS. 39 to 49, it was identified that the level of BRCA1 mRNA decreased in most modules, regardless of the position (N-terminus and/or C-terminus) at which the gene expression regulatory domain is fused to dead Cas12f1.

### Experimental Example 4.2. Effect of CRISPR activation system on promoting

### expression of target gene

In order to identify an effect of the CRISPR activation system disclosed herein on promoting expression of a target gene, by setting OCT4 gene as a target, a protospacer target therefor was selected. The selected target protospacer sequences are as shown in Table 28:

**[Table 28]**

| Label | PAM | Protospacer (5' to 3') | SEQ ID NO |
|---|---|---|---|
| activation-1 | TTTG | TTGCCCAGACTGGAGTGCAG | 450 |
| activation-2 | TTTG | GCCCAGTAGATCGAGGCTAC | 451 |
| activation-3 | TTTG | CCTAATGGTGGTGGCAATGG | 452 |
| activation-4 | TTTA | AGACAGGGTCTCACTTTGTTG | 453 |

A dCas12f1-expression regulating fusion protein, which comprises the dead Cas12f1 protein of SEQ ID NO: 5 and the VP64 gene expression promotion domain of SEQ ID NO: 329, was prepared with reference to Experimental Example 1.4. The vector encoding the prepared dCas12f1-expression regulating fusion protein and each guide RNA was transfected into HEK293T cells using FugeneHD (Promega) reagent. The transfection method was performed according to the protocol for the reagent. 72 hours after transfection, the cells were harvested and RNA was extracted therefrom. Using the extracted RNA, cDNA was synthesized using the SuperScript iV (Invitrogen) kit. Changes in expression were analyzed by qPCR using cDNA as a template.

As illustrated in FIG. 50, it was found that the CRISPR activation system promoted transcription of the OCT4 gene.

### Experimental Example 5. Further expansion for dead Cas12f1 protein mutations

The following experiment was performed to determine whether a dead form of the Cas12f1 protein could be also produced even in a case where replacement with an amino acid other than alanine occurs at the mutation position where a dead form of the Cas12f1 protein can be produced:

For the Cas12f1-expressing vector in Experimental Example 1.1, modification was performed using mutagenesis so that Cas12f1 became its dead form. The dead forms are D326A, E422A, R490A, or D510A, and include R490Q, R490W, R490L, D510L, D510V, or other mutated forms that have lost cleavage activity. Primers used for each mutagenesis are shown in Table 29.

**[Table 29]**

| LABEL | WT | Mut | Forward (5'-3') | Reverse (5'-3') |
|---|---|---|---|---|
| D360A | GAT | GCT | CGGAGGGATCgctGTGGGGGTCA | ATGATGCTGGGATCCACGC |
| | | | (SEQ ID NO: S23) | (SEQ ID NO: 532) |
| E422A | GAG | GCG | AGTGCAGATGgcgAACCTCGAGA | GTTCCGACCTTGTTCTTAATAAAG |
| | | | (SEQ ID NO: 524) | (SEQ ID NO: 533) |
| R490A | CGG | GCG | CTTCGAGTACgcgAAGAAGAACAAG | TTGAAGTAGTTGTTGAGG |
| | | | (SEQ ID NO: 525) | (SEQ ID NO: 534) |
| R490Q | CGG | CAG | CTTCGAGTACcagAAGAAGAACAAG | TTGAAGTAGTTGTTGAGG |
| | | | (SEQ ID NO: 526) | (SEQ ID NO: 535) |
| R490W | CGG | TGG | CTTCGAGTACtggAAGAAGAACAAG | TTGAAGTAGTTGTTGAGGTG |
| | | | (SEQ ID NO: 527) | (SEQ ID NO: 536) |
| R490L | CGG | CTG | CTTCGAGTACctgAAGAAGAACAAG | TTGAAGTAGTTGTTGAGG |
| | | | (SEQ ID NO: 528) | (SEQ ID NO: 537) |
| D510A | GAT | GCT | GGAGAACGCCgctT ACAACGCCG | TTAAAGTTGCACTTCTCGC |
| | | | (SEQ ID NO: 529) | (SEQ ID NO: 538) |
| D510L | GAT | CTT | GGAGAACGCCcttTACAACGCCG | TTAAAGTTGCACTTCTCG |
| | | | (SEQ ID NO: 530) | (SEQ ID NO: 539) |
| D510V | GAT | GTA | GGAGAACGCCgtaTACAACGCCG | TTAAAGTTGCACTTCTCGC |
| | | | (SEQ ID NO: 531) | (SEQ ID NO: 540) |

To identify whether cleavage activity of the produced dead Cas12f1 was removed, transfection of HEK293T cells was performed by setting 5'-CACACACACAGTGGGCTACCATT-3' (SEQ ID NO: 522) as a target. 96 hours after transfection, gDNA was extracted therefrom and comparison was performed through NGS analysis in terms of generation of indels (FIG. 58).

From the experimental results, it was found that all of R490A, R490Q, R490L, R490W, DS 10A, D510L, D510V, E422A, and E326A, which are based on the wild-type Cas12f1 protein of SEQ ID NO: 1, had lost nucleic acid cleavage activity, indicating that a dead form of Cas12f1 can be produced even in a case where the amino acid at the appropriate mutation position is replaced with glutamine, leucine, tryptophan, or valine which is an amino acid other than alanine.

### Industrial Applicability

The CRISPR gene regulating complex and the components thereof disclosed herein are capable of exhibiting various gene function regulation effects, such as base editing for a target gene in a cell and target gene expression regulation (promotion or inhibition), which allows the CRISPR gene regulating complex to be used in the technical fields of gene base editing and/or gene expression regulation. In addition, it is possible to load the CRISPR gene regulation system onto a single unit of adeno-associated virus (AAV) vector, from which high efficiency and usability can be expected.

## Claims

1. A wild-type Cas12f1-based dead Cas12f1 protein, represented by the following amino acid sequence: 
MAKNTITKTLKLRIVRPYNSAEVEKIVADEKNNREKIALEKNKDKVKE
ACSKHLKVAAYCTTQVERNACLFCKARKLDDKFYQKLRGQFPDAVFWQEISE
IFRQLQKQAAEIYNQSLIELYYEIFIKGKGX1ANASX2VEHYLSDVCYTRAAELF
KNAAIASGLRSKIKSNFRLKELKNMKSGLPTTKSDNFPIPLVKQKGGQYTGFEI
SNHNSDFIIKIPFGRWQVKKEIDKYRPWEKFDFEQVQKSPKPISLLLSTQRRKR
NKGWSKDEGTEAEIKKVMNGDYQTSYIEVKRGSKIGEKSAWMLNLSIDVPKI
DKGVDPSIIGGIX3VGVKSPLVCAINNAFSRYSISDNDLFHFNKKMFARRRILLK
KNRHKRAGHGAKNKLKPITILTEKSERRFKKLIERWACEIADFFIKNKVGTVQ
MX4NLESMKRKEDSYFNIRLRGFWPYAEMQNKIEFKLKQYGIEIRKVAPNNTS
KTCSKCGHLNNYFNFEYX5KKNKFPHFKCEKCNFKENAX6YNAALNISNPKLK
STKEEP (SEQ ID NO: 9),
wherein X₁ is isoleucine or tryptophan, X₂ is serine or tyrosine, X₃ is aspartic acid, alanine, glutamine, leucine, tryptophan, or valine, X₄ is glutamic acid, alanine, glutamine, leucine, tryptophan, or valine, X₅ is arginine, alanine, glutamine, leucine, tryptophan, or valine, and X₆ is aspartic acid, alanine, glutamine, leucine, tryptophan, or valine, and
at least one of X₃, X₄, X₅, and X₆ is alanine, glutamine, leucine, tryptophan, or valine.

2. A dead Cas12f1 protein, comprising:
a dummy portion comprising 20 to 30 amino acids; and
a wild-type Cas12f1-based dead Cas12f1 protein,
wherein the dummy portion is represented by an amino acid sequence selected from MGEKSSRRRRNGKSGAWTAAITSCVGGK (SEQ ID NO: 10), MAGGPGAGSAAPVSSTSSLPLAALNMRV (SEQ ID NO: 11), MAGGPGAGSAAPVSSTSSLPLAALNM (SEQ ID NO: 12), and MEKRINKIRKKLSADNATKPVSRSGP (SEQ ID NO: 13);
the wild-type Cas12f1-based dead Cas12f1 protein is represented by 
MAKNTITKTLKLRIVRPYNSAEVEKIVADEKNNREKIALEKNKDKVKEACSKH
LKVAAYCTTQVERNACLFCKARKLDDKFYQKLRGQFPDAVFWQEISEIFRQLQ
KQAAEIYNQSLIELYYEIFIKGKGX1ANASX2VEHYLSDVCYTRAAELFKNAAI
ASGLRSKIKSNFRLKELKNMKSGLPTTKSDNFPIPLVKQKGGQYTGFEISNHNS
DFIIKIPFGRWQVKKEIDKYRPWEKFDFEQVQKSPKPISLLLSTQRRKRNKGWS
KDEGTEAEIKKVMNGDYQTSYIEVKRGSKIGEKSAWMLNLSIDVPKIDKGVD
PSIIGGIX3VGVKSPLVCAINNAFSRYSISDNDLFHFNKKMFARRRILLKKNRHK
RAGHGAKNKLKPITILTEKSERFRKKLIERWACEIADFFIKNKVGTVQMX4NLE
SMKRKEDSYFNIRLRGFWPYAEMQNKIEFKLKQYGIEIRKVAPNNTSKTCSKC
GHLNNYFNFEYX5KKNKFPHFKCEKCNFKENAX6YNAALNISNPKLKSTKEEP
(SEQ ID NO: 9),
wherein X₁ is isoleucine or tryptophan, X₂ is serine or tyrosine, X₃ is aspartic acid, alanine, glutamine, leucine, tryptophan, or valine, X₄ is glutamic acid, alanine, glutamine, leucine, tryptophan, or valine, X₅ is arginine, alanine, glutamine, leucine, tryptophan, or valine, and X₆ is aspartic acid, alanine, glutamine, leucine, tryptophan, or valine,
at least one of X₃, X₄, X₅, and X₆ is alanine, glutamine, leucine, tryptophan, or valine, and
the dummy portion and the wild-type Cas12f1-based dead Cas12f1 protein are sequentially linked to each other in a direction from the N terminus to the C terminus of the dead Cas12f1 protein.

3. The dead Cas12f1 protein of claim 1, wherein the dead Cas12f1 protein is represented by an amino acid sequence selected from SEQ ID NOS: 2 to 8.

4. The dead Cas12f1 protein of claim 2, wherein the dead Cas12f1 protein is represented by an amino acid sequence selected from SEQ ID NOS: 15 to 24, SEQ ID NOS: 26 to 29, SEQ ID NOS: 31 to 34, and SEQ ID NOS: 36 to 39.

5. A dCas12f1-base editing fusion protein, comprising:
the dead Cas12f1 protein of any one of claims 1 to 4; and
deaminase,
wherein the deaminase has an amino acid sequence selected from SEQ ID NO: 245, SEQ ID NOS: 247 to 249, and SEQ ID NOS: 274 to 283.

6. The dCas 12f1-base editing fusion protein of claim 5, wherein the deaminase has an amino acid sequence selected from SEQ ID NOS: 274 to 279.

7. The dCas 12f1-base editing fusion protein of claim 6, wherein the deaminase is fused to the N terminus of the dead Cas12f1 protein.

8. The dCas 12f1-base editing fusion protein of claim 6, wherein the deaminase is fused to the C terminus of the dead Cas12f1 protein.

9. The dCas12f1-base editing fusion protein of claim 6, wherein the dCas12f1-base editing fusion protein has an amino acid sequence selected from SEQ ID NOS: 284 to 324 and SEQ ID NOS: 418 to 442.

10. The dCas12f1-base editing fusion protein of claim 5, wherein the dCas12f1-base editing fusion protein further comprises at least one uracil glycosylase inhibitor (UGI),
the deaminase has an amino acid sequence selected from SEQ ID NOS: 280 to 283, and
the at least one UGI is fused to the dead Cas12f1 protein.

11. The dCas12f1-base editing fusion protein of claim 10, wherein the deaminase is fused to the N terminus of the dead Cas12f1 protein, and
the at least one UGI is fused to the C terminus of the dead Cas12f1 protein.

12. The dCas12f1-base editing fusion protein of claim 10, wherein the deaminase is fused to the C terminus of the dead Cas12f1 protein, and
the at least one UGI is fused to the N terminus of the dead Cas12f1 protein.

13. The dCas12f1-base editing fusion protein of claim 10, which is represented by an amino acid sequence selected from SEQ ID NOS: 325 to 328.

14. The dCas12f1-base editing fusion protein of any one of claims 5 to 13, wherein the dead Cas12f1 protein and the deaminase are linked via a linker, and
the linker is represented by an amino acid sequence selected from SEQ ID NOS: 260 to 273.

15. The dCas12f1-base editing fusion protein of any one of claims 5 to 14, wherein the dCas12f1-base editing fusion protein further comprises at least one nuclear localization signal (NLS), and
the NLS is located at the N-terminus, the C-terminus, or both termini.

16. A dCas12f1-expression regulating fusion protein, comprising:
the dead Cas12f1 protein of any one of claims 1 to 4; and
at least one expression regulatory domain, each of which is selected from VP64, KRAB, MeCP2, DNMT, HDAC, Tet1, and p300.

17. The dCas12f1-expression regulating fusion protein of claim 16, wherein the expression regulatory domain is each independently represented by an amino acid sequence selected from SEQ ID NOS: 329 to 333.

18. The dCas12f1-expression regulating fusion protein of any one of claims 16 to 17, wherein the expression regulatory domains are all located at the N terminus of the dead Cas12f1 protein.

19. The dCas12f1-expression regulating fusion protein of claim 18, which is represented by an amino acid sequence selected from SEQ ID NOS: 511 to 513.

20. The dCas12f1-expression regulating fusion protein of any one of claims 16 to 17, wherein the expression regulatory domains are all located at the C terminus of the dead Cas12f1 protein.

21. The dCas12f1-expression regulating fusion protein of claim 20, which is represented by an amino acid sequence selected from SEQ ID NOS: 514 to 518.

22. The dCas12f1-expression regulating fusion protein of any one of claims 16 to 17, wherein the dCas12f1-expression regulating fusion protein comprises a first expression regulatory domain and a second expression regulatory domain,
the first expression regulatory domain is located at the N terminus of the dead Cas12f1 protein, and
the second expression regulatory domain is located at the C terminus of the dead Cas12f1 protein.

23. The dCas12f1-expression regulating fusion protein of claim 22, which is represented by an amino acid sequence selected from SEQ ID NOS: 519 to 521.

24. An engineered CRISPR/Cas12f1 composition, comprising:
an engineered Cas12f1 protein selected from the dead Cas12f1 protein of any one of claims 1 to 4, the dCas12f1-base editing fusion protein of any of claims 5 to 15, and the dCas12f1-expression regulating fusion protein of any one of claims 16 to 23, or a nucleic acid encoding the engineered Cas12f1 protein; and
at least one guide RNA, or a nucleic acid encoding the guide RNA,
wherein each of the guide RNAs comprises a scaffold, a spacer, and a U-rich tail,
the scaffold, the spacer, and the U-rich tail are sequentially linked to each other in a 5' to 3' direction,
the scaffold is represented by a nucleotide sequence selected from SEQ ID NOS: 197 to 199,
the U-rich tail is represented by a nucleotide sequence of (UₐN)_{b}U_{c}, where N is each independently selected from A, U, C, and G, a is an integer of between 1 to 5 inclusive, and b is an integer of 0 or more, and
the spacer comprises nucleosides of between 10 and 50 inclusive and has a nucleotide sequence complementary to a predetermined target sequence.

25. The engineered CRISPR/Cas12f1 composition of claim 24, wherein the engineered Cas12f1 protein is the dCas12f1-base editing fusion protein of any one of claims 5 to 15.

26. The engineered CRISPR/Cas12f1 composition of claim 25, wherein the engineered CRISPR/Cas12f1 composition comprises the engineered Cas12f1 protein and the guide RNA in a form of a ribonucleoprotein (RNP).

27. The engineered CRISPR/Cas12f1 composition of claim 25, wherein the engineered CRISPR/Cas12f1 composition comprises the nucleic acid encoding the engineered Cas12f1 protein and the nucleic acid encoding the guide RNA in a form of a vector.

28. The engineered CRISPR/Cas12f1 composition of claim 27, wherein the vector comprises the nucleic acid encoding the engineered Cas12f1 protein, a nucleic acid encoding a first guide RNA, and a nucleic acid encoding a second guide RNA, and
a nucleotide sequence of a spacer in the first guide RNA and a nucleotide sequence of a spacer in the second guide RNA are different from each other.

29. The engineered CRISPR/Cas12f1 composition of any one of claims 25 to 28, wherein the engineered Cas12f1 protein is the dCas12f1-base editing fusion protein of any one of claims 6 to 9.

30. The engineered CRISPR/Cas12f1 composition of any one of claims 25 to 28, wherein the engineered Cas12f1 protein is the dCas12f1-base editing fusion protein of any one of claims 10 to 14.

31. The engineered CRISPR/Cas12f1 composition of claim 24, wherein the engineered Cas 12f1 protein is the dCas12f1-expression regulating fusion protein of any one of claims 16 to 23.

32. The engineered CRISPR/Cas12f1 composition of claim 31, wherein the dCas12f1-expression regulating fusion protein comprises at least one VP64.

33. The engineered CRISPR/Cas12f1 composition of claim 31, wherein the dCas12f1-expression regulating fusion protein comprises at least one expression regulatory domain selected from VP64, KRAB, MeCP2, DNMT, HDAC, Tet1, and p300.

34. The engineered CRISPR/Cas12f1 composition of claim 31, wherein the engineered CRISPR/Cas12f1 composition comprises the engineered Cas12f1 protein and the guide RNA in a form of a ribonucleoprotein (RNP).

35. The engineered CRISPR/Cas12f1 composition of claim 31, wherein the engineered CRISPR/Cas12f1 composition comprises a vector that comprises the nucleic acid encoding the engineered Cas12f1 protein and the nucleic acid encoding the guide RNA.

36. The engineered CRISPR/Cas12f1 composition of claim 35, wherein the vector comprises the nucleic acid encoding the engineered Cas12f1 protein, a nucleic acid encoding a first guide RNA, and a nucleic acid encoding a second guide RNA, and
a nucleotide sequence of a spacer in the first guide RNA and a nucleotide sequence of a spacer in the second guide RNA are different from each other.

37. A method for base editing of a target gene in a cell, comprising:
introducing the engineered CRISPR/Cas12f1 composition of claim 29 into a living cell,
wherein the target gene in the cell is double-stranded DNA comprising a target strand and a non-target strand,
the target strand has a target sequence,
the non-target strand has a protospacer adjacent motif (PAM) and a protospacer,
the protospacer is a nucleotide sequence of 10 to 50 nts which is complementary to the target sequence,
a spacer in the guide RNA of the engineered CRISPR/Cas12f1 composition is capable of hybridizing with the target sequence in the target strand, and
the introduction of the engineered CRISPR/Cas12f1 composition into the cell causes a CRISPR-base editing complex to be formed in the cell, and the CRISPR-base editing complex replaces at least one adenine in the protospacer with guanine.

38. The method of claim 37, wherein the protospacer in the target gene contains at least one adenine at 2nd, 3rd, 4th, 5th, 6th, 7th, 8th, 9th, 15th, 16th, 17th, 18th, 19th, and 20th positions from the 5' end, and performing the method for base editing results in replacement of at least one of the adenines at the 2nd, 3rd, 4th, 5th, 6th, 7th, 8th, 9th, 15th, 16th, 17th, 18th, 19th, and 20th positions with guanine.

39. A method for base editing of a target gene in a cell, comprising:
introducing the engineered CRISPR/Cas12f1 composition of claim 30 into the cell,
wherein the target gene in the cell is double-stranded DNA comprising a target strand and a non-target strand,
the target strand has a target sequence,
the non-target strand has a protospacer adjacent motif (PAM) and a protospacer,
the protospacer is a nucleotide sequence of 10 to 50 nts which is complementary to the target sequence,
a spacer in the guide RNA of the engineered CRISPR/Cas12f1 composition is capable of hybridizing with the target sequence in the target strand, and
the introduction of the engineered CRISPR/Cas12f1 composition into the cell causes a CRISPR-base editing complex to be formed in the cell, and the CRISPR-base editing complex replaces at least one cytosine in the protospacer with thymine.

40. The method of claim 39, wherein the protospacer in the target gene contains at least one cytosine at 2nd, 3rd, 4th, 5th, 6th, 7th, 8th, and 9th positions from the 5' end, and performing the method for base editing results in replacement of at least one of the cytosines at the 2nd, 3rd, 4th, 5th, 6th, 7th, 8th, and 9th positions with thymine.

41. The method of any one of claims 37 to 40, wherein the cell is a eukaryotic cell.

42. A method for regulating expression of a target gene in a cell, comprising:
introducing the engineered CRISPR/Cas12f1 composition of any one of claims 31 to 36 into the cell,
wherein the introduction of the engineered CRISPR/Cas12f1 composition into the cell causes a CRISPR gene regulating complex to be formed, and
the CRISPR gene regulating complex regulates expression of the target gene.

43. The method of claim 42, wherein the cell is a eukaryotic cell.
